# EUROPÄISCHE PATENTSCHRIFT

(11) **EP 0 835 243 B1**
(45) Veröffentlichungstag und Bekanntmachung des Hinweises auf die Patenterteilung: **29.01.2003**
(21) Anmeldenummer: 96922817.0
(22) Anmeldetag: 17.06.1996
(51) Int. Cl.: C07D 207/38, C07D 307/60, C07D 333/32, C07D 309/32, C07D 279/06, A01N 43/36, A01N 43/86, A01N 43/08, A01N 43/10

(54) **DIALKYL-HALOGENPHENYLSUBSTITUIERTE KETOENOLE ZUR VERWENDUNG ALS HERBIZIDE UND PESTIZIDE**
DIALKYL PHENYL HALIDE-SUBSTITUTED KETO-ENOLS FOR USE AS HERBICIDES AND PESTICIDES
CETOENOLS A SUBSTITUTION DIALKYLE-HALOGENURE DE PHENYLE S'UTILISANT COMME HERBICIDES ET COMME PESTICIDES

(30) Priorität: 30.06.1995 DE 19523850; 31.01.1996 DE 19603332
(43) Veröffentlichungstag der Anmeldung: 15.04.1998
(73) Patentinhaber: Bayer CropScience AG, 40789 Monheim (DE)
(72) Erfinder: FISCHER, Reiner, D-40789 Monheim (DE); BRETSCHNEIDER, Thomas, D-53797 Lohmar (DE); HAGEMANN, Hermann, D-51375 Leverkusen (DE); LIEB, Folker, D-51375 Leverkusen (DE); RUTHER, Michael, D-40789 Monheim (DE); WIDDIG, Arno, D-51519 Odenthal (DE); DAHMEN, Peter, D-41470 Neuss (DE); DOLLINGER, Markus, D-51381 Leverkusen (DE); ERDELEN, Christoph, D-42799 Leichlingen (DE); SANTEL, Hans-Joachim, D-51371 Leverkusen (DE); WACHENDORFF-NEUMANN, Ulrike, D-56566 Neuwied (DE); GRAFF, Alan, D-51427 Bergisch Gladbach (DE); ANDERSCH, Wolfram, D-51469 Bergisch Gladbach (DE)
(86) Internationale Anmeldenummer: EP9602601
(87) Internationale Veröffentlichungsnummer: WO97002243

(56) Entgegenhaltungen:
- EP-A- 0 456 063
- EP-A- 0 528 156
- EP-A- 0 588 137
- EP-A- 0 595 130
- EP-A- 0 596 298
- EP-A- 0 613 885
- WO-A-94/14785
- WO-A-95/01358
- WO-A-95/01971

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide und/oder Herbizide wirksam sind.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, DE 4 440 594, EP-A-613 885, WO 94/01 997 und WO 95/ 01 358).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans, 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156 bekannt, jedoch ist die dort beschriebene Wirkung nicht immer ausreichend. 3-Aryl-4-hydroxy-Δ³-dihydrofuranon-Derivate sind aus der EP-A-647 637 bekannt. Thiotetronsäuren sind aus der WO 95/26345 bekannt.

Aus der Literatur sind ferner bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J. Heterocycl. Chem., 25(5), 1301-1305, 1988 oder J. Heterocycl. Chem., 25(5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4,4',4"-(5-Hydroxy-3-oxo-1H-pyrazol-1,2,4(3H)-triyl)-tris-benzolsulfonsäure pharmakologische Eigenschaften besitzt (vgl. Farmakol. Toksikol. (Moscow), 38(2), 180-186, 1976). Seine Verwendung im Pflanzenschutz ist aber nicht bekannt.

Außerdem sind in EP-A-508 126 und in WO 92/16 510 4-Arylpyrazolidin-3,5-dion-Derivate mit herbiziden, akariziden und insektiziden Eigenschaften beschrieben.

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785 beschrieben.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit der bekannten Verbindungen für Kulturpflanzen nicht immer ausreichend.

Es wurden nun neue Verbindungen der Formel (I) 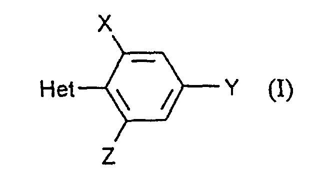 gefunden,
in welcher
- X: für Methyl, Ethyl, n-Propyl oder iso-Propyl steht,
- Y: für Fluor, Chlor, Brom, Methyl, Ethyl, n-Propyl oder iso-Propyl steht,
- Z: für Fluor, Chlor, Brom, Methyl, Ethyl, n-Propyl oder iso-Propyl steht,
wobei immer einer der Reste Y und Z für Halogen und der andere für Alkyl steht,
- Het: für eine der Gruppen 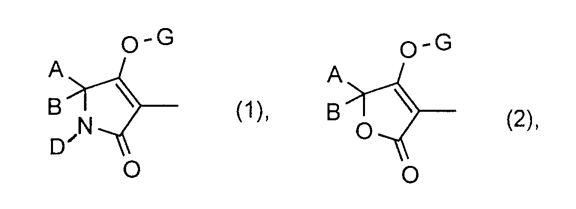 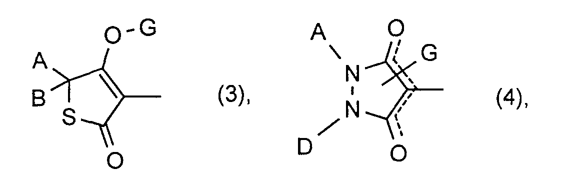 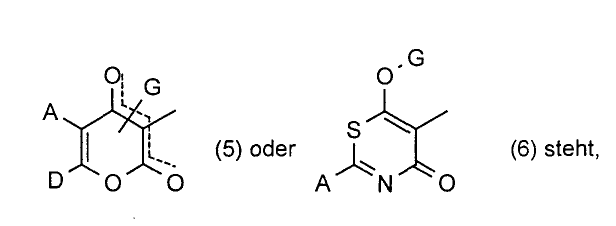
- A: für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄-alkyl, Poly-C₁-C₄-alkoxy-C₁-C₄-alkyl oder C₁-C₆-Alkylthio-C₁-C₄-alkyl, oder für gegebenenfalls durch Fluor, Chlor, Methyl oder Methoxy substituiertes C₃-C₆-Cycloalkyl, in welchem gegebenenfalls eine oder zwei Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Thienyl, Pyridyl oder Benzyl steht,
- B: für Wasserstoff, C₁-C₈-Alkyl oder C₁-C₄-Alkoxy-C₁-C₂-alkyl steht oder
- A, B: und das Kohlenstoffatom an das sie gebunden sind, für C₃-C₈-Cycloalkyl oder C₅-C₈-Cycloalkenyl stehen, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
- A, B: und das Kohlenstoffatom, an das sie gebunden sind, für C₅-C₆-Cycloalkyl stehen, welches durch eine gegebenenfalls ein Sauerstoff- oder Schwefelatom enthaltende Alkylendiyl- oder durch eine Alkylendioxyl-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, für C₃-C₆-Cycloalkyl oder C₅-C₆-Cycloalkenyl stehen, in dem zwei Substituenten gemeinsam mit den Kohlenstoffatomen, an die sie gebunden sind, für C₃-C₄-Alkandiyl, C₃-C₄-Alkendiyl oder Butadiendiyl stehen, worin jeweils gegebenenfalls eine Methylengruppe durch Sauerstoff oder Schwefel ersetzt ist,
- D: für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl steht,
oder
- A und D: gemeinsam für eine C₃-C₅-Alkandiyl- oder C₃-C₅-Alkendiylgruppe stehen, worin jeweils gegebenenfalls eine Methylengruppe durch Sauerstoff oder Schwefel ersetzt ist und welche gegebenenfalls durch Fluor, Chlor, Hydroxy, Mercapto oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy substituiert sind oder
worin gegebenenfalls eine der folgenden Gruppen 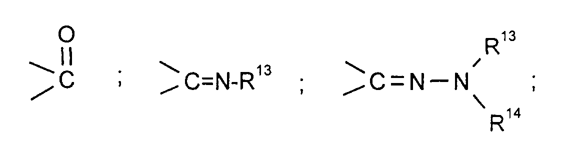 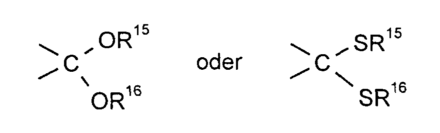 enthalten ist,
oder A und D im Fall der Verbindungen der Formel (I-1) gemeinsam mit den Atomen, an die sie gebunden sind, für eine der folgenden Gruppen 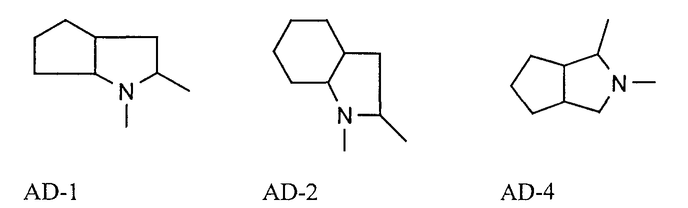 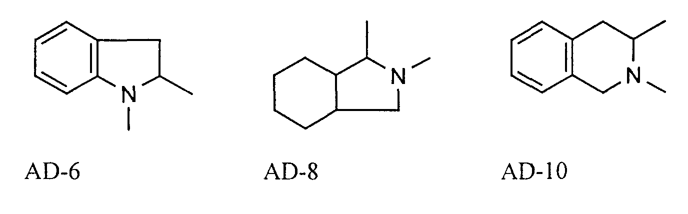 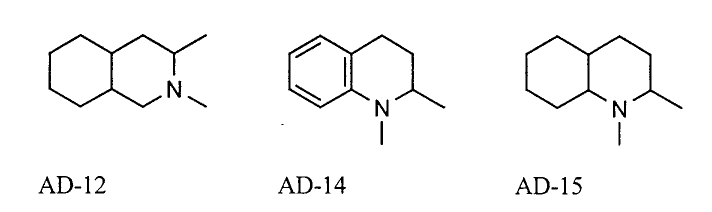 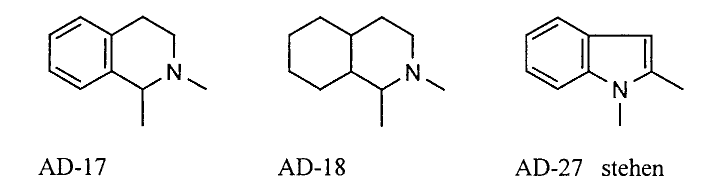
- G: im Fall, dass Het für einen der Reste (1), (2), (3), (5) oder (6) steht, für Wasserstoff (a) oder, im Fall, dass Het für einen der Reste (1), (2), (3), (4), (5) oder (6) steht, für eine der Gruppen 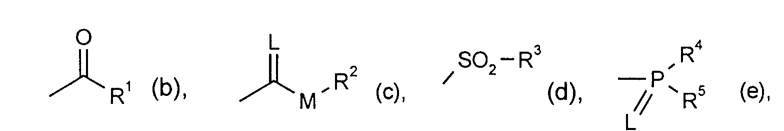 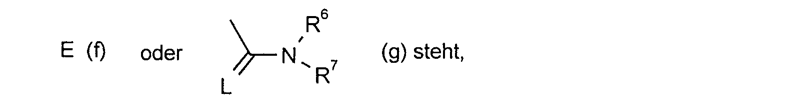 in welchen
E für ein Metallionäquivalent oder ein Ammoniumion steht,
L für Sauerstoff oder Schwefel steht und
M für Sauerstoff oder Schwefel steht,
- R¹: für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei Methylengruppen durch Sauerstoff und/ oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
- R²: für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
- R³: für jeweils gegebenenfalls durch Fluor oder Chlor substituiertes Methyl, Ethyl, Propyl, Isopropyl oder jeweils gegebenenfalls durch Fluor, Chlor, Brom, Methyl, Ethyl, Propyl, iso-Propyl, tert.-Butyl, Methoxy, Ethoxy, Isopropoxy, tert.-Butoxy, Trifluormethyl, Trifluormethoxy, Cyano oder Nitro substituiertes Phenyl oder Benzyl steht,
- R⁴ und R⁵: unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen,
- R⁶ und R⁷: unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebenenfalls eine Methylengruppe durch Sauerstoff oder Schwefel ersetzt ist,
- R¹³: für Wasserstoff oder für jeweils gegebenenfalls durch Fluor oder Chlor substituiertes C₁-C₄-Alkyl oder C₁-C₄-Alkoxy, für C₃-C₆-Cycloalkyl oder für jeweils gegebenenfalls durch Fluor, Chlor, Brom, Methyl, Ethyl, iso-Propyl, tert.-Butyl, Methoxy, Ethoxy, iso-Propoxy, tert.-Butoxy, Trifluormethyl, Trifluormethoxy, Nitro oder Cyano substituiertes Phenyl, Phenyl-C₁-C₂-alkyl oder Benzyloxy steht,
- R¹⁴: für Wasserstoff oder C₁-C₄-Alkyl steht oder
- R¹³ und R¹⁴: gemeinsam für C₄-C₆-Alkandiyl stehen,
- R¹⁵ und R¹⁶: gleich oder verschieden sind und für Methyl oder Ethyl stehen oder
- R¹⁵ und R¹⁶: zusammen für einen C₂-C₃-Alkandiylrest stehen, der gegebenenfalls durch Methyl, Ethyl, n-Propyl, iso-Propyl, n-Butyl, iso-Butyl, sek.-Butyl, tert.-Butyl oder durch gegebenenfalls durch Fluor, Chlor, Methoxy, Trifluormethyl, Trifluormethoxy, Nitro oder Cyano substituiertes Phenyl substituiert ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (6) der Gruppe Het ergeben sich folgende hauptsächliche Strukturen (I-1) bis (I-6): 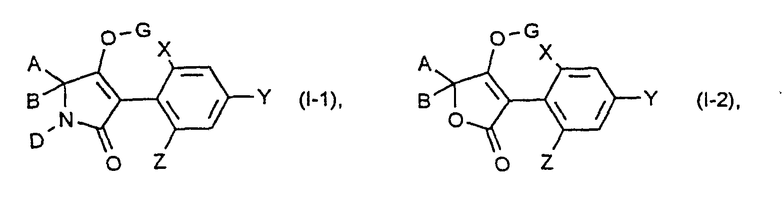 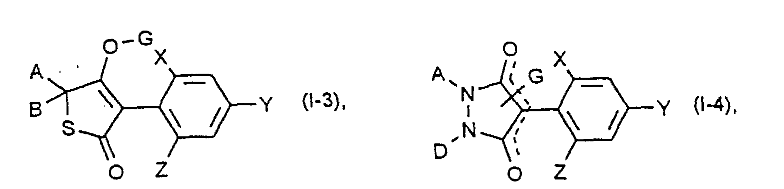 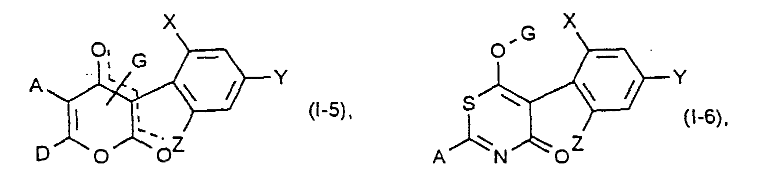 worin in welchen
A, B, D, G, X, Y und Z die oben angegebene Bedeutung haben.
Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 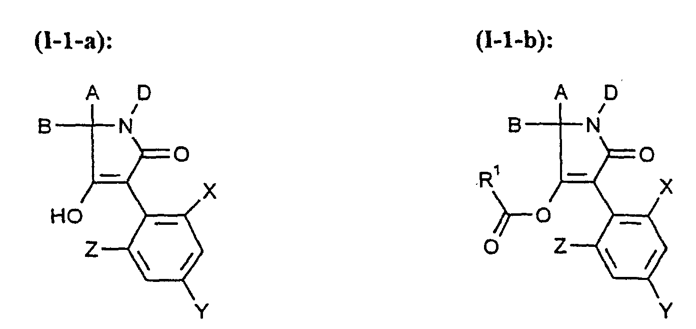 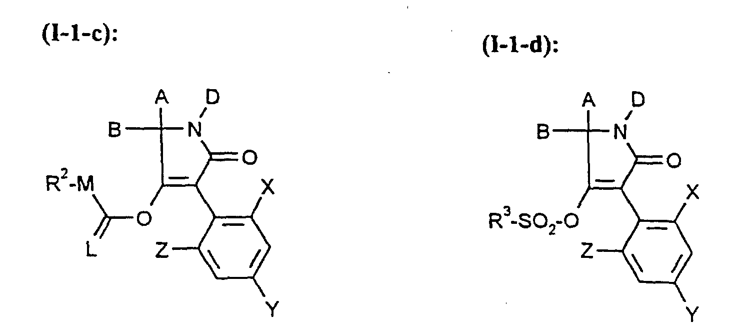 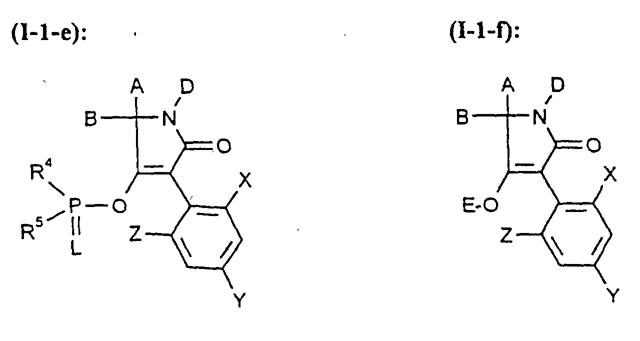 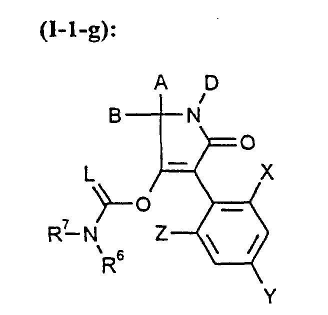 worin
A, B, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn Het für die Gruppe (2) steht, 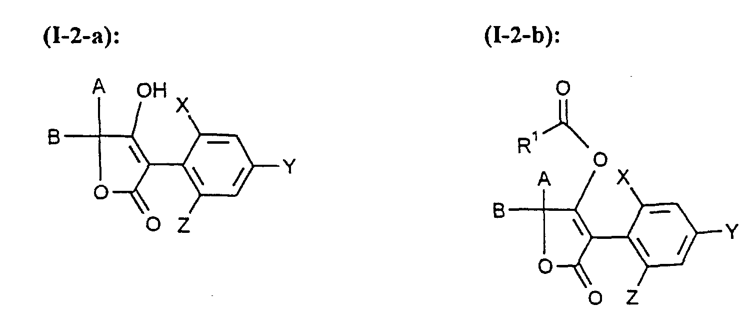 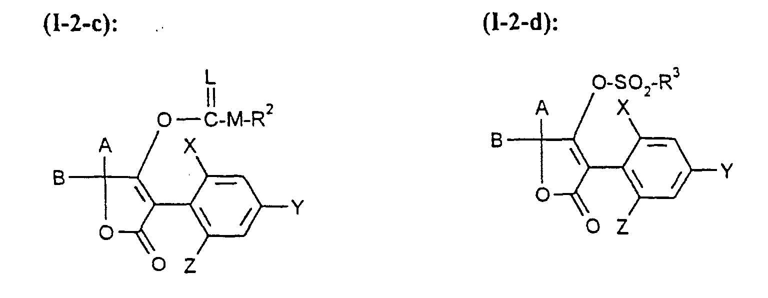 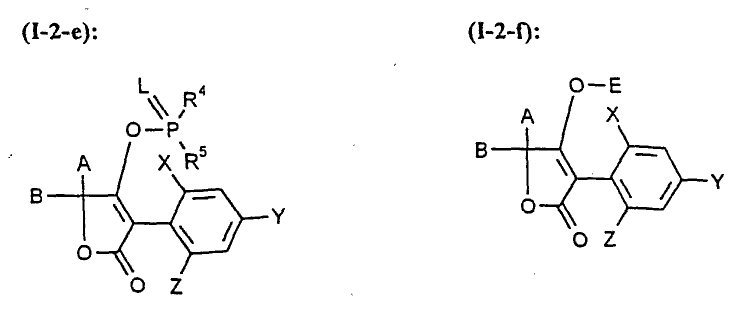 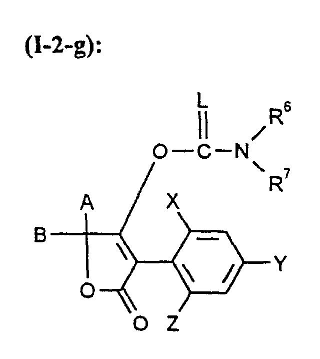 worin
A, B, E, L, M, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn Het für die Gruppe (3) steht, 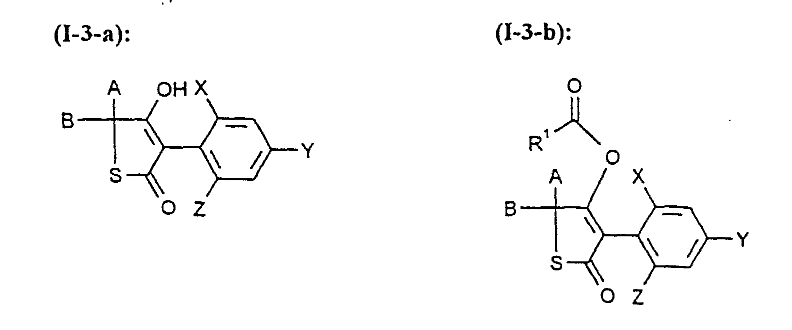 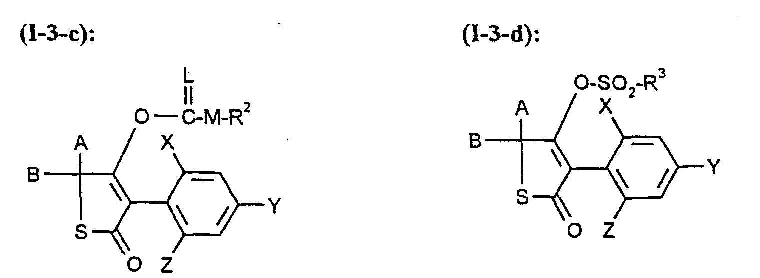 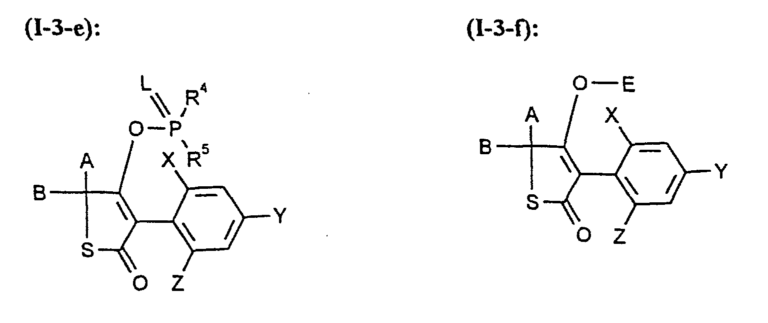 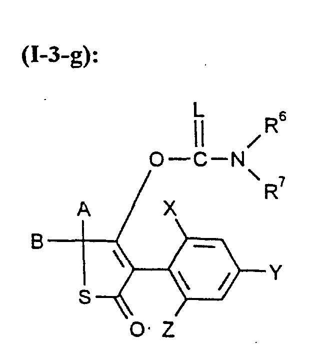 worin
A, B, E, L, M, X, Y, Z, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Forr In (I-4)ₐ und (I-4)_{b} vorliegen, 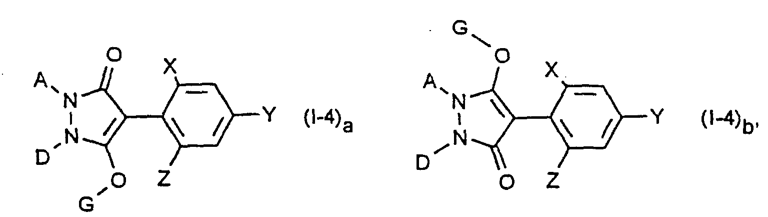 was durch die gestrichelte Linie in der Formel (I-4) zum Ausdruck gebracht werden soll:

Die Verbindungen der Formeln (I-4)ₐ und (I-4)_{b} können sowohl als Gemische als auch in Form ihrer reinen Isomeren vorliegen. Gemische der Verbindungen der Formel (I-4)ₐ und (I-4)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (b), (c), (d), (e), (f) und (g) der Gruppe G ergeben sich folgende hauptsächliche Strukturen (I-4-b) bis (I-4-g), wenn Het für die Gruppe (4) steht, 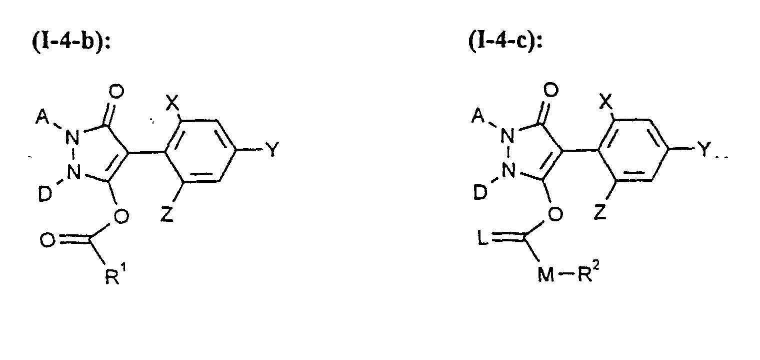 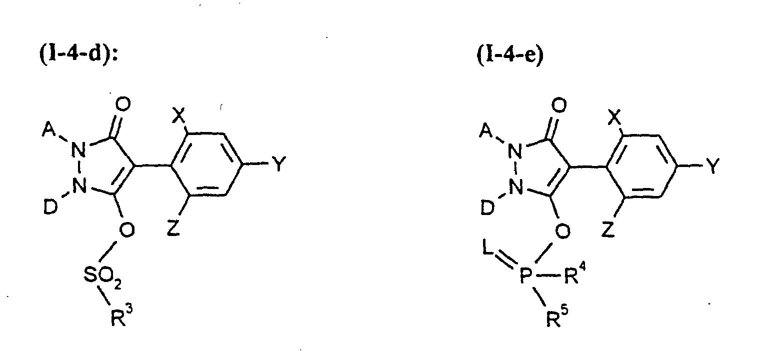 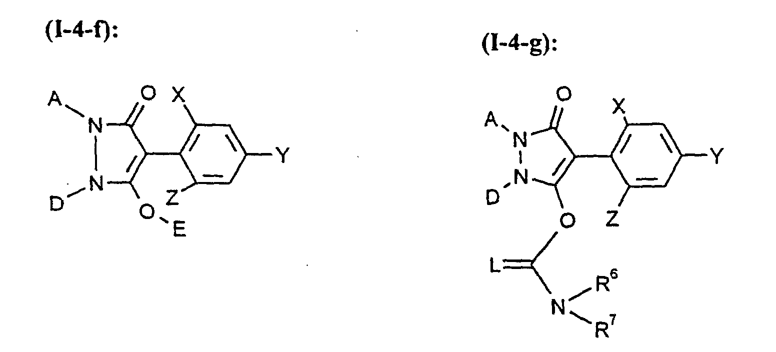 worin
A, D, E, L, M, X, Y, Z, R¹, R², R³, R⁴, R⁵, R⁶ und R⁷ die oben angegebenen Bedeutungen besitzen.

Die Verbindungen der Formel (I-5) können in Abhängigkeit von der Stellung des Substituenten G in den zwei isomeren Formen der Formeln (I-5)ₐ und (I-5)_{b} vorliegen, 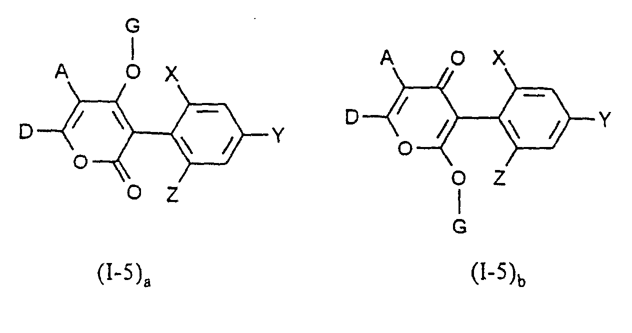 was durch die gestrichelte Linie in der Formel (I-5) zum Ausdruck gebracht werden soll.

Die Verbindungen der Formeln (I-5)ₐ und (I-5)_{b} können sowohl als Gemische als auch in Form ihrer reinen Isomeren vorliegen. Gemische der Verbindungen der Formeln (I-5)ₐ und (I-5)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungengegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn Het für die Gruppe (5) steht, 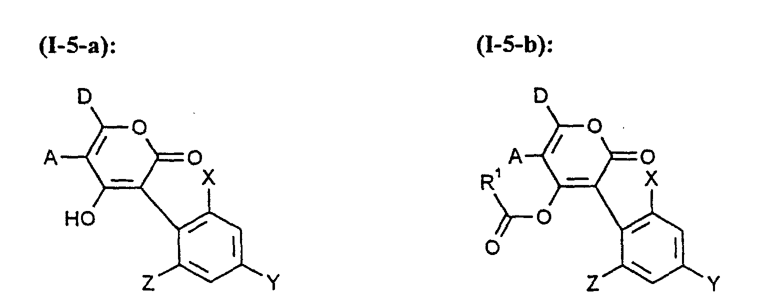 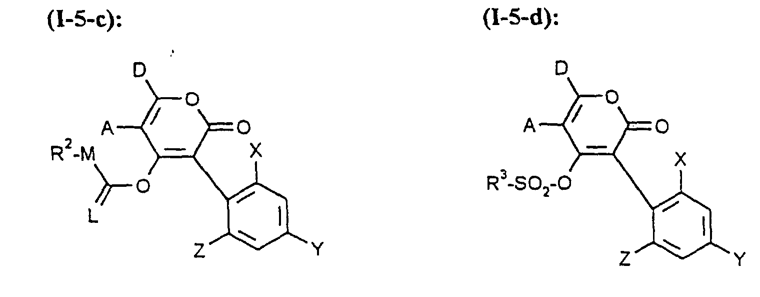 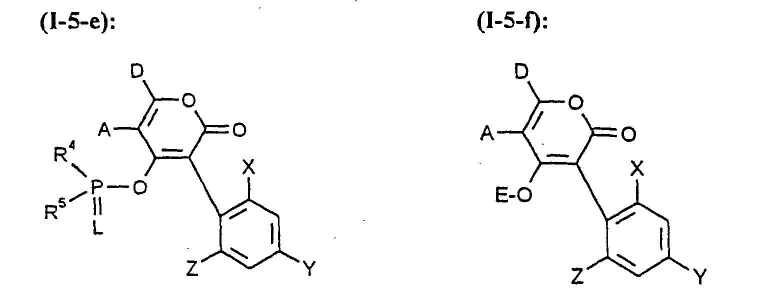 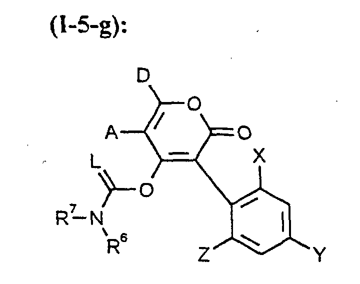 worin
A, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-6-a) bis (I-6-g), wenn Het für die Gruppe (6) steht, 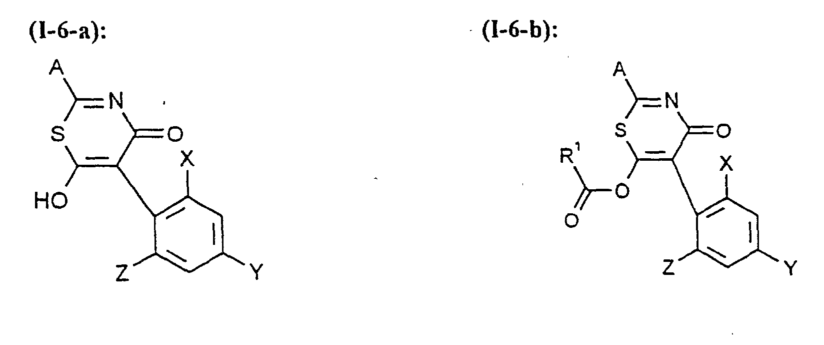 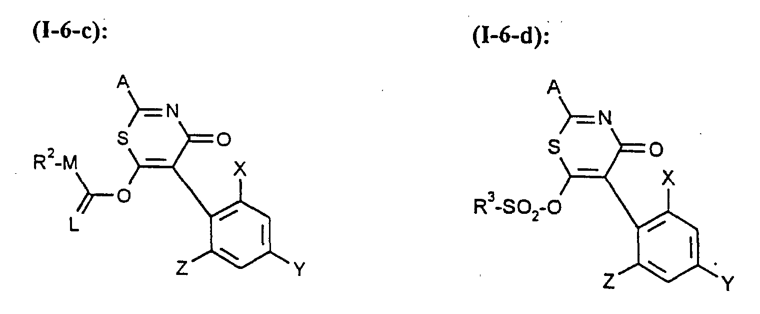 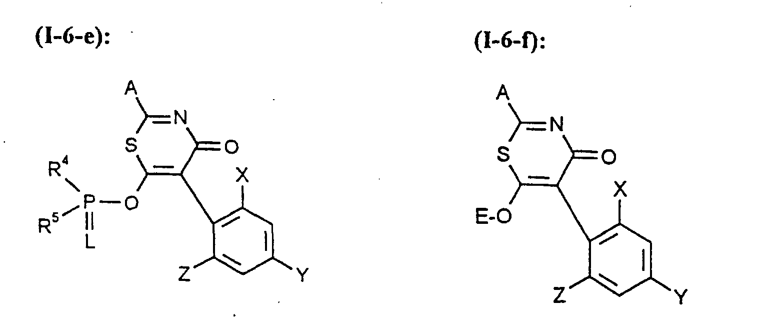 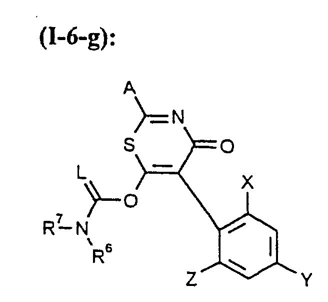 worin
A, E, L, M, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält Verbindungen der Formel (I-1-a) 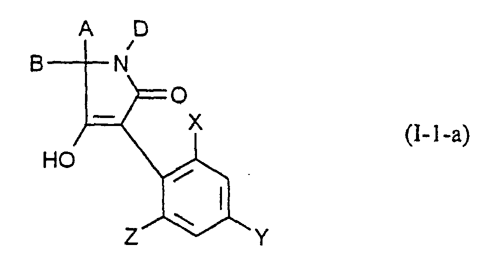 in welcher
   A, B, D, X, Y und Z die oben angegebenen Bedeutungen haben,
   wenn man
   Verbindungen der Formel (II) 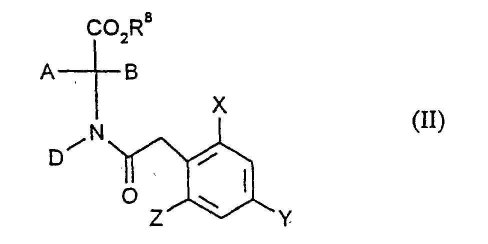 in welcher
   A, B, D, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man Verbindungen der Formel (I-2-a) 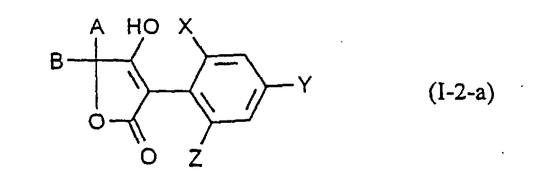 in welcher
   A, B, X, Y und Z die oben angegebenen Bedeutungen haben,
   erhält, wenn man
   Verbindungen der Formel (III) 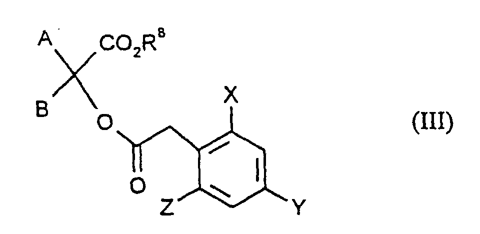 in welcher
   A, B, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, daß man Verbindungen der Formel (I-3-a) 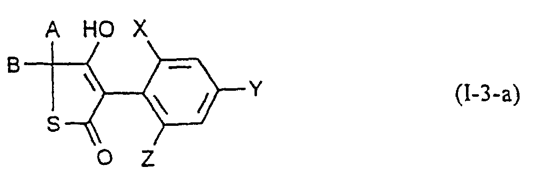 in welcher
   A, B, X, Y und Z die oben angegebenen Bedeutungen haben,
   erhält, wenn man
   Verbindungen der Formel (IV) 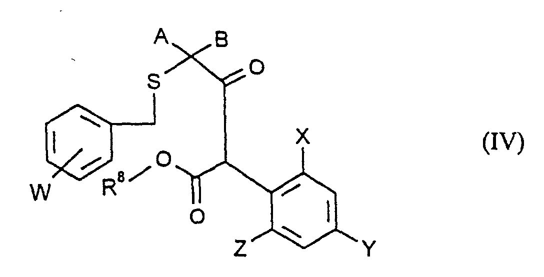 in welcher
   A, B, X, Y, Z und R⁸ die oben angegebenen Bedeutungen haben und
   - W: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(E) Weiterhin wurde gefunden, daß man die Verbindungen der Formel (I-5-a) 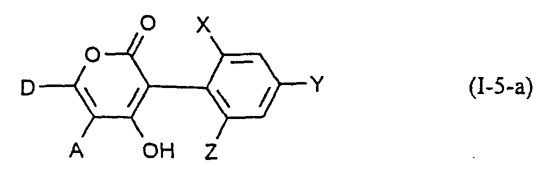 in welcher
   A, D, X, Y und Z die oben angegebenen Bedeutungen haben,
   erhält, wenn man
   Verbindungen der Formel (VIII) 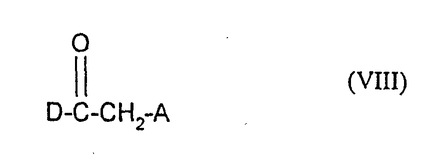 in welcher
   - A und D: die oben angegebenen Bedeutungen haben,
   oder deren Silylenolether der Formel (VIIIa) 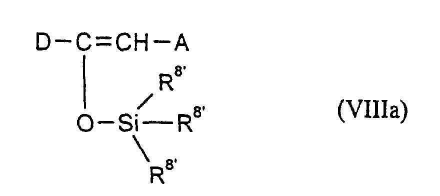 in welcher
   - A und D: die oben genannte Bedeutung haben und R^{8'} für Alkyl (bevorzugt Methyl) steht,
   mit Verbindungen der Formel (V) 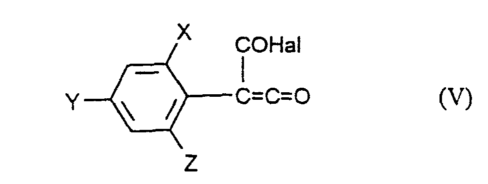 in welcher
   - X, Y und Z: die oben angegebenen Bedeutungen haben und
   - Hal: für Halogen (vorzugsweise für Chlor oder Brom) steht,
   gegebenenfalls in Gegenwart eines Verdünnungsmittels und gegebenenfalls in Gegenwart eines Säureakzeptors umsetzt.
(F) Weiterhin wurde gefunden, daß man die Verbindungen der Formel (I-6-a) 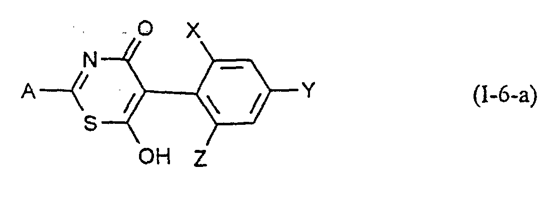 in welcher
   A, X, Y und Z die oben angegebenen Bedeutungen haben,
   erhält, wenn man Verbindungen der Formel (IX) 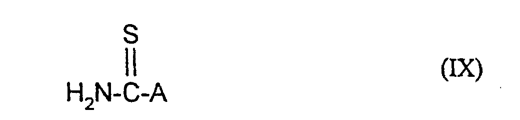 in welcher
   - A: die oben angegebene Bedeutung hat,
   mit Verbindungen der Formel (V) 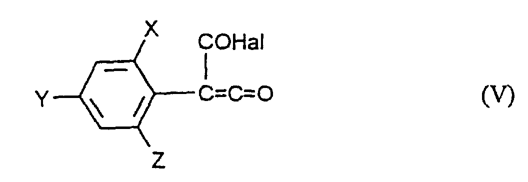 in welcher
   Hal, X, Y und Z die oben angegebenen Bedeutungen haben,
   gegebenenfalls in Gegenwart eines Verdünnungsmittels und gegebenenfalls in Gegenwart eines Säureakzeptors umsetzt.
   Außerdem wurde gefunden,
(G) daß man die Verbindungen der obe gezeigten Formeln (I-1-b) bis (I-3-b), (I-5-b) und (I-6-b), in welchen A, B, D, R¹, X, Y und Z die oben angebenen Bedeutungen haben, erhält, wenn man Verbindungen der oben gezeigten Formeln (I-1-a) bis (I-3-a), (I-5-a) und (I-6-a), in welchen A, B, D, X, Y und Z die oben angegebenen Bedeutungen haben, und daß man Verbindungen der oben gezeigten Formel (I-4-b), in welcher A, D, R¹, X, Y und Z die oben angegebene Bedeutung haben, erhält, wenn man Verbindungen der Formel (I-4-a) 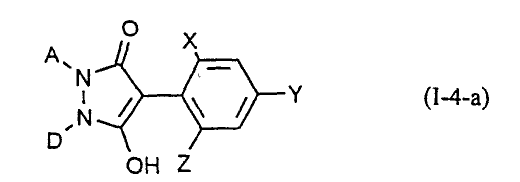 in welcher
   A, D, X, Y und Z die oben angegebene Bedeutung haben, jeweils
   α) mit Säurehalogeniden der Formel (X) 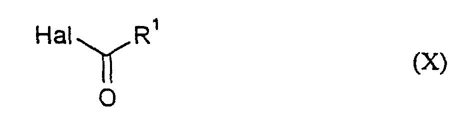 in welcher
      - R¹: die oben angegebene Bedeutung hat und
      - Hal: für Halogen (insbesondere Chlor oder Brom) steht
      oder
   β) mit Carbonsäureanhydriden der Formel (XI)

      R¹-CO-O-CO-R¹ (XI)
   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(H) daß man die Verbindungen der oben gezeigten Formeln (I-1-c) bis (I-6-c), in welchen A, B, D, R², M, X, Y und Z die oben angegebenen Bedeutungen haben und L für Sauerstoff steht, erhält, wenn man Verbindungen der oben gezeigten Formeln (I-1-a) bis (I-6-a), in welchen A, B, D, X, Y und Z die oben angegebenen Bedeutungen haben, jeweils
   mit Chlorameisensäureestern oder Chlorameisensäurethioestern der Formel (XII)

   R²-M-CO-Cl (XII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(I) daß man Verbindungen der oben gezeigten Formeln (I-1-c) bis (I-6-c), in welchen A, B, D, R², M, X, Y und Z die oben angegebenen Bedeutungen haben und L für Schwefel steht, erhält, wenn man Verbindungen der oben gezeigten Formeln (I-1-a) bis (I-6-a), in welchen A, B, D, X, Y und Z die oben angegebenen Bedeutungen haben, jeweils
   α) mit Chlormonothioameisensäureestern oder Chlordithioameisensäureestern der Formel (XIII) 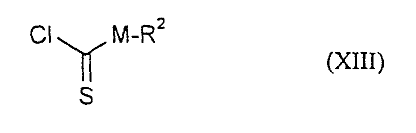 in welcher
      - M und R²: die oben angegebenen Bedeutungen haben,
      gegebenenfalls in Gegenwart eines Verdünnungsmittels und gegebenenfalls in Gegenwart eines Säurebindemittels umsetzt
      oder
   β) mit Schwefelkohlenstoff und anschließend mit Verbindungen der Formel (XIV)

      R²-Hal (XIV)
   in welcher
   - R²: die oben angegebene Bedeutung hat und
   - Hal: für Chlor, Brom oder Iod steht,
   gegebenenfalls in Gegenwart eines Verdünnungsmittels und gegebenenfalls in Gegenwart einer Base umsetzt,
(J) daß man Verbindungen der oben gezeigten Formeln (I-1-d) bis (I-6-d), in welchen A, B, D, R³, X, Y und Z die oben angegebenen Bedeutungen haben, erhält, wenn man Verbindungen der oben gezeigten Formeln (I-1-a) bis (I-6-a), in welchen A, B, D, X, Y und Z die oben angegebenen Bedeutungen haben, jeweils
   mit Sulfonsäurechloriden der Formel (XV)

   R³-SO₂-Cl (XV)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(K) daß man Verbindungen der oben gezeigten Formeln (I-1-e) bis (I-6-e), in welchen A, B, D, L, R⁴, R⁵, X, Y und Z die oben angegebenen Bedeutungen haben, erhält, wenn man Verbindungen der oben gezeigten Formeln (I-1-a) bis (I-6-a), in welchen A, B, D, X, Y und Z die oben angegebenen Bedeutungen haben, jeweils
   mit Phosphorverbindungen der Formel (XVI) 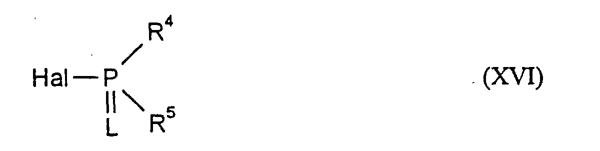 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(L) daß man Verbindungen der oben gezeigten Formeln (I-1-f) bis (I-6-f), in welchen A, B, D, E, X, Y und Z die oben angegebenen Bedeutungen haben, erhält, wenn man Verbindungen der Formeln (I-1-a) bis (I-6-a), in welchen A, B, D, X, Y und Z die oben angegebenen Bedeutungen haben, jeweils
   mit Metallverbindungen oder Aminen der Formeln (XVII) oder (XVIII)

   Me(OR¹⁰)ₜ (XVII)

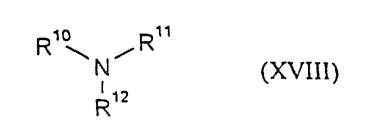

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(M) daß man Verbindungen der oben gezeigten Formeln (I-1-g) bis (I-6-g), in welchen A, B, D, L, R⁶, R⁷, X, Y und Z die oben angegebenen Bedeutungen haben, erhält, wenn man Verbindungen der oben gezeigten Formeln (I-1-a) bis (I-6-a), in welchen A, B, D, X, Y und Z die oben angegebenen Bedeutungen haben, jeweils
   α) mit Isocyanaten oder Isothiocyanaten der Formel (XIX)

      R⁶-N=C=L (XIX)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XX) 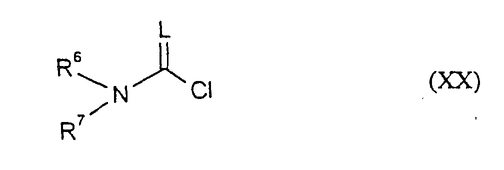 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

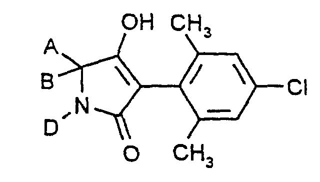

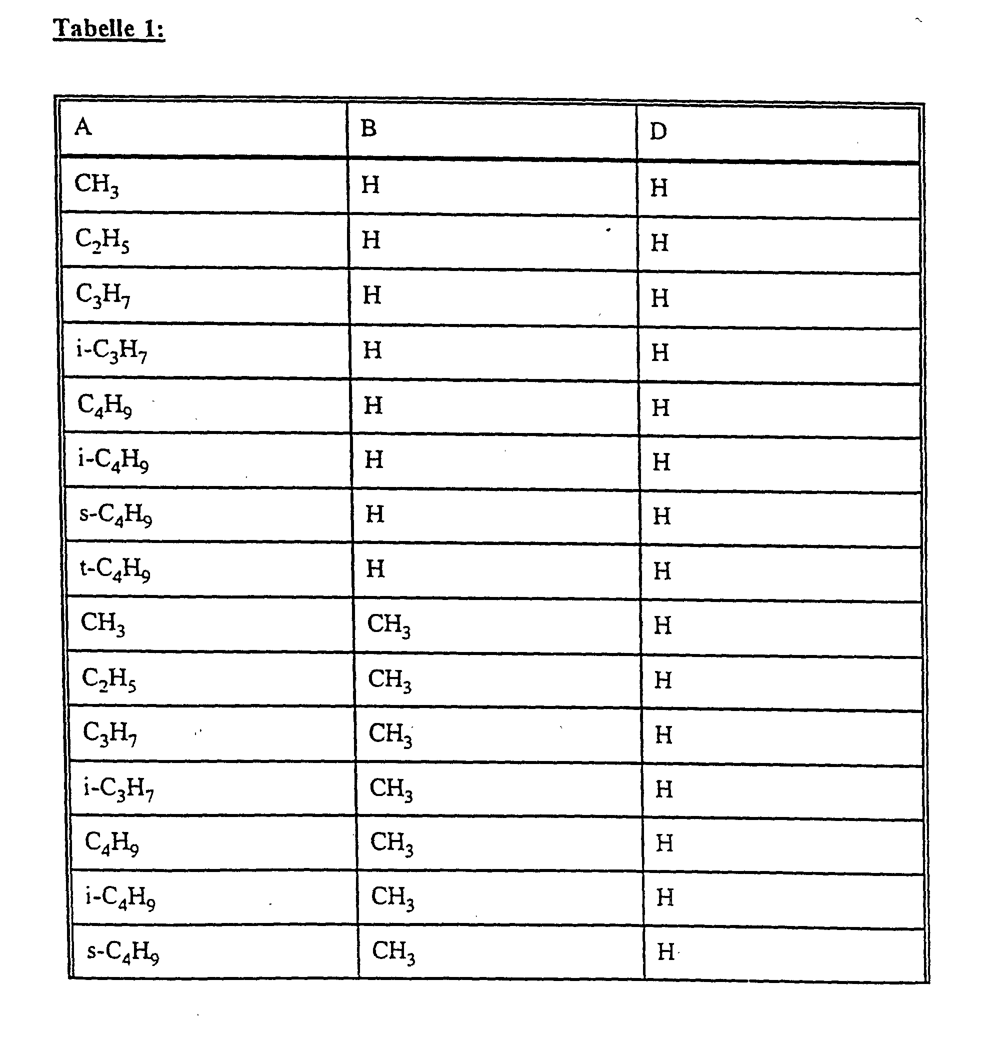

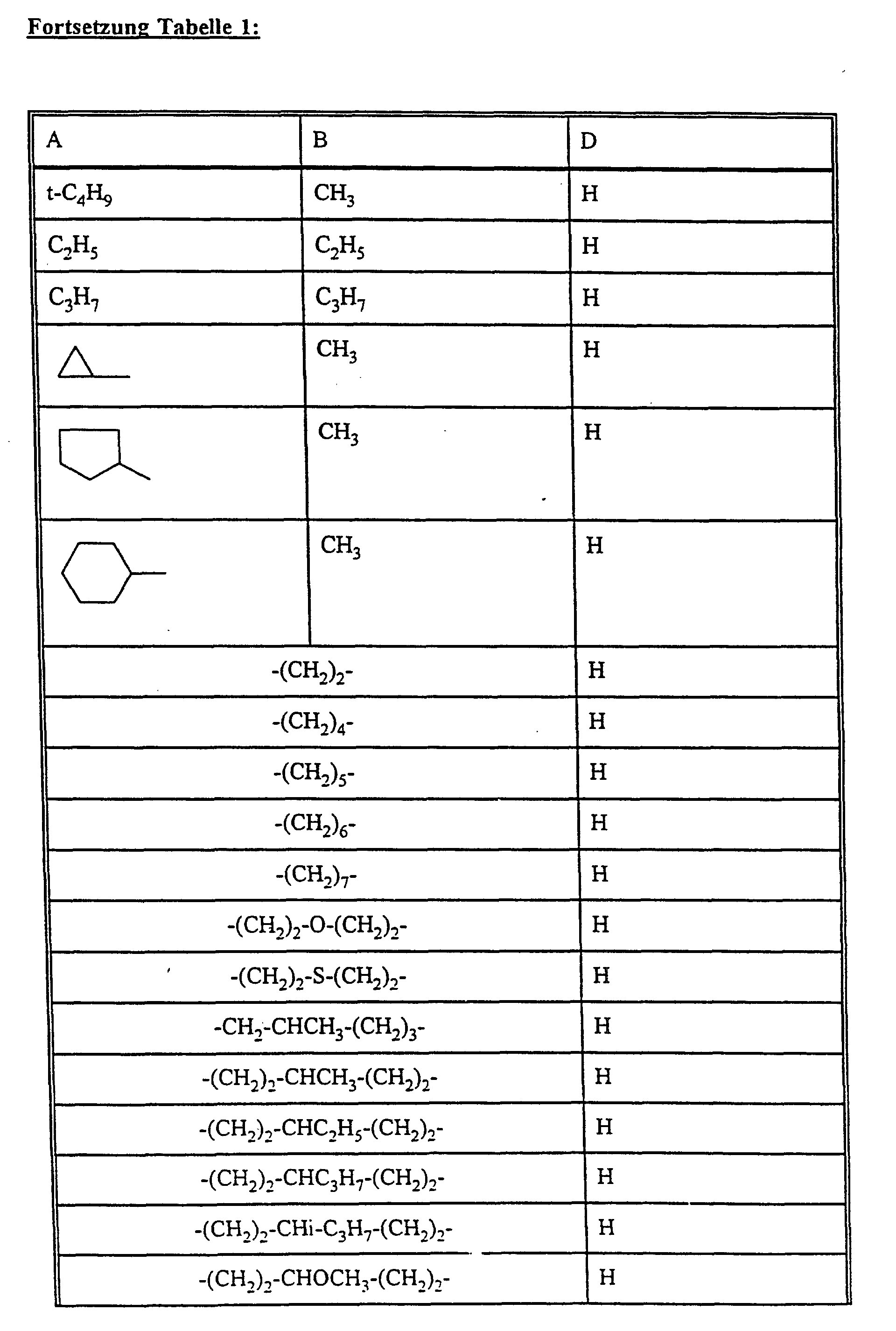

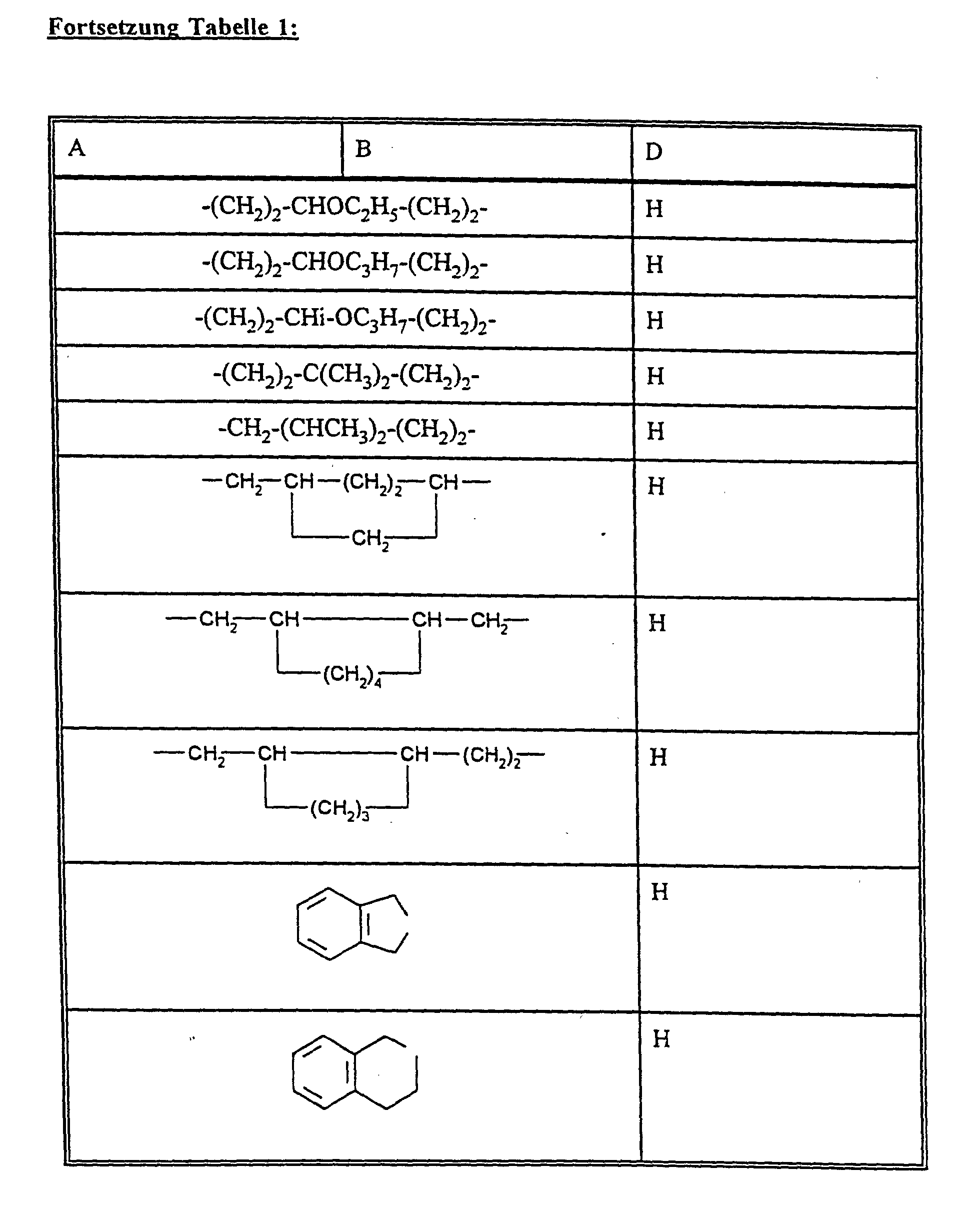

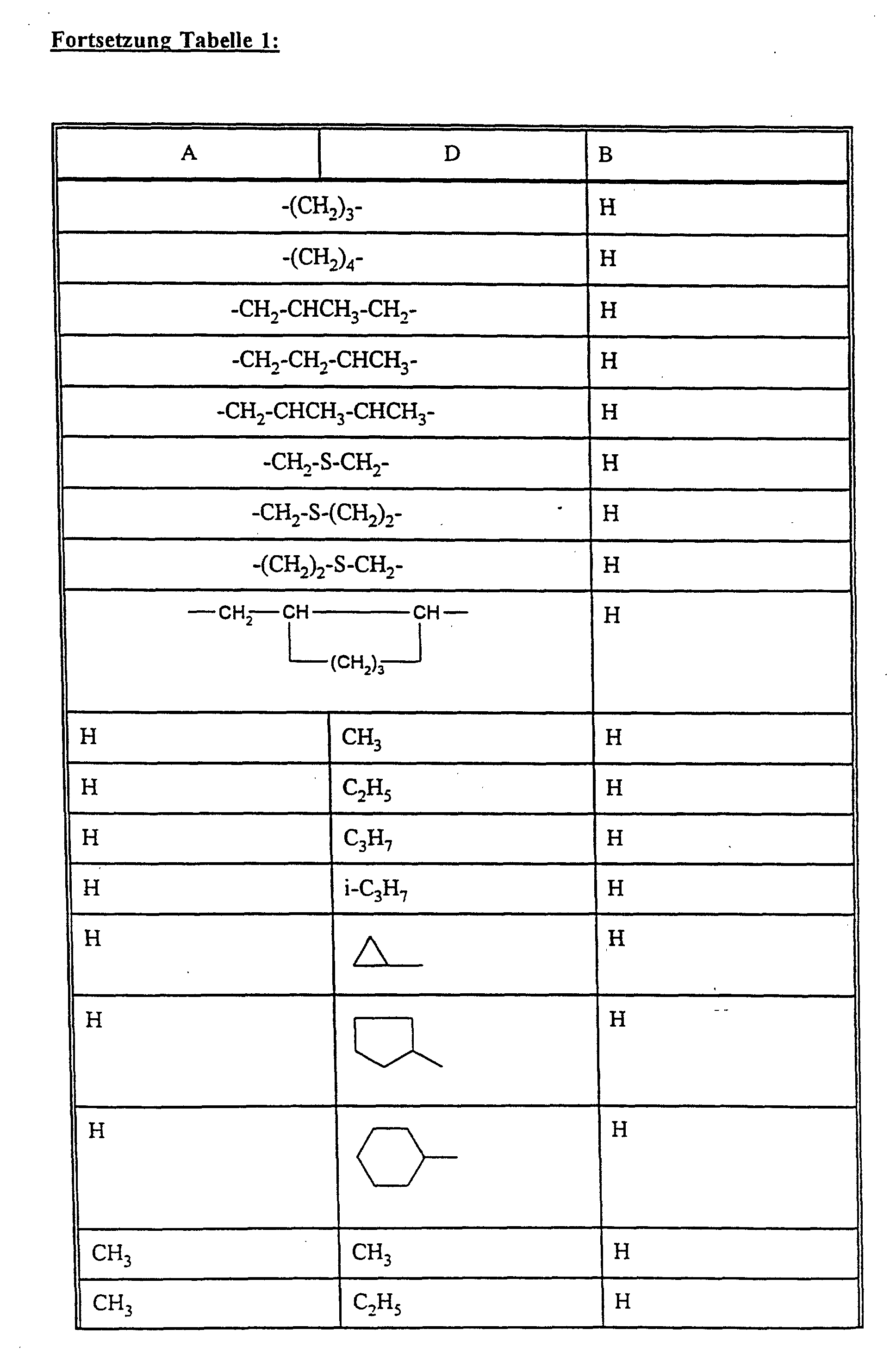

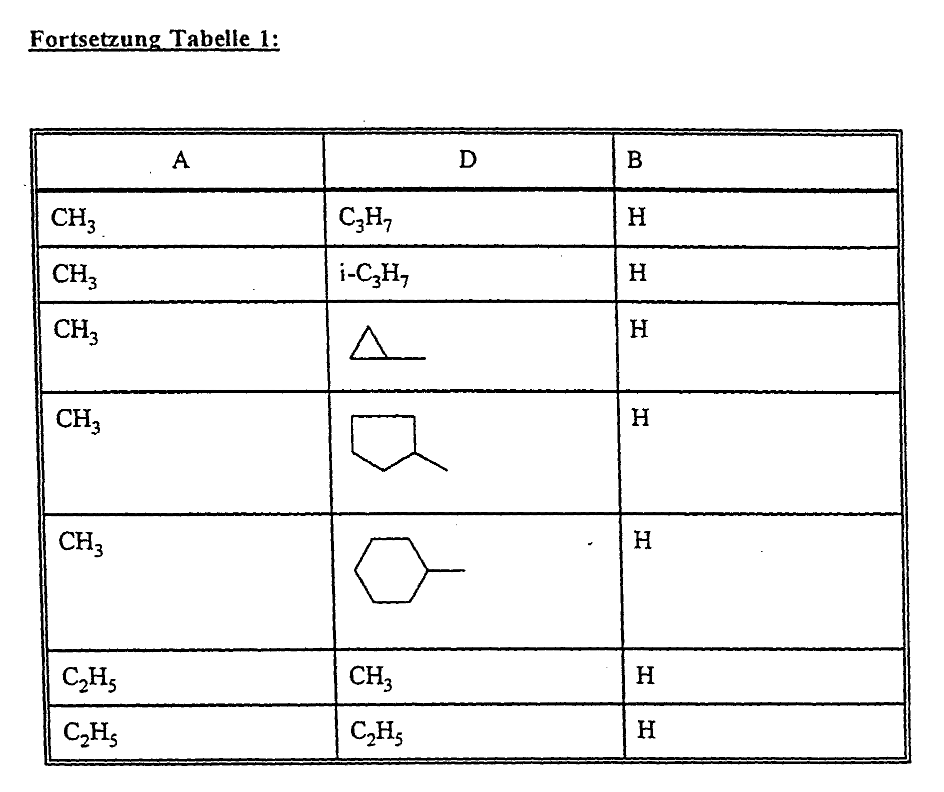

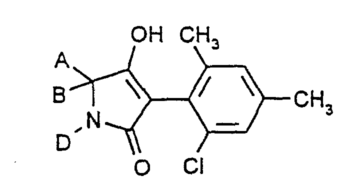

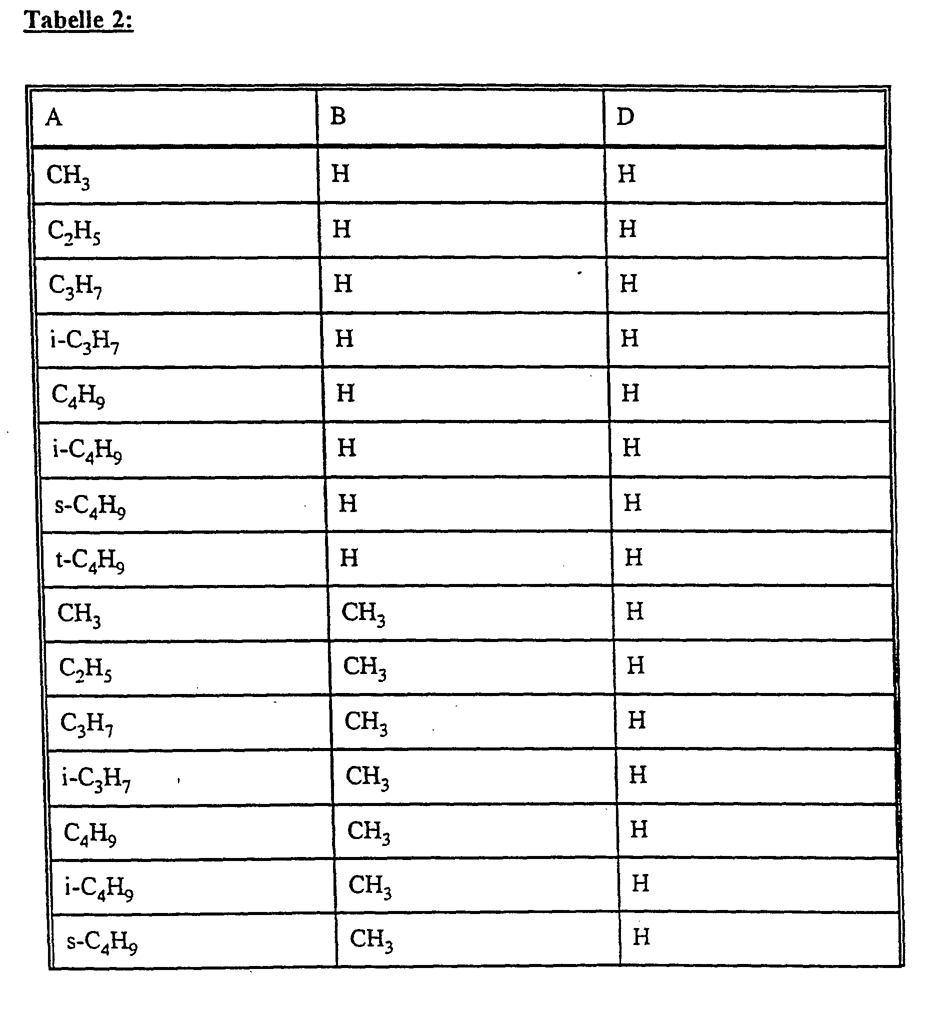

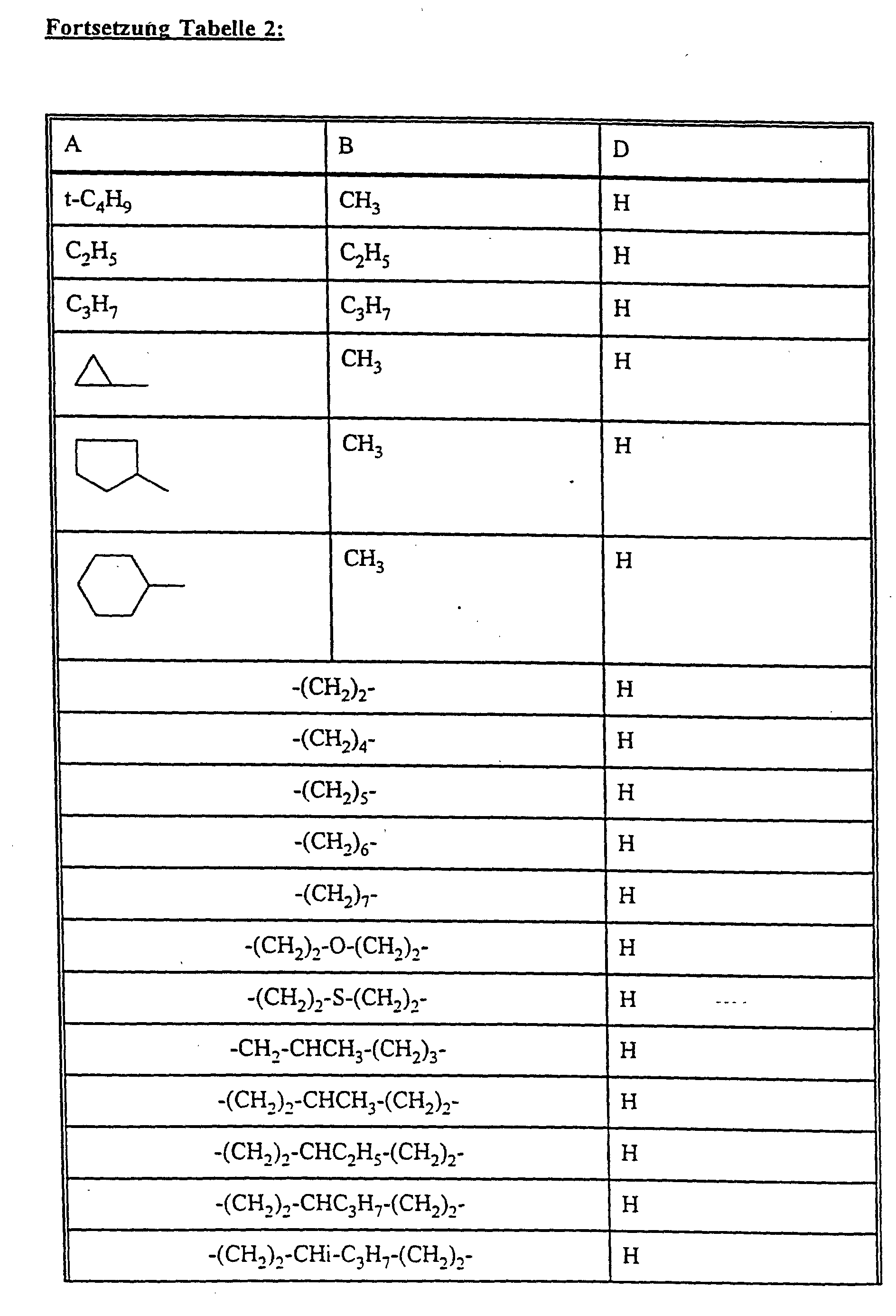

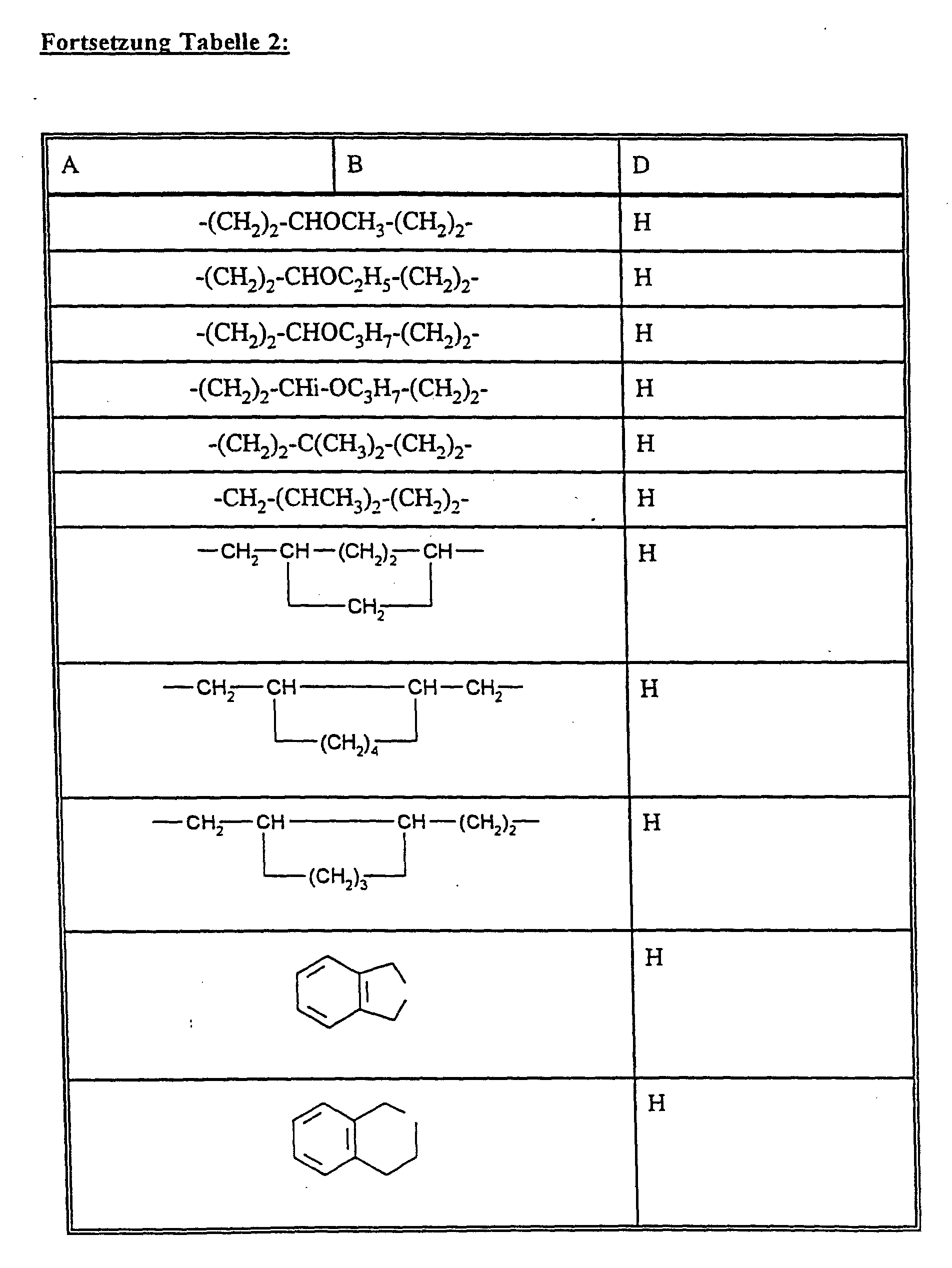

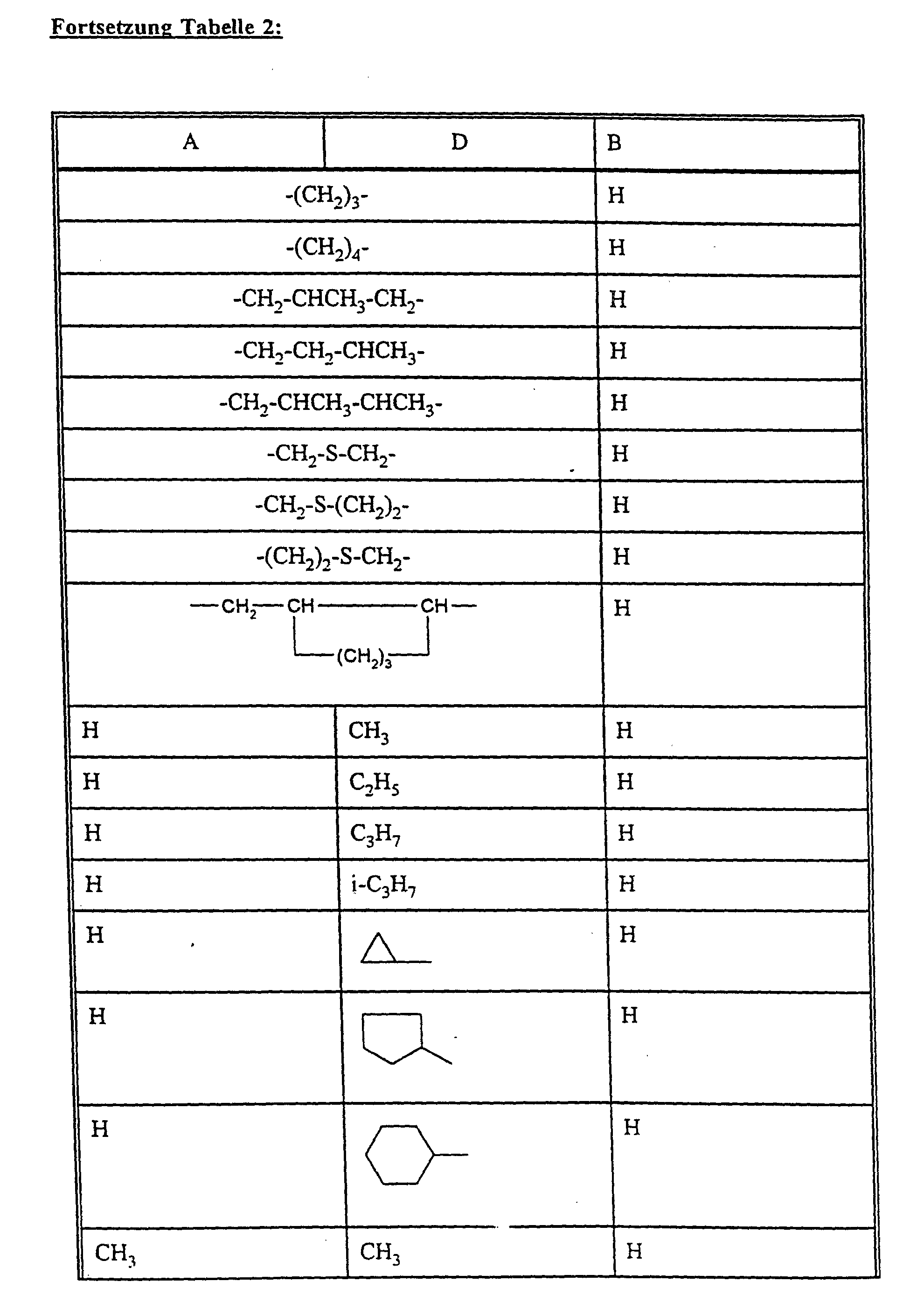

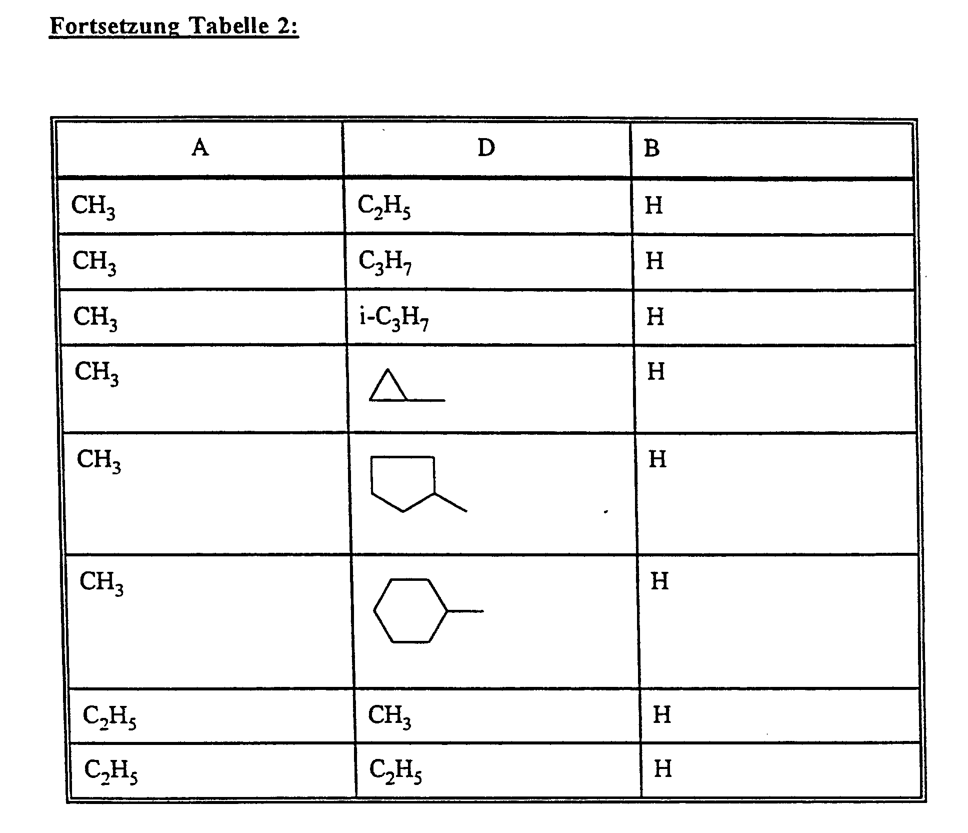

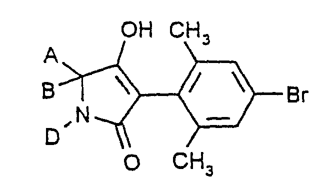

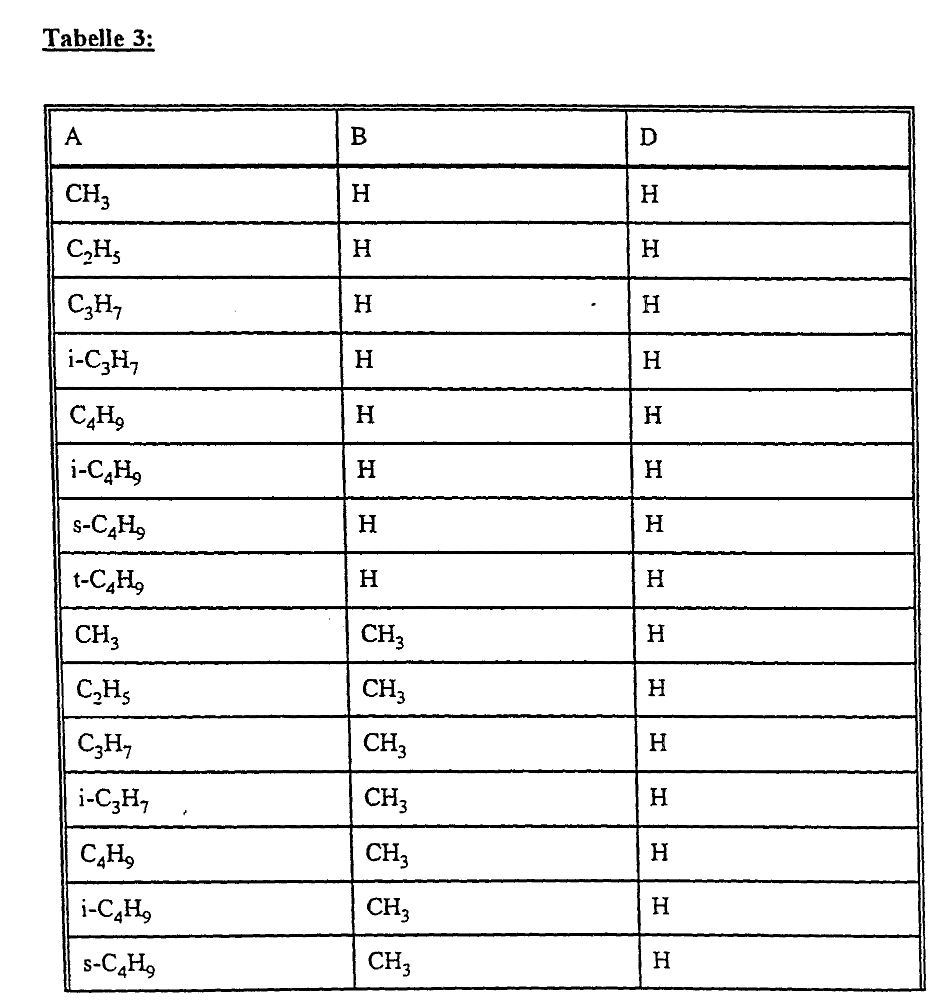

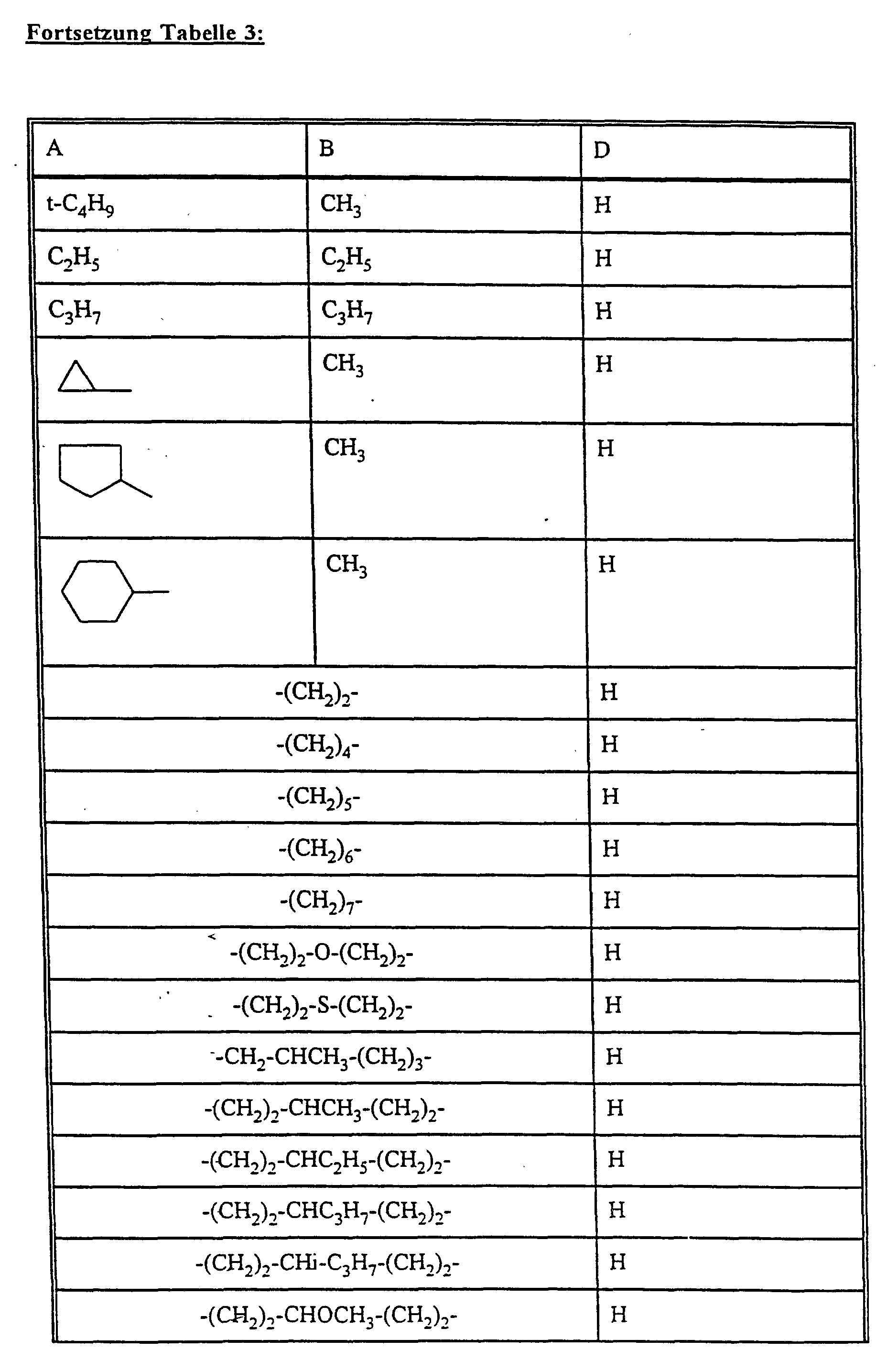

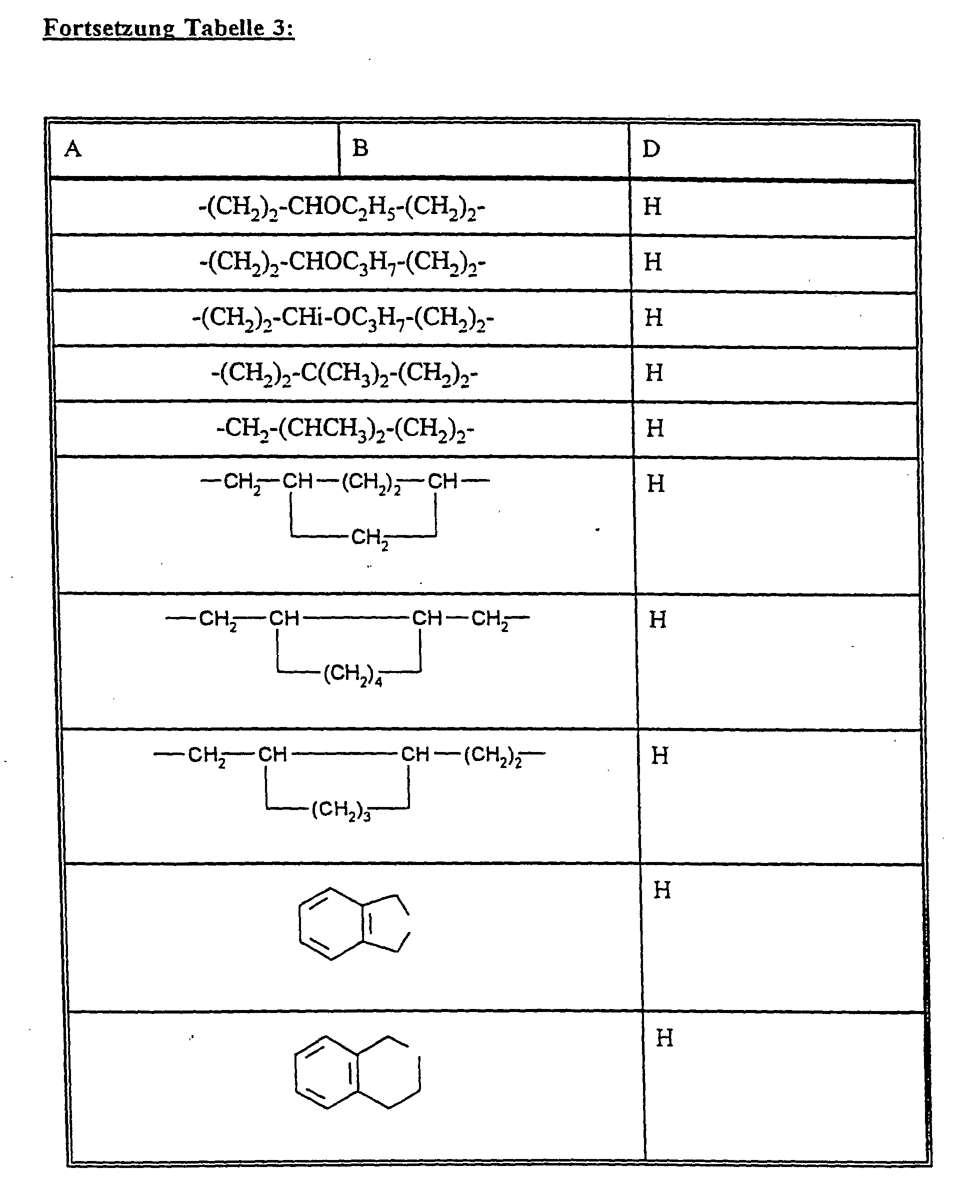

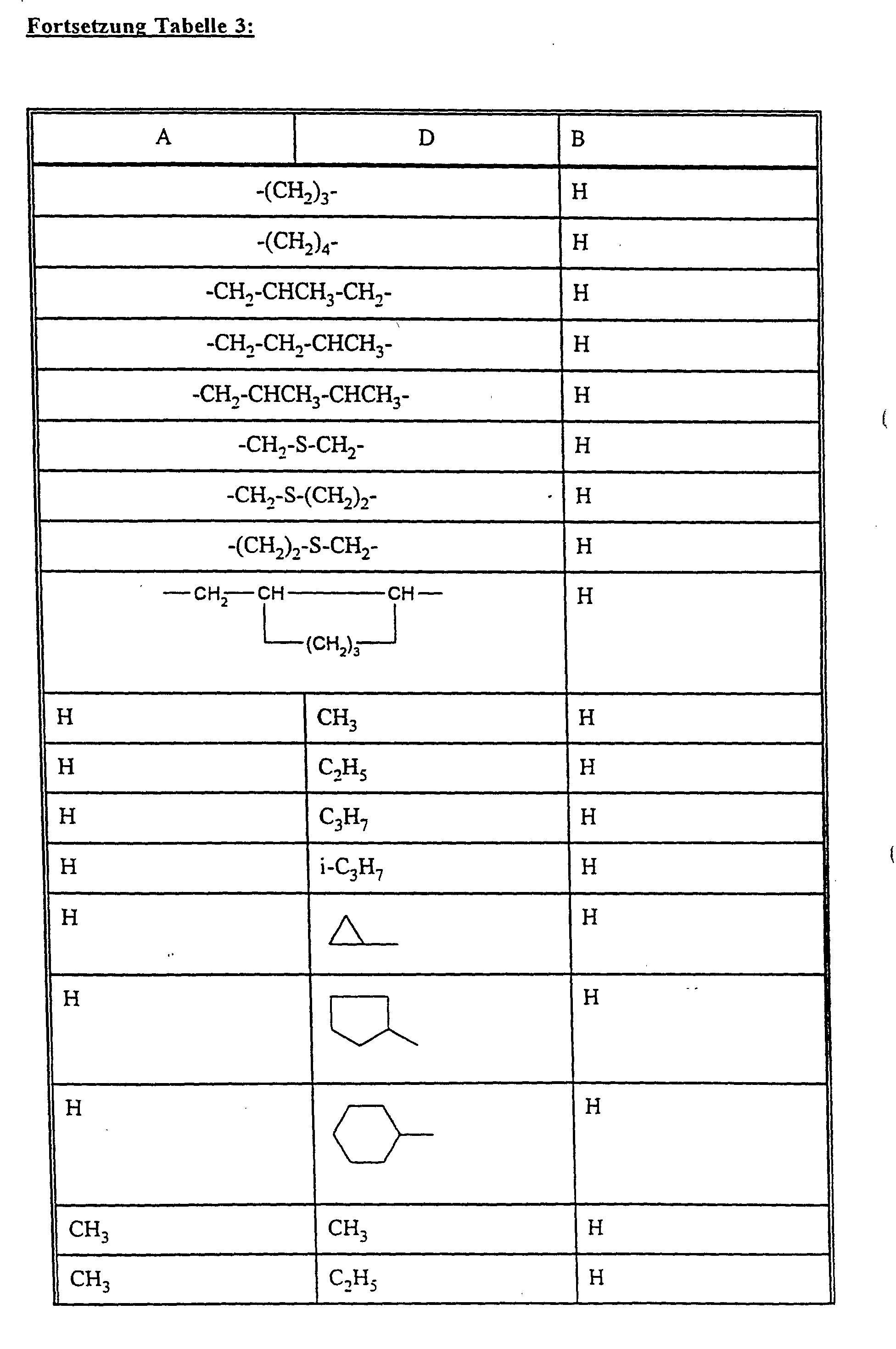

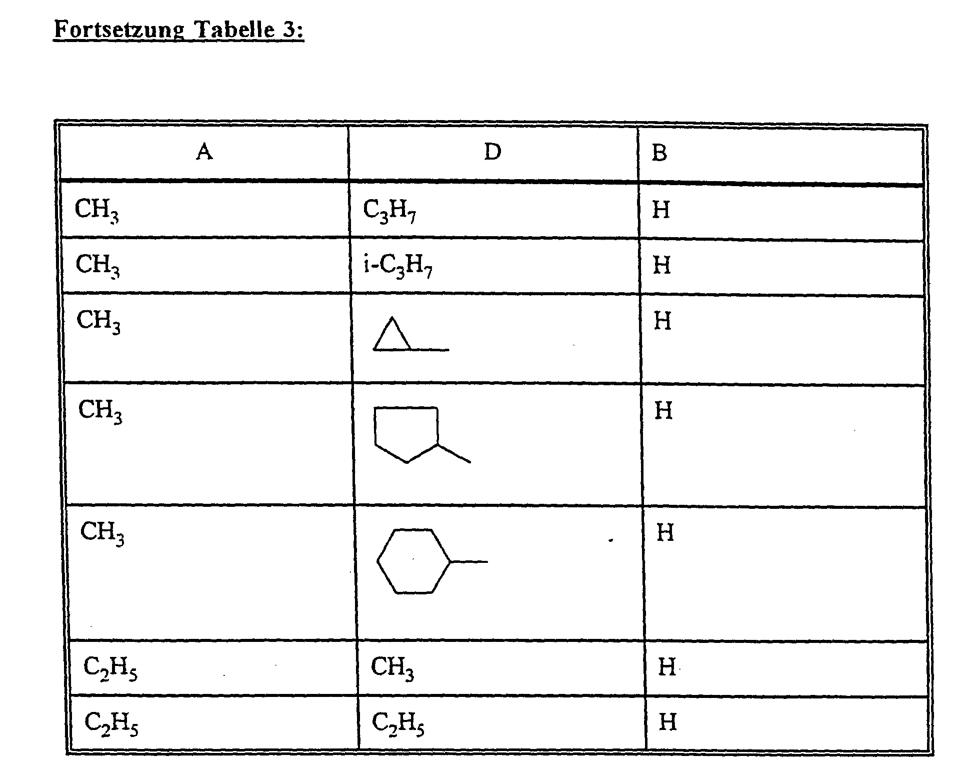

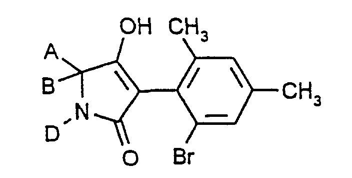

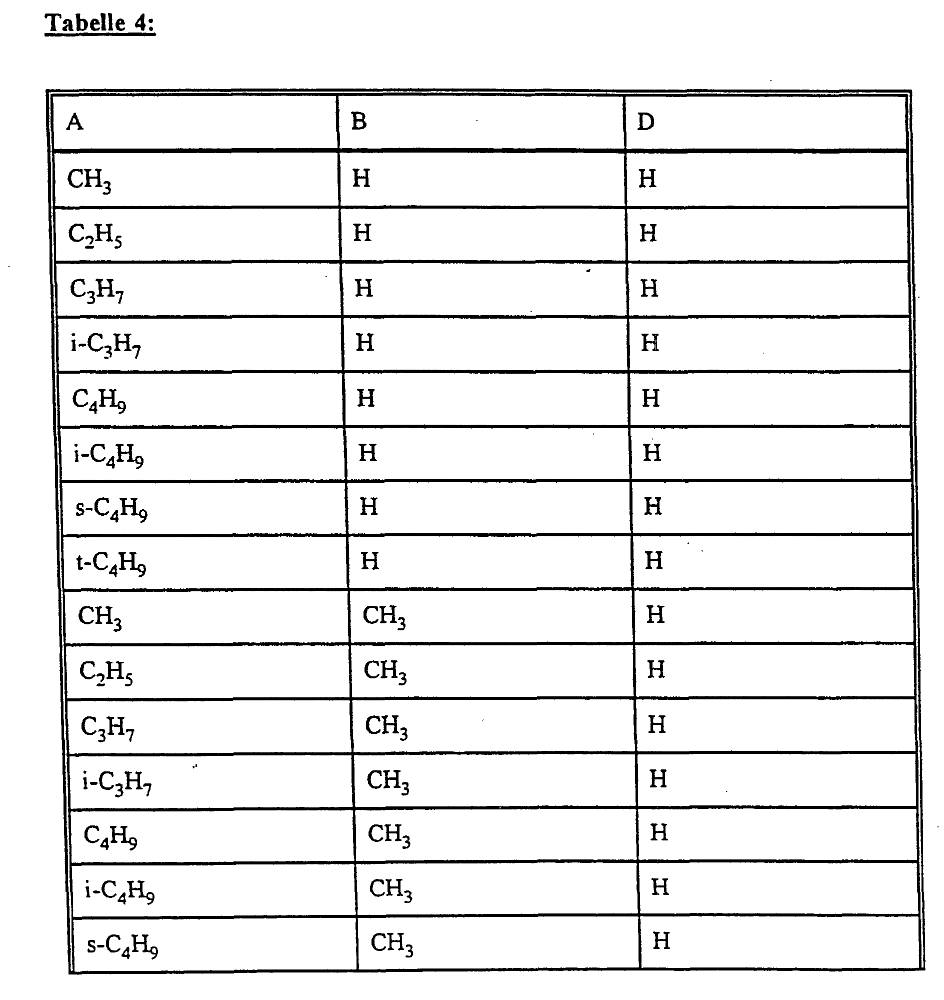

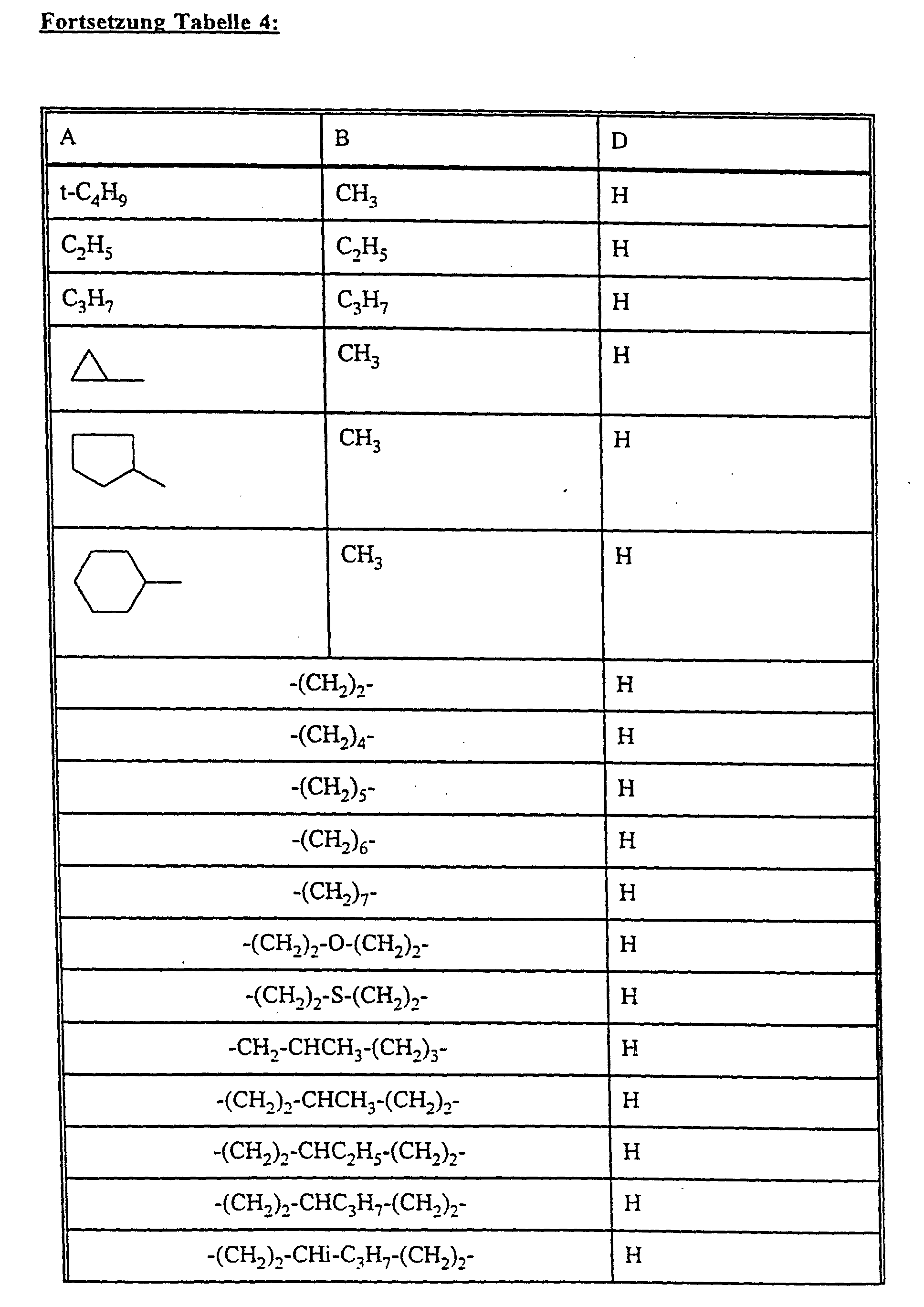

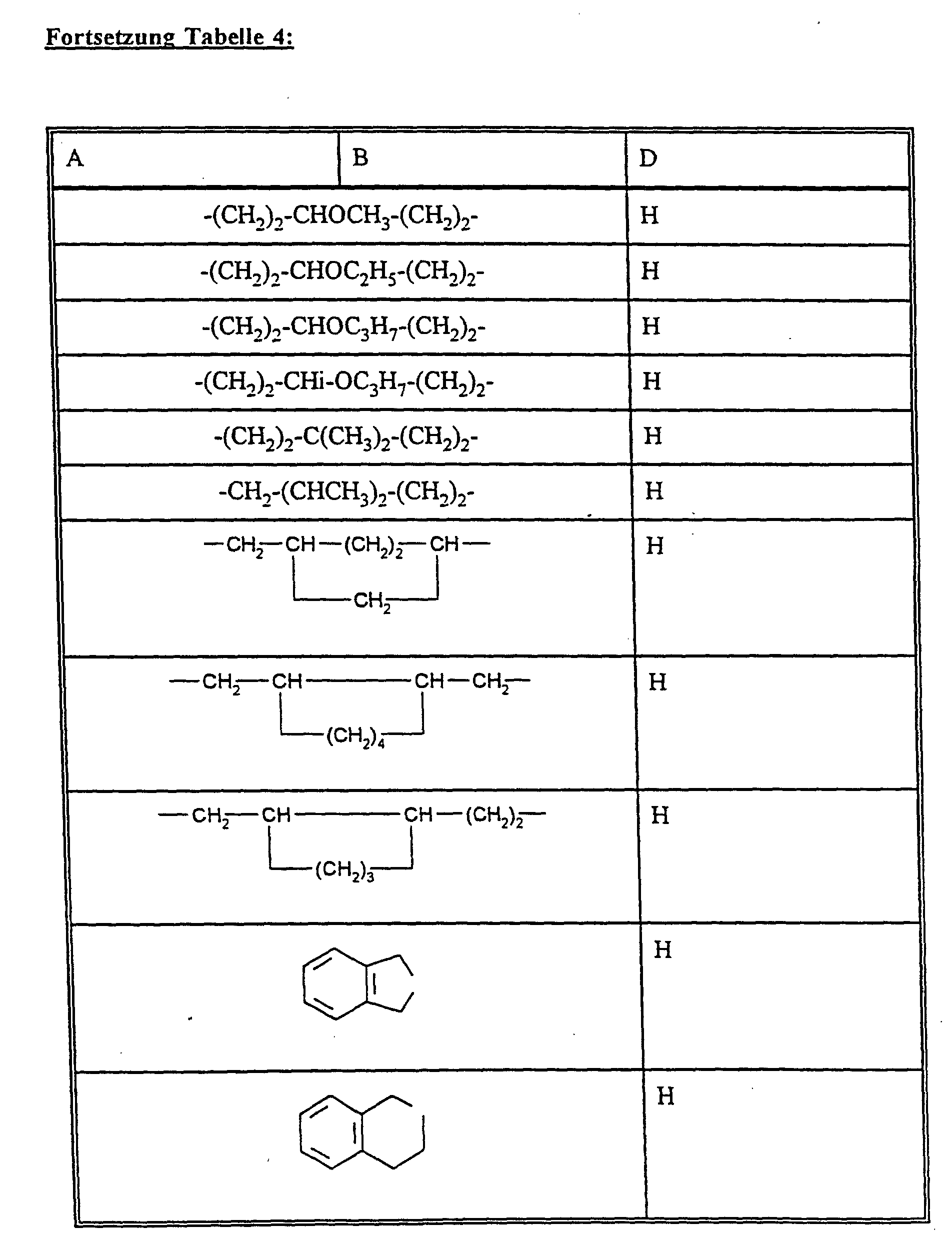

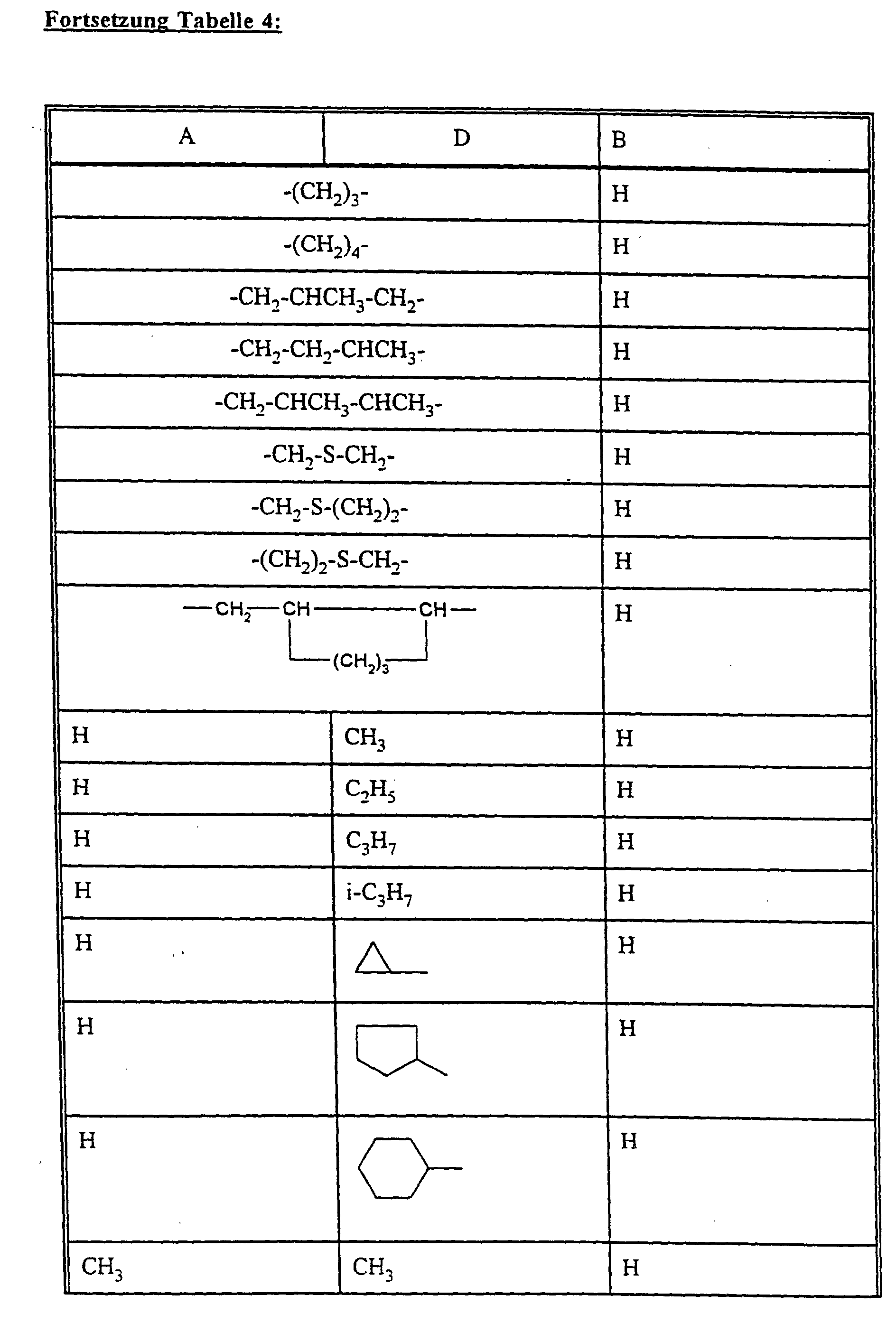

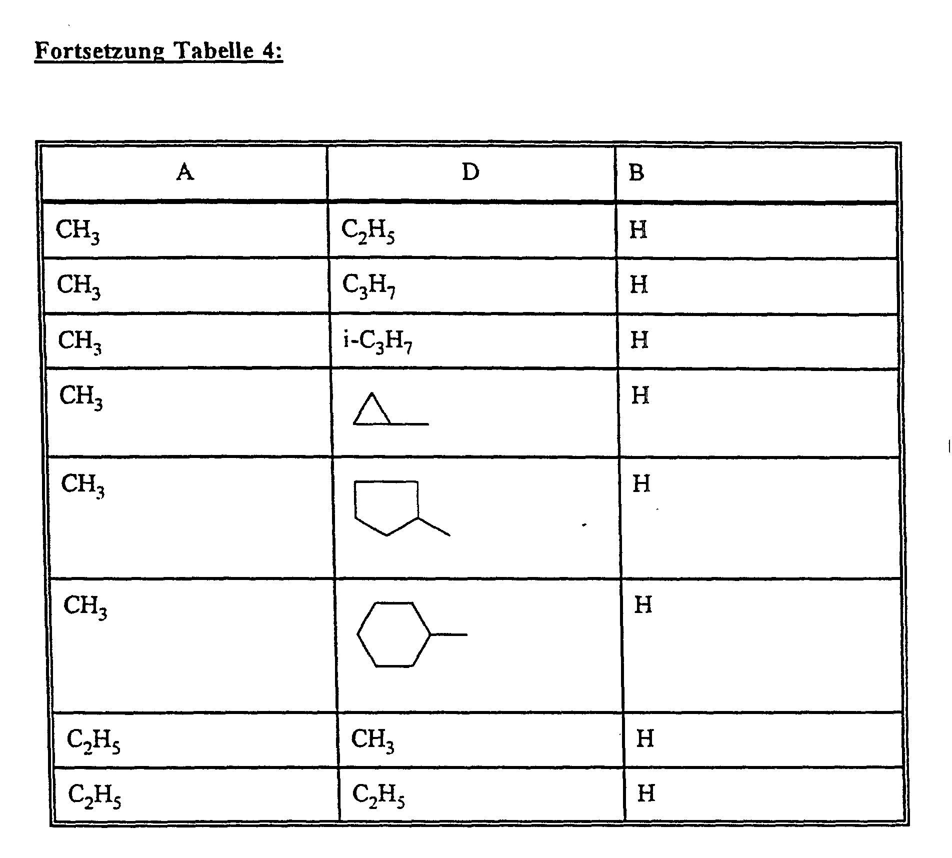

Im einzelnen seien außer den bei den Herstellungsbeispielen genannten Verbindungen die folgenden Verbindungen der Formel (I-2-a) genannt:

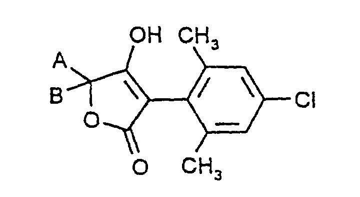

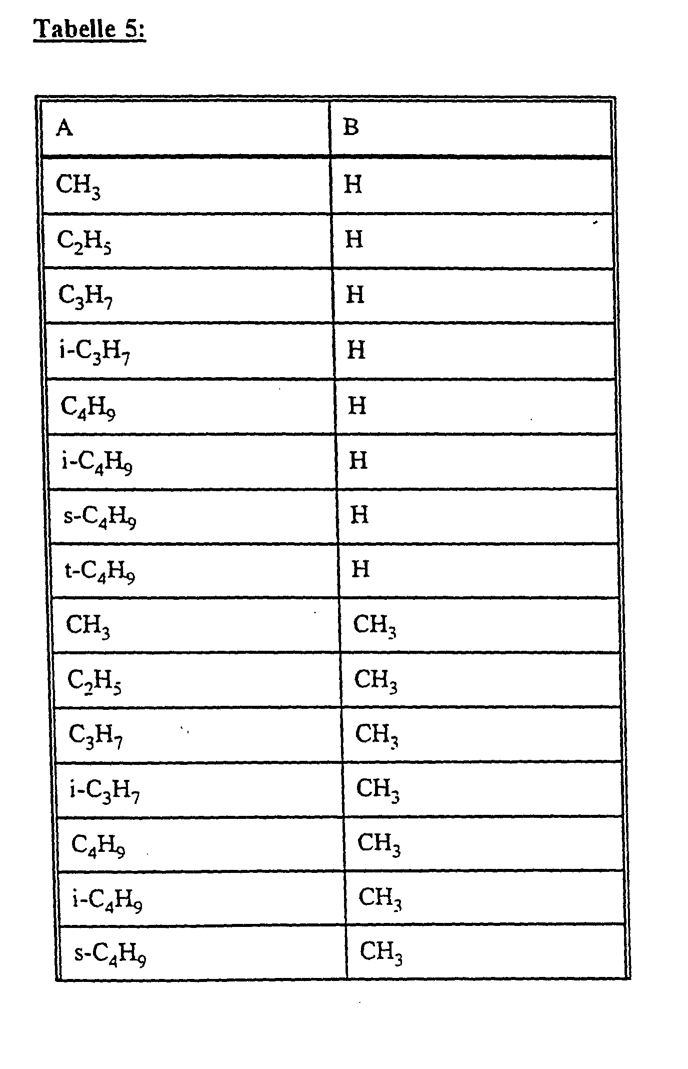

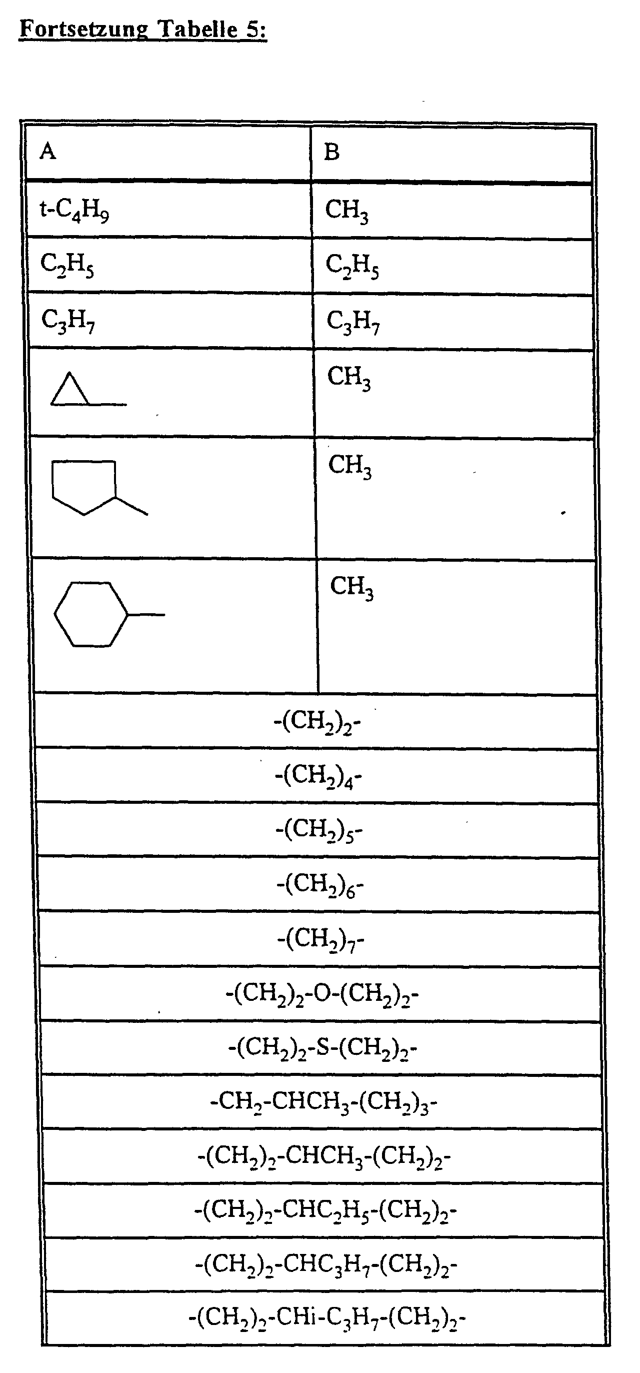

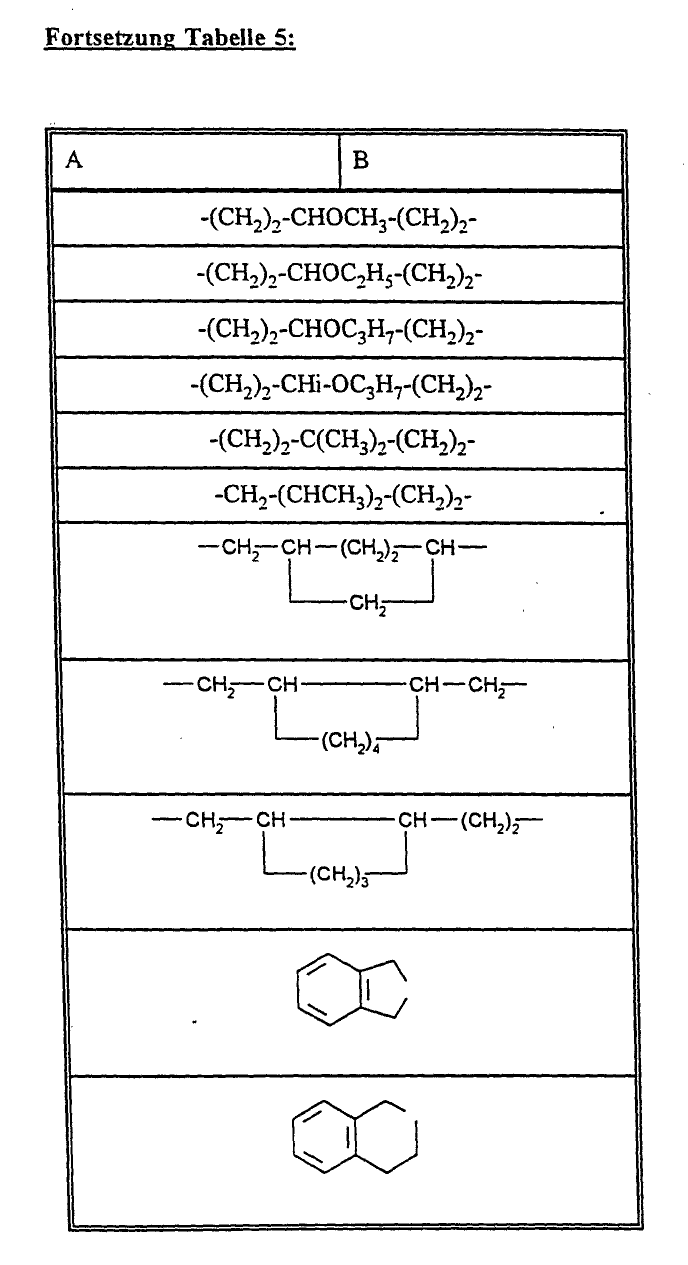

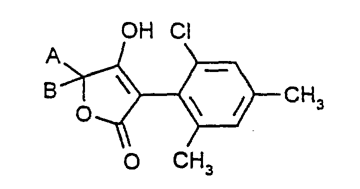

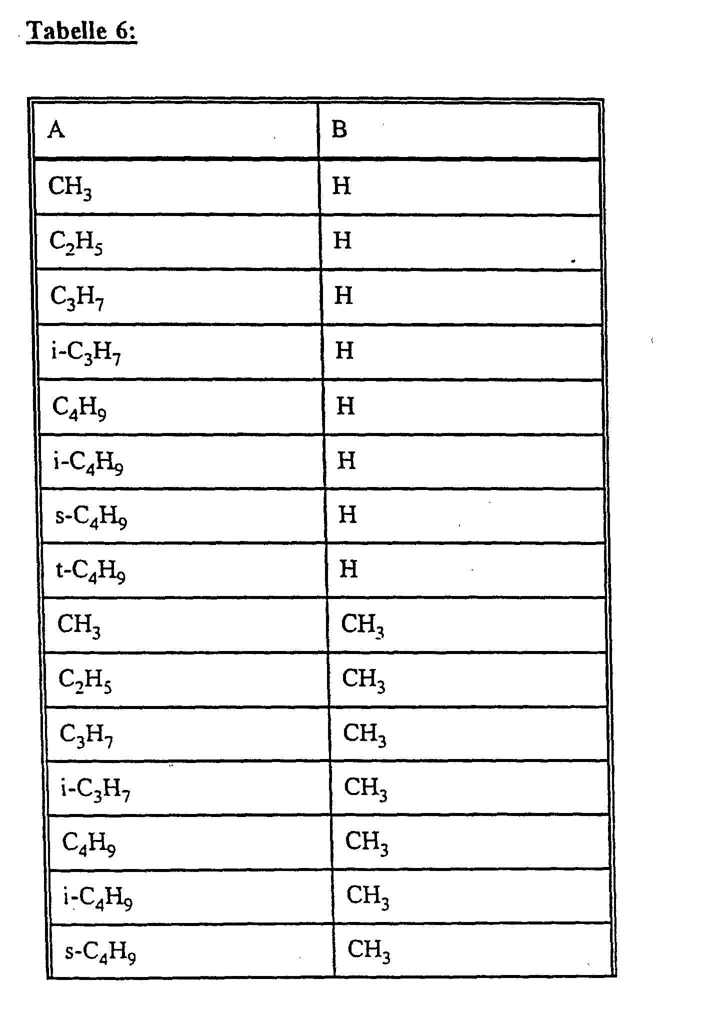

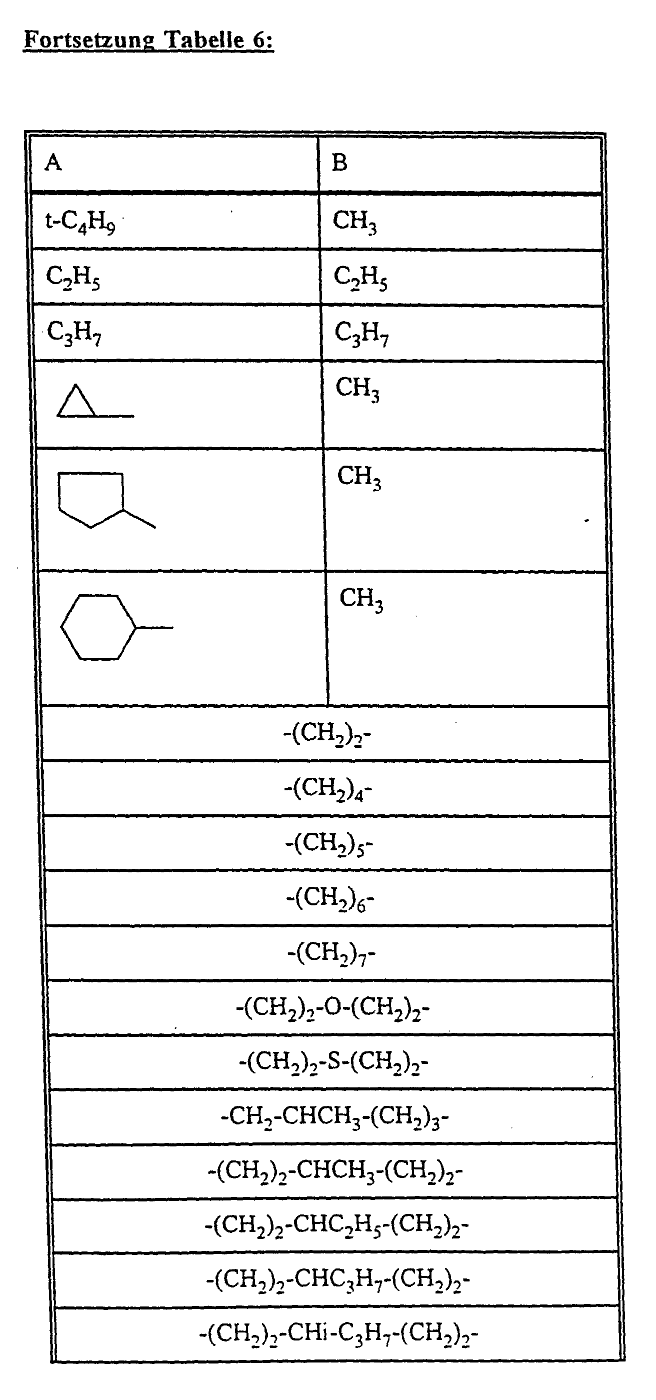

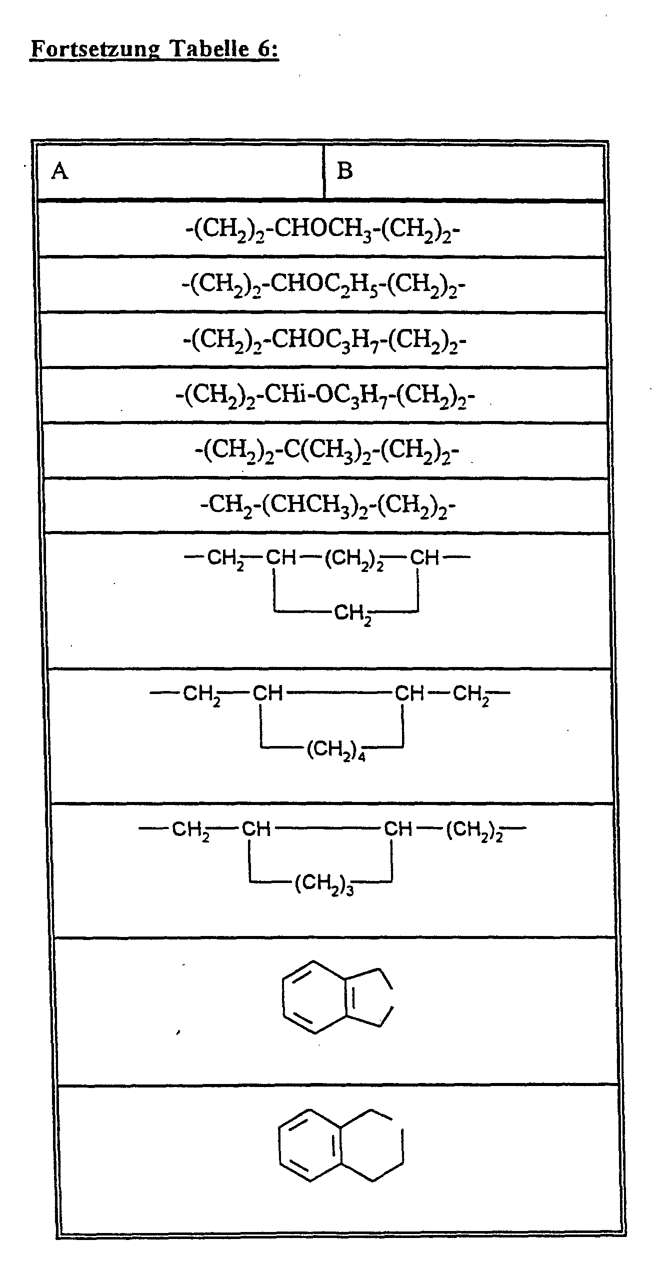

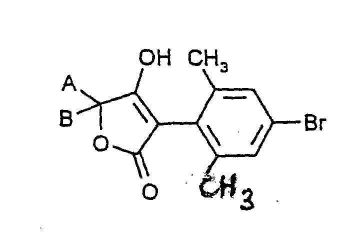

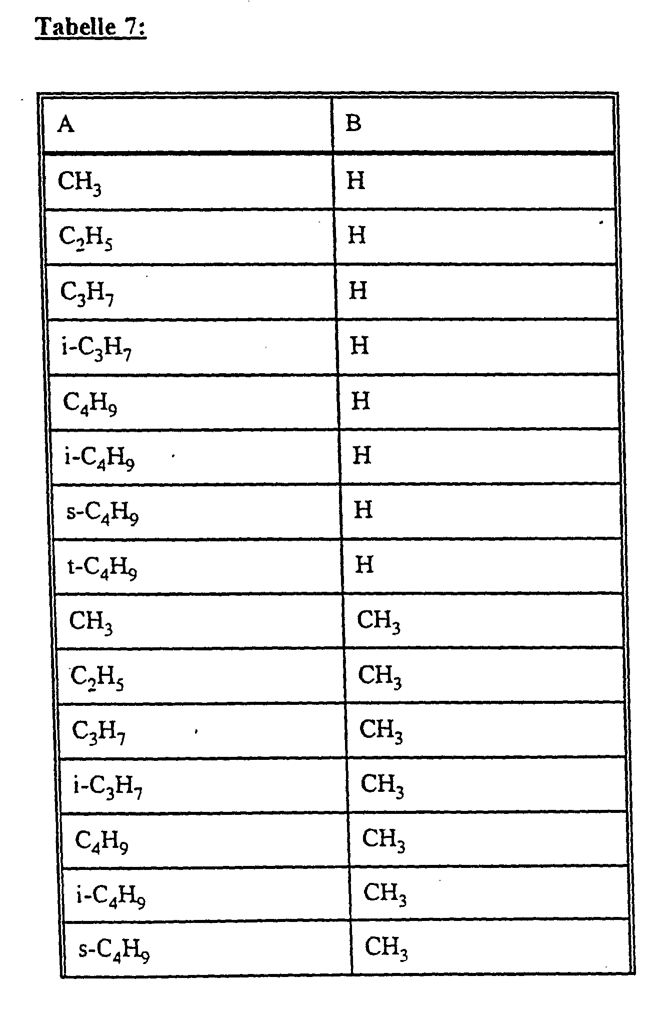

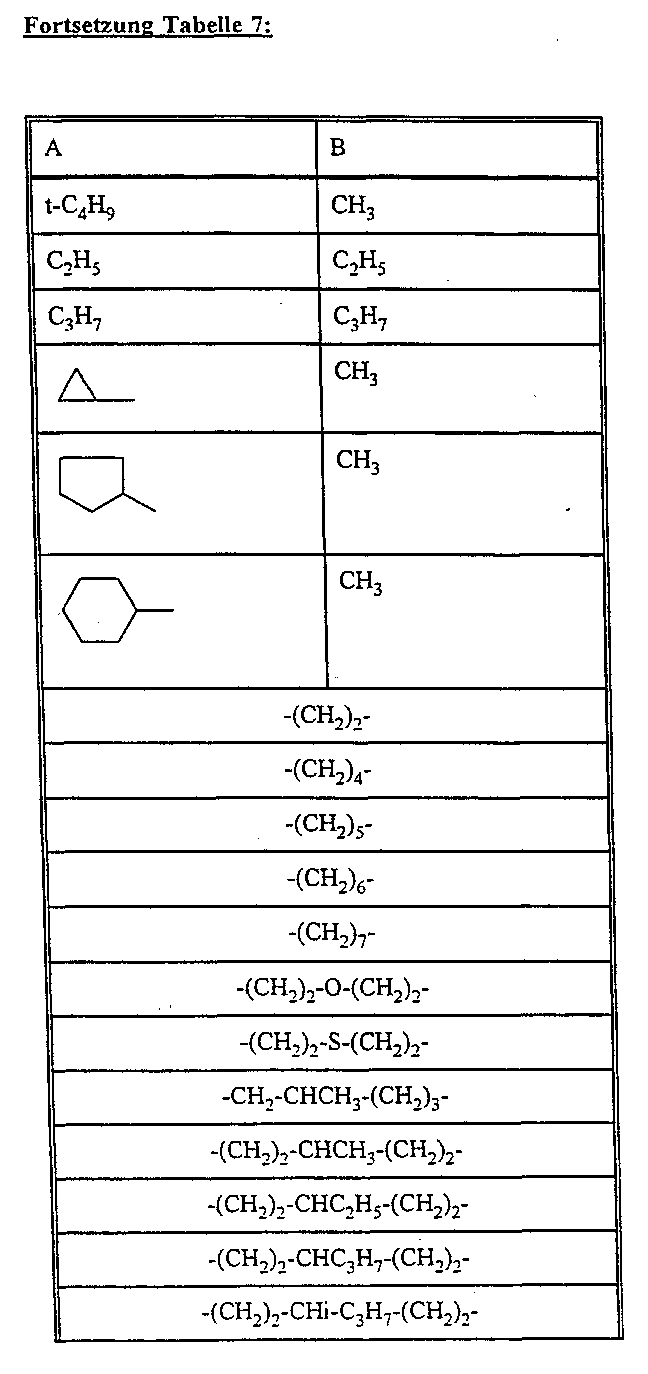

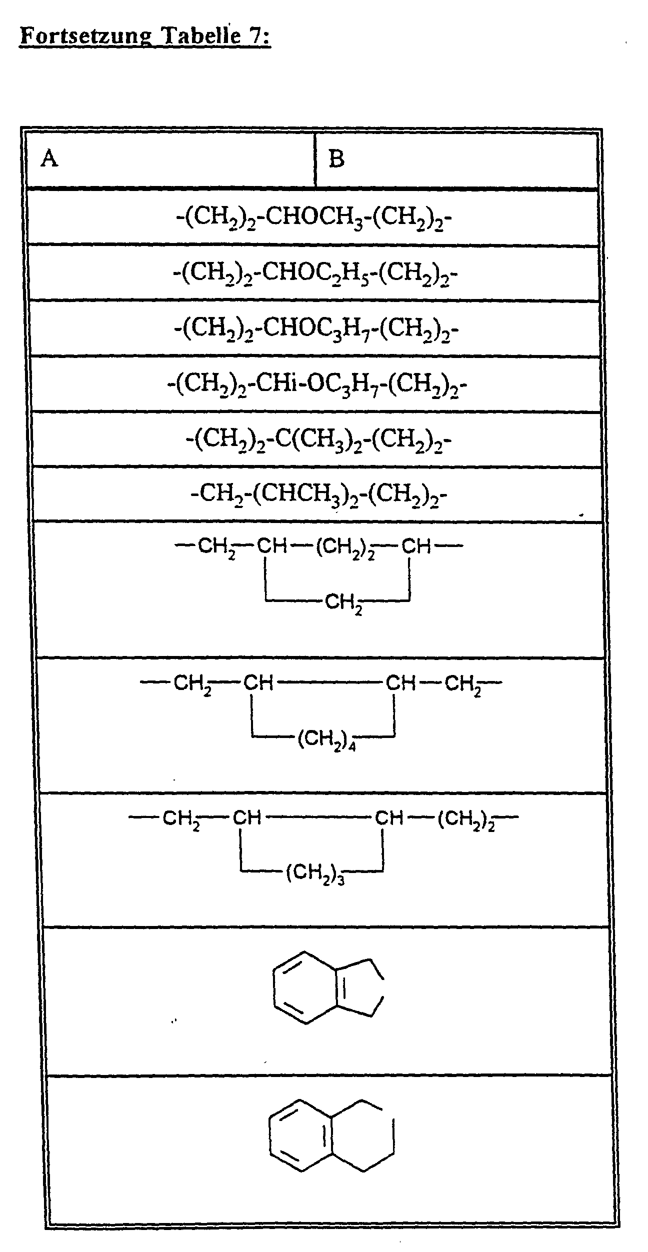

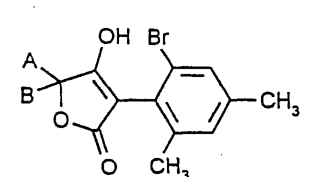

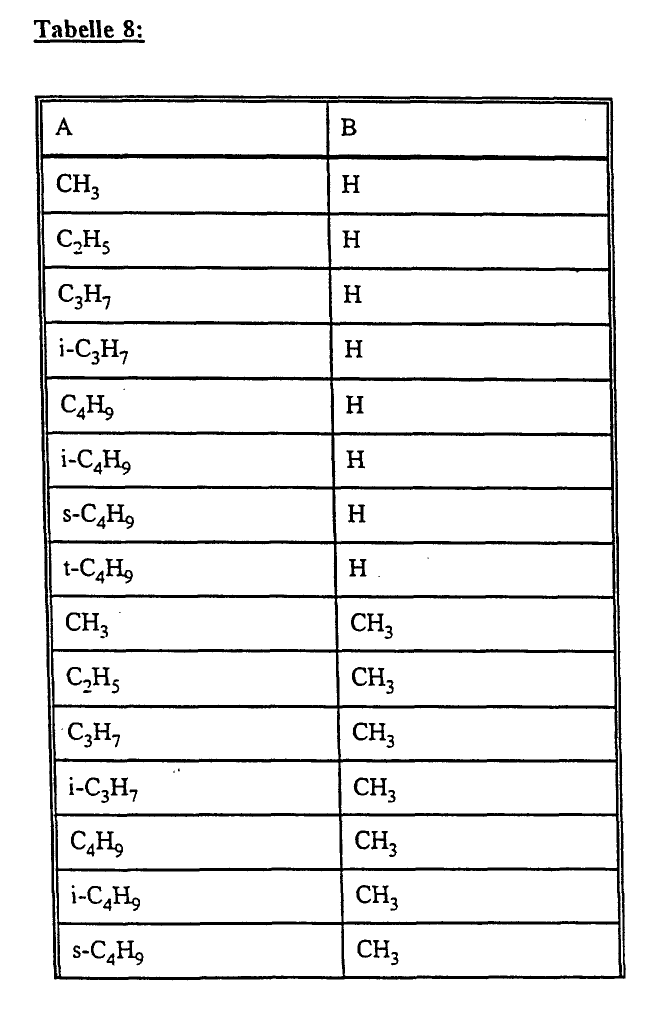

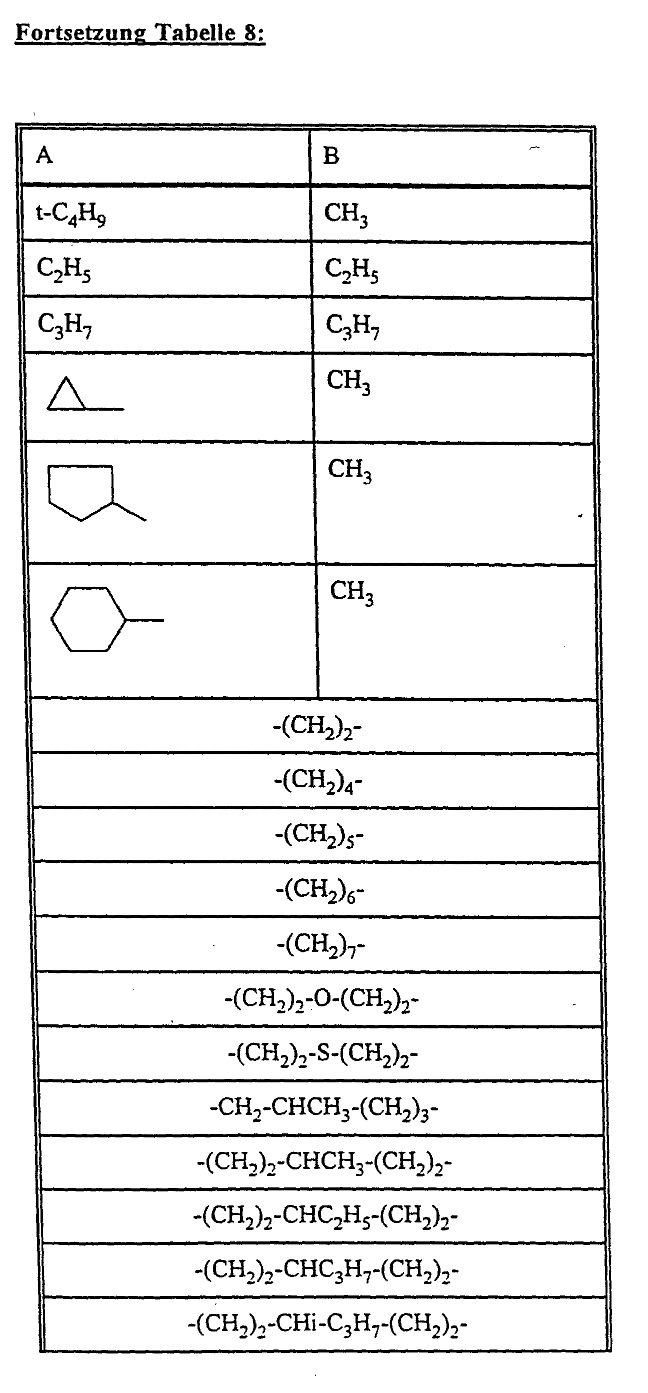

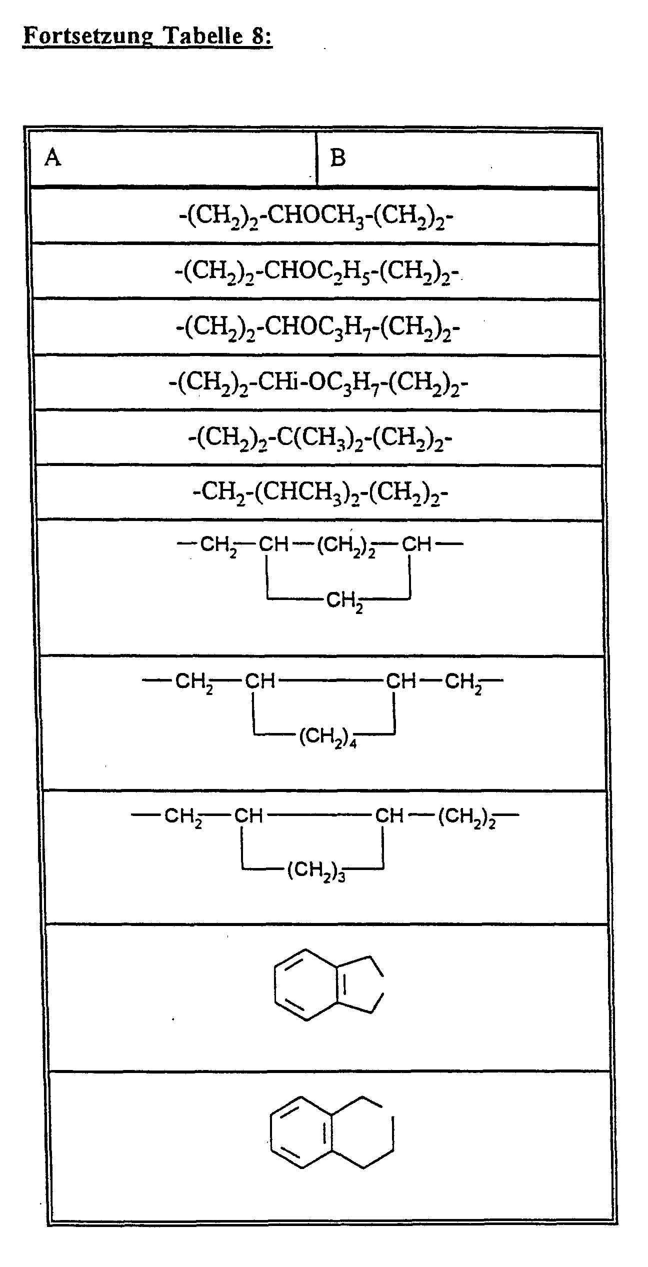

Im einzelnen seien außer den bei den Herstellungsbeispielen genannten Verbindungen die folgenden Verbindungen der Formel (I-3-a) genannt:

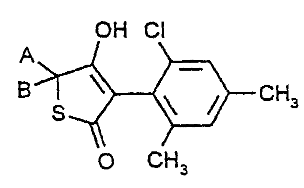

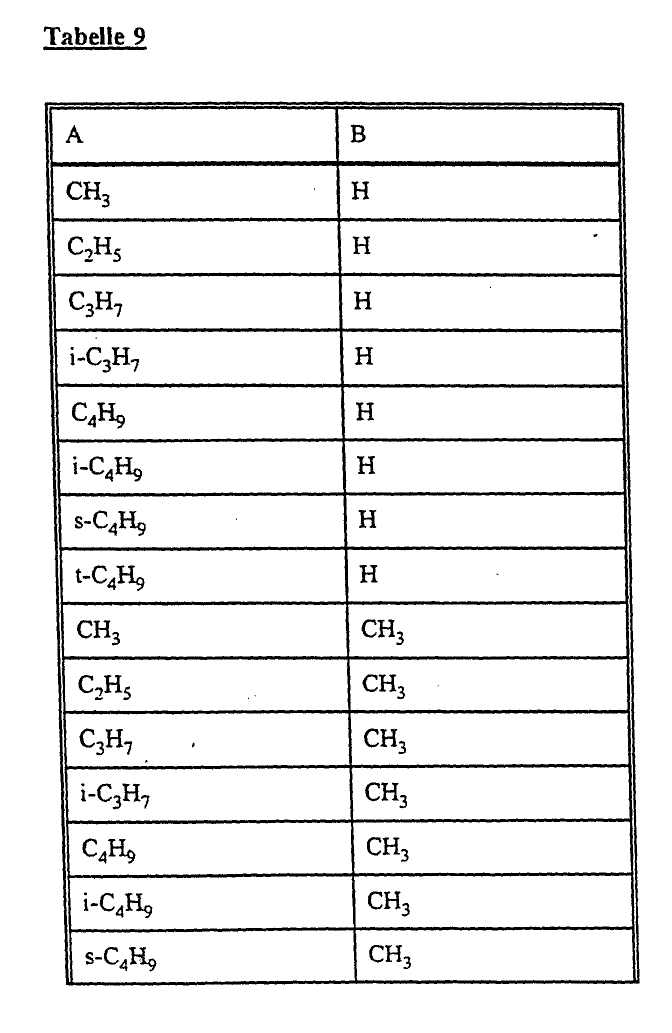

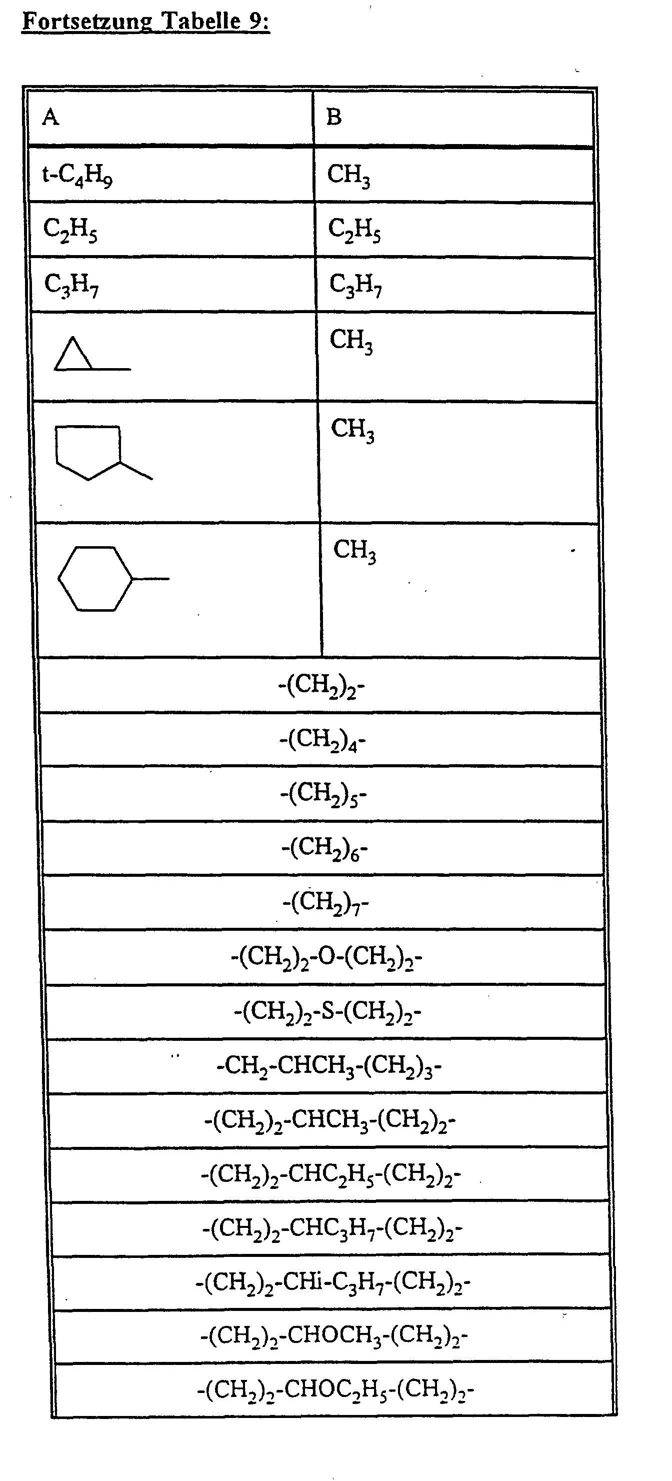

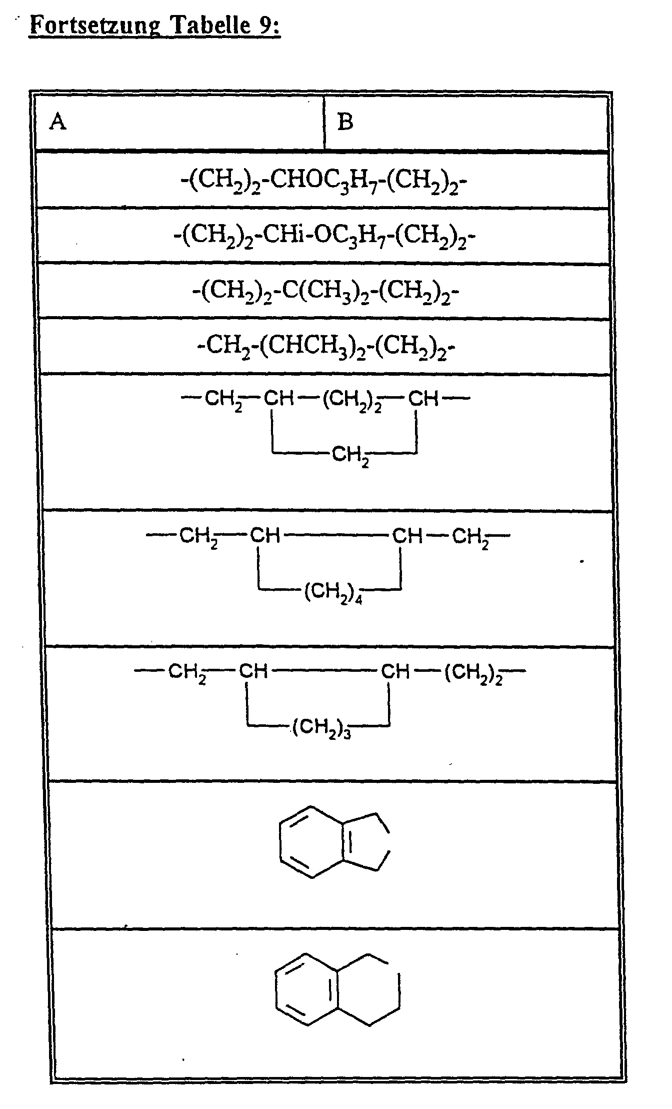

### Tabelle 10

A, B wie in Tabelle 9 angegeben
X = CH₃; Y = Cl; Z = CH₃

### Tabelle 11

A, B wie in Tabelle 9 angegeben
X = CH₃; Y = CH₃; Z = Br

### Tabelle 12

A, B wie in Tabelle 9 angegeben
X = CH₃; Y = Br; Z = CH₃

Im einzelnen seien außer den bei den Herstellungsbeispielen genannten Verbindungen die folgenden Verbindungen der Formel (I-5-a) genannt:

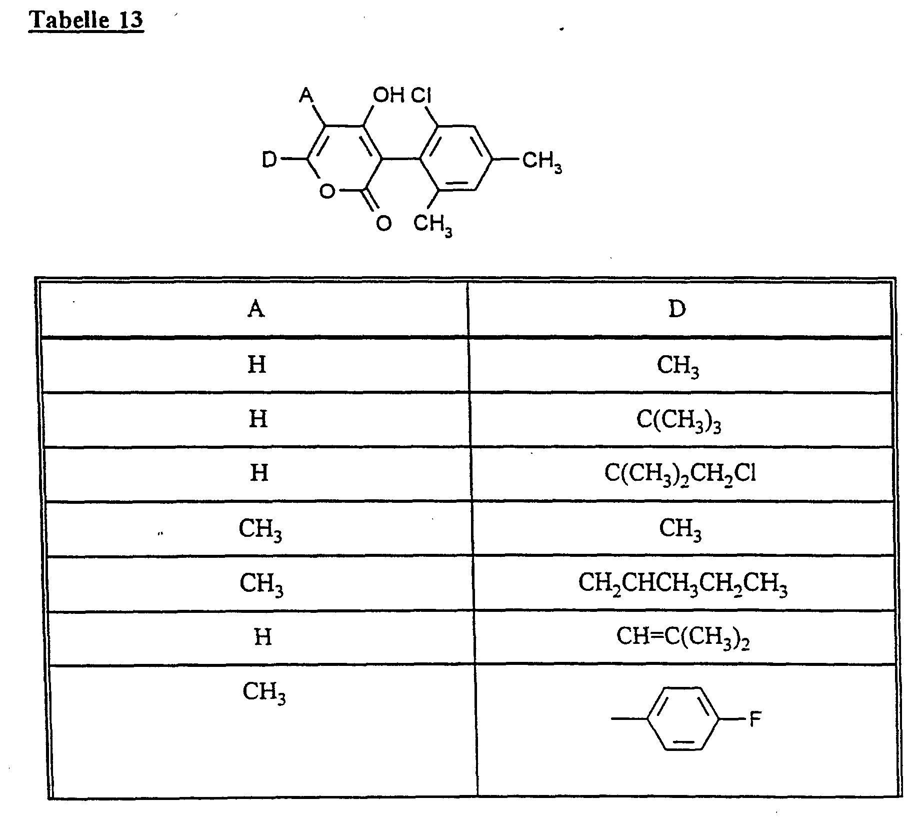

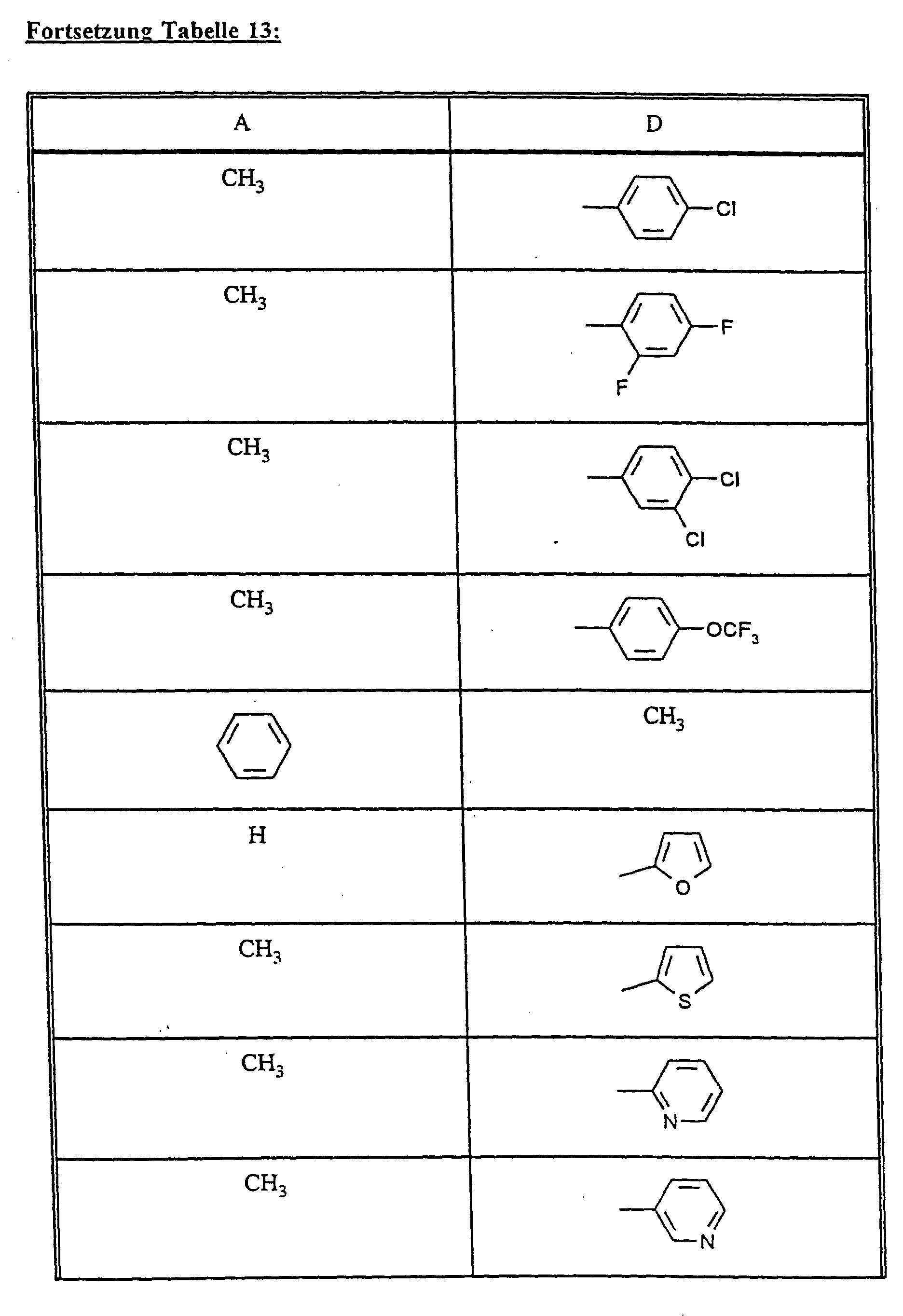

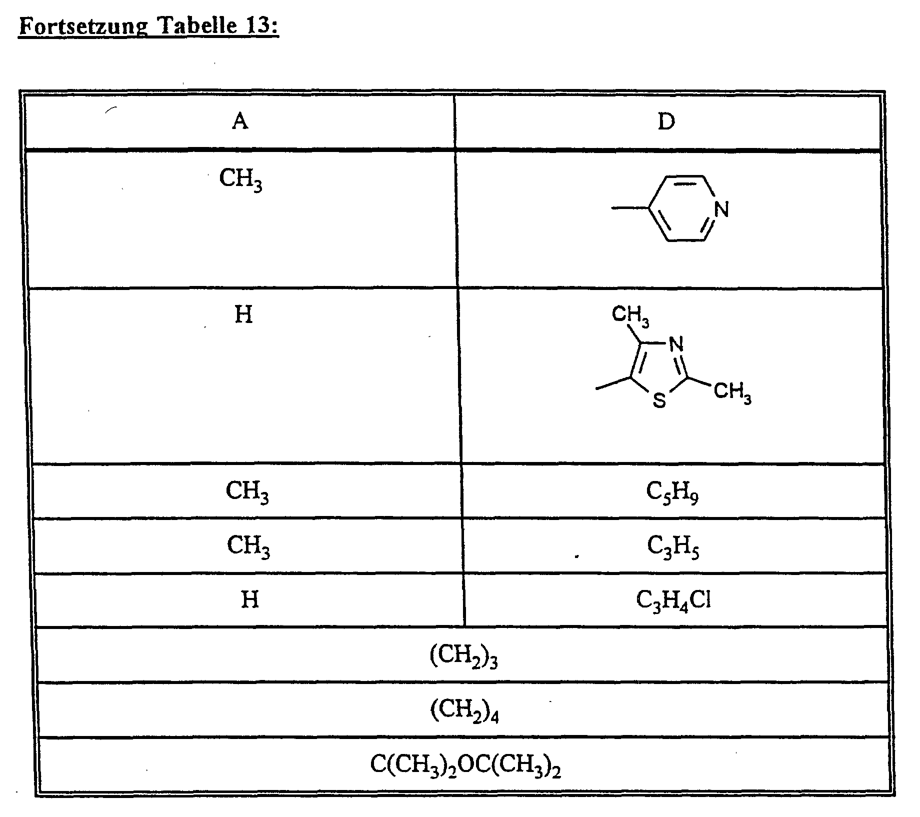

### Tabelle 14:

A und D wie in Tabelle 13 angegeben
X = CH₃; Y = Cl; Z = CH₃

### Tabelle 15:

A und D wie in Tabelle 13 angegeben
X = CH₃; Y = CH₃; Z = Br

### Tabelle 16:

A und D wie in Tabelle 13 angegeben
X = CH₃; Y = Br; Z = CH₃

Im einzelnen seien außer den bei den Herstellungsbeispielen genannten Verbindungen die folgenden Verbindungen der Formel (I-6-a) genannt:

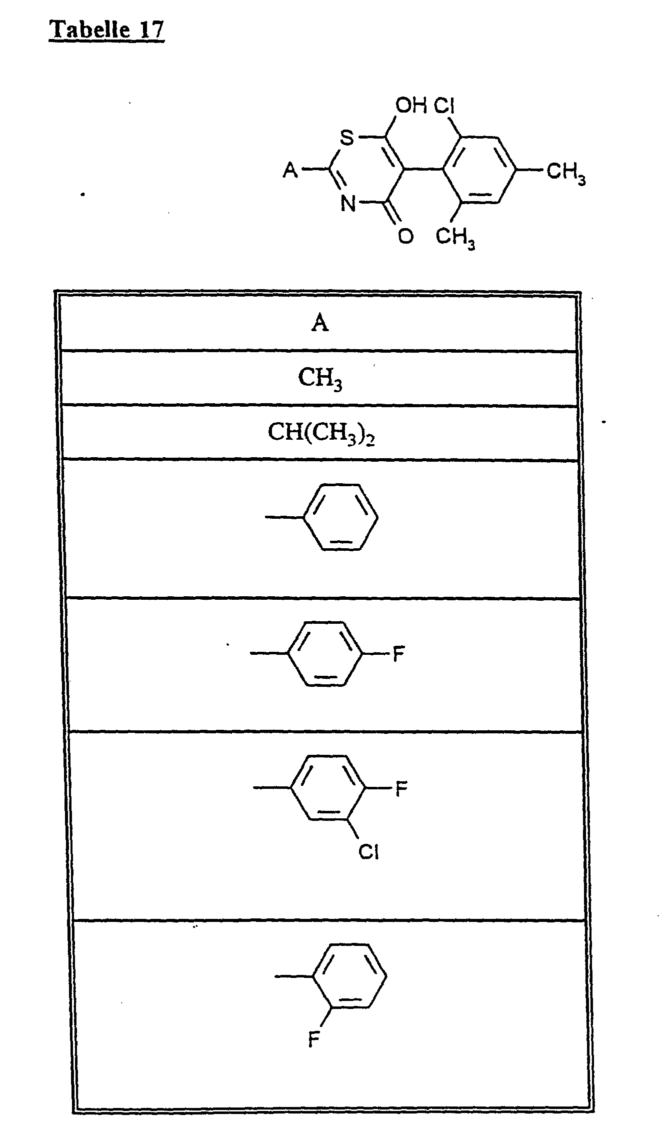

### Tabelle 18:

A wie in Tabelle 17 angegeben
X = CH₃; Y = Cl; Z = CH₃

### Tabelle 19:

A wie in Tabelle 17 angegeben
X = CH₃; Y = CH₃; Z = Br

### Tabelle 20:

A wie in Tabelle 17 angegeben
X = CH₃; Y = Br; Z = CH₃

Verwendet man gemäß Verfahren (A) N-[(2-Chlor-4,6-dimethyl)-phenylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

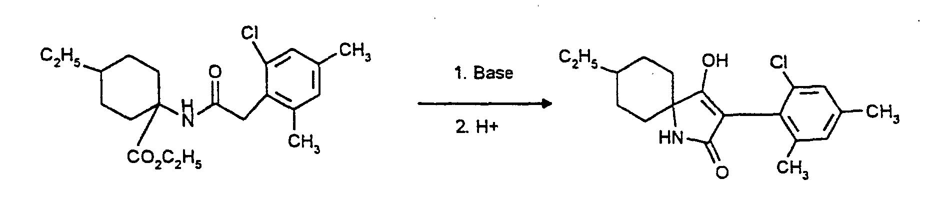

Verwendet man gemäß Verfahren (B) O-[(2-Chlor-4,6-dimethyl)-phenylacetyl]-hydroxyessigsäureethylester, so kann der Verlauf des erfindungsgemäßen Verfahrens durch folgendes Reaktionsschema wiedergegeben werden:

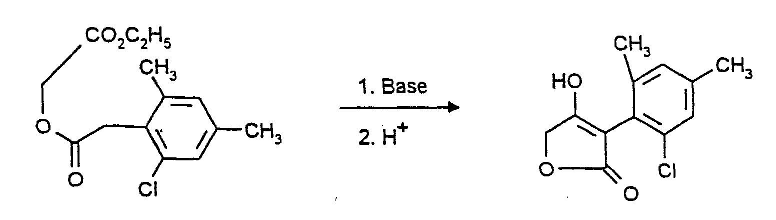

Verwendet man gemäß Verfahren (C) 2-[(2-Brom-4,6-dimethyl)-phenyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

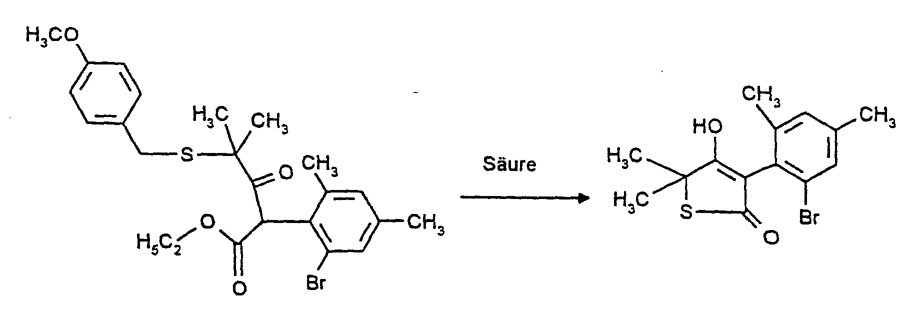

Verwendet man beispielsweise gemäß Verfahren (E) (Chlorcarbonyl)-2-[(2-brom-4,6-dimethyl)-phenyl]-keten und Aceton als Ausgangsverbindungen, so kann der Verlauf des erfindungsgemäßen Verfahrens durch das folgende Reaktionsschema wiedergegeben werden:

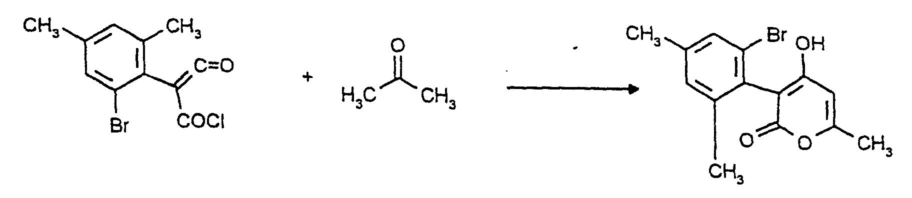

Verwendet man beispielsweise gemäß Verfahren (F) (Chlorcarbonyl)-2-[(4-brom-2,6-dimethyl)-phenyl]-keten und Thiobenzamid als Ausgangsverbindungen, so kann der Verlauf des erfindungsgemäßen Verfahrens durch das folgende Reaktionsschema wiedergegeben werden:

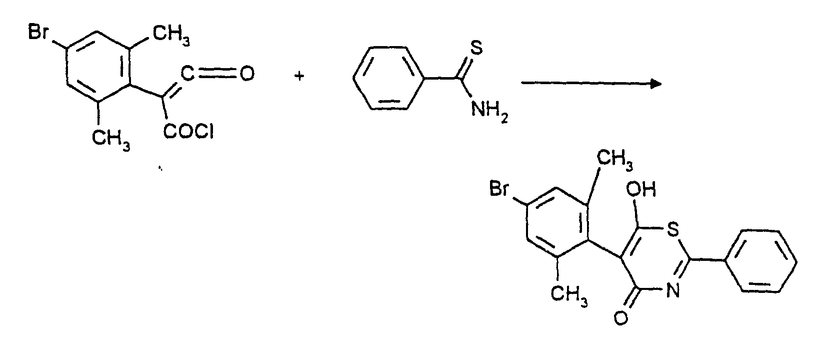

Verwendet man gemäß Verfahren (Gα) 3-[(2-Chlor-4,6-dimethyl)-phenyl]-5,5-dimethyl-pyrrolidin-2,4-dion und Pivaloylchlorid als Ausgangsstoffe, so kann der Verlauf des erfindungsgemäßen Verfahre s durch folgendes Reaktionsschema wiedergegeben werden:

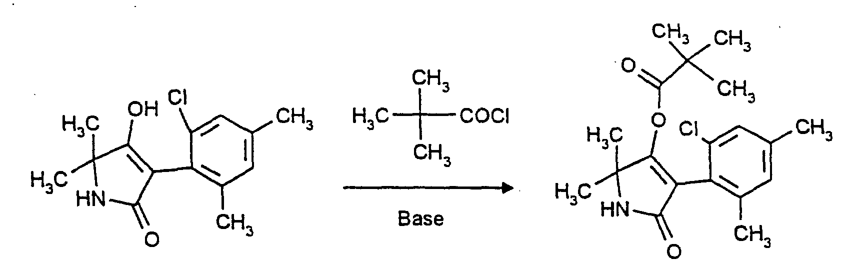

Verwendet man gemäß Verfahren (G) (Variante β) 3-[(4-Chlor-2,6-dimethyl)-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

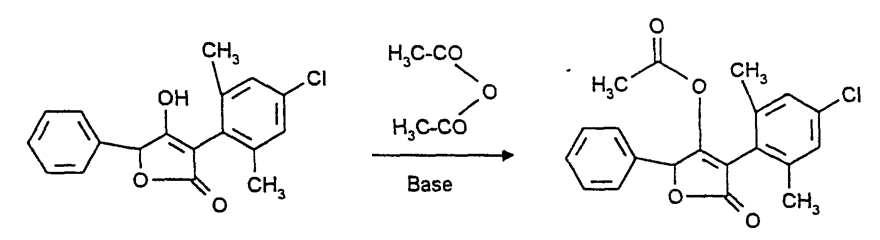

Verwendet man gemäß Verfahren (H) 8-[(4-Brom-2-ethyl-6-methyl)-phenyl]-1,6-diaza-bicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

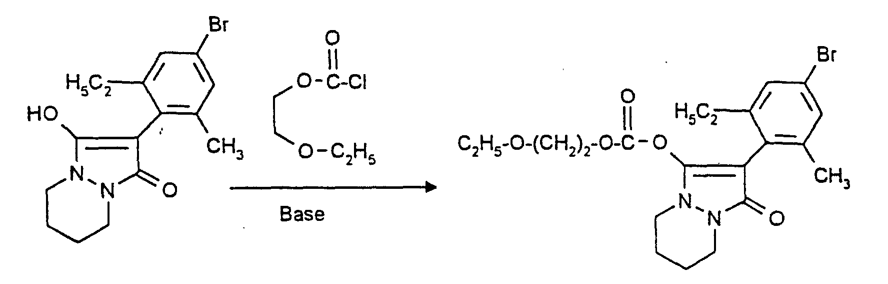

Verwendet man gemäß Verfahren (I), (Variante α) 3-[(2-Chlor-4,6-dimethyl)-phenyl]-4-hydroxy-6-(3-pyridyl)-pyron und Chlormonothioameisensäuremethylester als Ausgangsprodukte, so kann der Reaktionsverlauf folgendermaßen wiedergegeben werden:

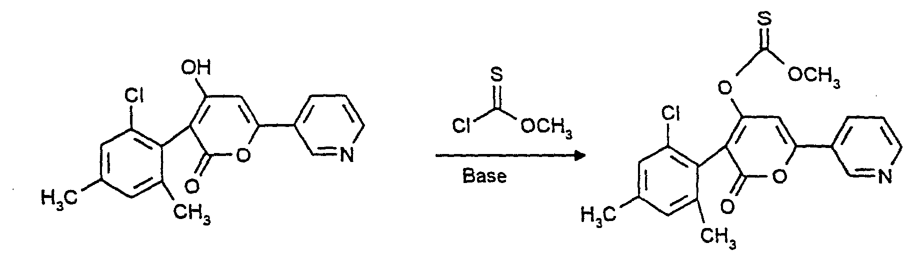

Verwendet man gemäß Verfahren (I), (Variante β) 5-[(2-Brom-4-methyl-6-methyl)-phenyl]-6-hydroxy-2-(4-chlorphenyl)-thiazin-4-on, Schwefelkohlenstoff und Methyliodid als Ausgangskomponenten, so kann der Reaktionsverlauf wie folgt wiedergegeben werden:

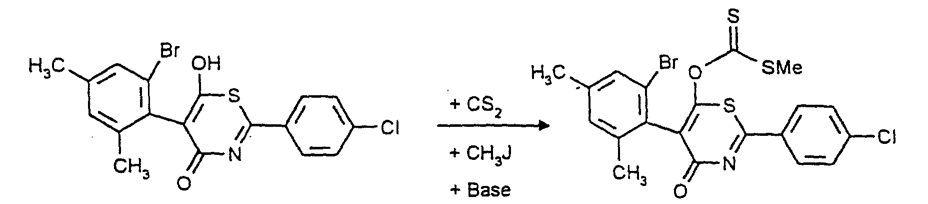

Verwendet man gemäß Verfahren (J) 2-[(2-Chlor-4,6-dimethyl)-phenyl]-1,5-trimethylen-pyrrolidin-2,4-dion und Methansulfonsäurechlorid als Ausgangsprodukt, so kann der Reaktionsverlauf durch folgendes Reaktionsschema wiedergegeben werden:

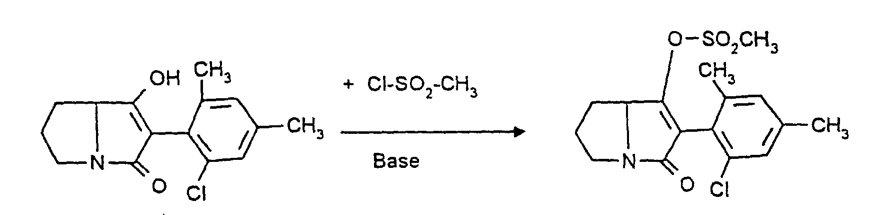

Verwendet man gemäß Verfahren (K) 2-[(2-Chlor-6-ethyl-4-methyl)-phenyl]-4-hydroxy-5-methyl-6-(2-pyridyl)-pyron und Methanthio-phosphonsäurechlorid-(2,2,2-trifluorethylester) als Ausgangsprodukte, so kann der Reaktionsverlauf durch folgendes Reaktionsschema wiedergegeben werden:

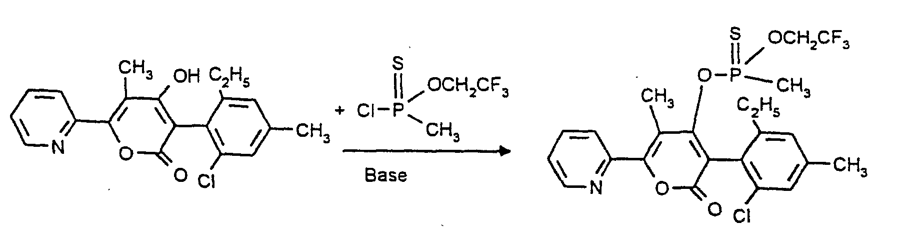

Verwendet man gemäß Verfahren (L) 3-[(4-Brom-2,6-diethyl)-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

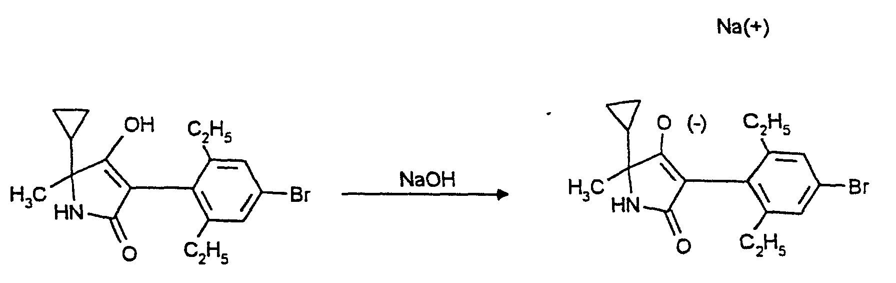

Verwendet man gemäß Verfahren (M) (Variante α) 3-[(2-Brom-4,6-dimethyl)-phenyl]-4-hydroxy-5,5-tetramethylen-Δ³-dihydro-furan-2-on und Ethylisocyanat als Ausgangsprodukte, so kann der Reaktionsverlauf durch folgendes Reaktionsschema wiedergegeben werden:

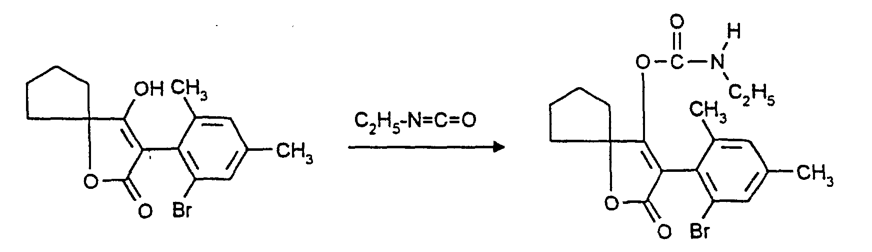

Verwendet man gemäß Verfahren (M) (Variante β) 3-[(2-Chlor-4,6-dimethyl)-phenyl]-5-methyl-pyrrolidin-2,4-dion und Dimethylcarbamidsäurechlorid als Ausgangsprodukte, so kann der Reaktionsverlauf durch folgendes Schema wiedergegeben werden:

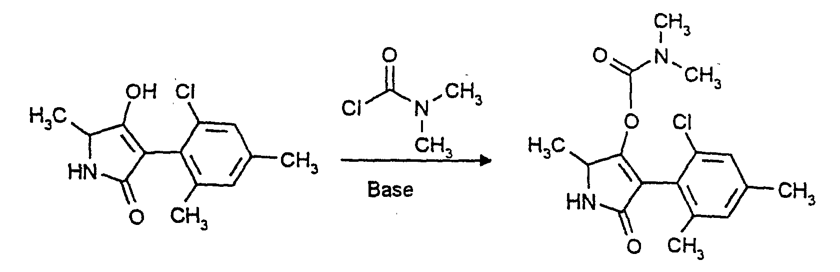

Die beim erfindungsgemäßen Verfahren (A) als Ausgangsstoffe benötigten Verbindungen der Formel (II) 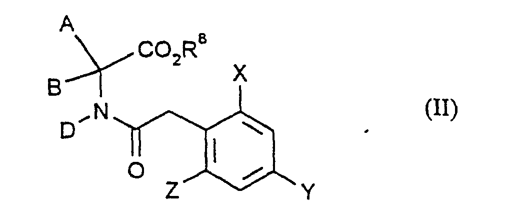 in welcher
- A, B, D, X, Y, Z und R⁸: die oben angegebene Bedeutungen haben,
sind neu.

Man erhält die Acylaminosäureester der Formel (II) beispielsweise, wenn man Aminosäurederivate der Formel (XXI) 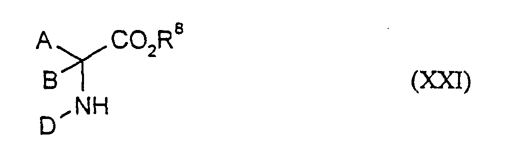 in welcher
- A, B, R⁸ und D: die oben angegebenen Bedeutungen haben,
mit substituierten Phenylessigsäurehalogeniden der Formel (XXII) 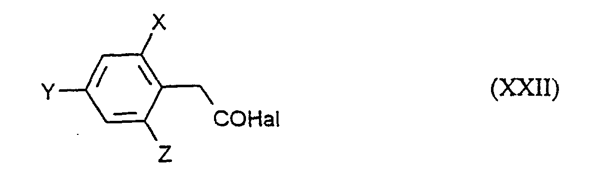 in welcher
- X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXIII) 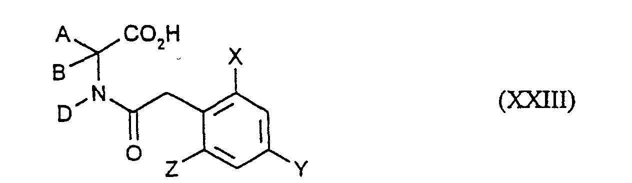 in welcher
- A, B, D, X, Y und Z: die oben angegebenen Bedeutungen haben,
verestert (Chem. Ind. (London) 1568 (1968)).

Die Verbindungen der Formel (XXIII) 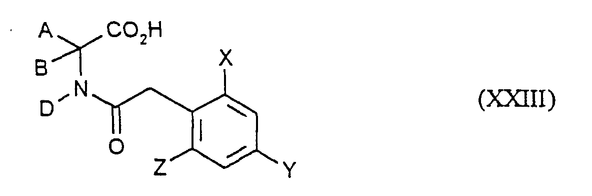 in welcher
- A, B, D, X, Y und Z: die oben angegebenen Bedeutungen haben,
sind neu.

Man erhält die Verbindungen der Formel (XXIII), wenn man Aminosäuren der Formel (XXIV) 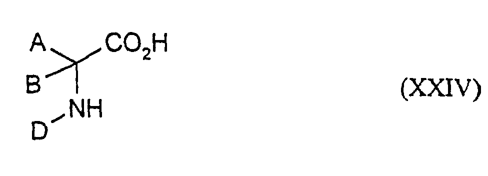 in welcher
- A, B und D: die oben angegebenen Bedeutungen haben,
mit substituierten Phenylessigsäurehalogeniden der Formel (XXII) 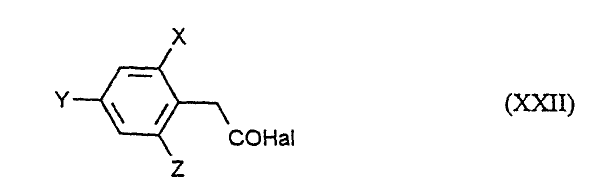 in welcher
- X, Y und Z: die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XXII) sind neu.

Man erhält die Verbindungen der Formel (XXII) beispielsweise, indem man substituierte Phenylessigsäuren der Formel (XXV) 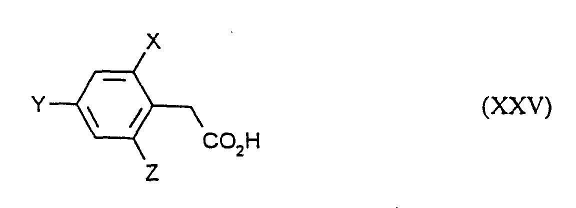 in welcher
- X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXV) sind neu.

Man erhält die Verbindungen der Formel (XXV) beispielsweise, indem man substituierte Phenylessigsäureester der Formel (XXVI) 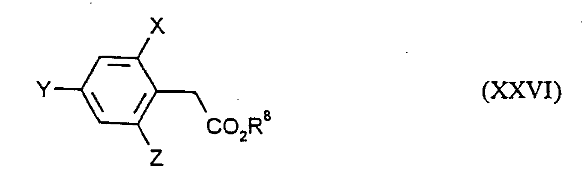 in welcher
- X, Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXVI) sind neu.

Man erhält die Verbindungen der Formel (XXVI) beispielsweise, indem man substituierte 1,1,1-Trichlor-2-phenylethane der Formel (XXVII) 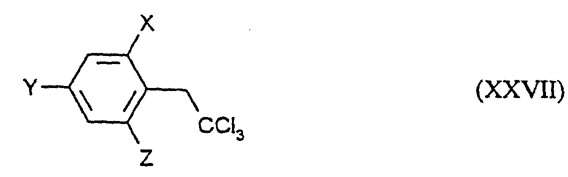 in welcher
- X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vorn Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE-33 14 249).

Die Verbindungen der Formel (XXVII) sind neu.

Man erhält die Verbindungen der Formel (XXVII) beispielsweise, wenn man Aniline der Formel (XXVIII) 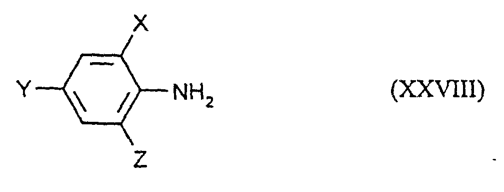 in welcher
- X, Y und Z: die oben angegebene Bedeutung haben,
in Gegenwart eines Alkylnitrits der Formel (XXIX)

R²¹-ONO (XXIX)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt (vgl. J. Org. Chem. 53 (1988), 3637).

Die Verbindungen der Formel (XXVIII) und (XXIX) sind bekannte Verbindungen der Organischen Chemie. Kupfer(II)-chlorid und Vinylidenchlorid sind lange bekannt und käuflich.

Die Verbindungen der Formel (XXI) und (XXIV) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIVa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 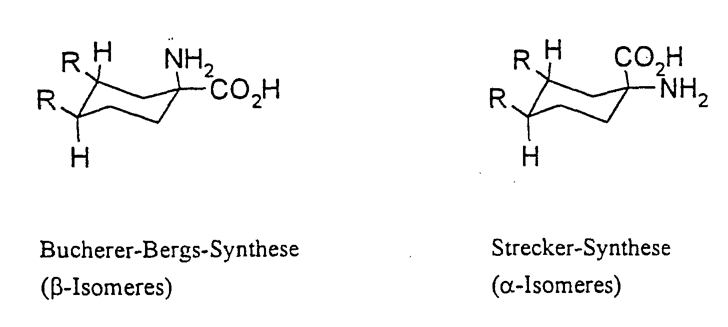 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 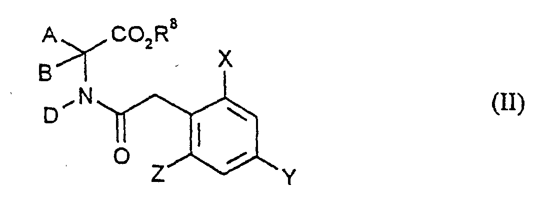 in welcher
- A, B, D, X, Y, Z und R⁸: die oben angegebenen Bedeutungen haben,
herstellen, wenn man Aminonitrile der Formel (XXX) 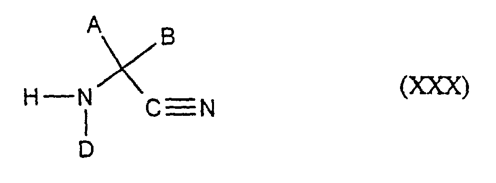 in welcher
- A, B und D: die oben angegebenen Bedeutungen haben,
mit substituierten Phenylessigsäurehalogeniden der Formel (XXII) 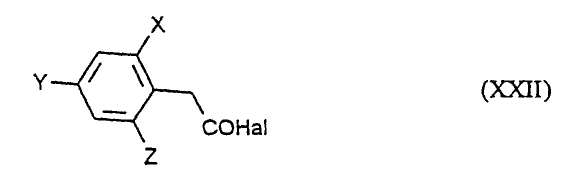 in welcher
- X, Y, Z und Hal: die oben angegebenen Bedeutungen haben,
zu Verbindungen der Formel (XXXI) 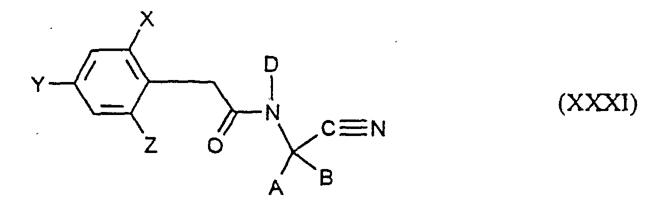 in welcher
- A, B, D, X, Y und Z: die oben angegebenen Bedeutungen haben,
umsetzt,
und diese anschließend einer sauren Alkoholyse unterwirft.

Die Verbindungen der Formel (XXXI) sind ebenfalls neu.

Die bei dem erfindungsgemäßen Verfahren (B) als Ausgangstoffe benötigten Verbindungen der Formel (III) 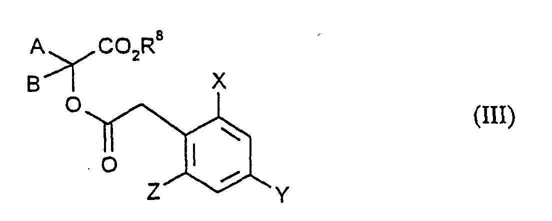 in welcher
- A, B, X, Y, Z und R⁸: die oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man 2-Hydroxycarbonsäureester der Formel (XXXII) 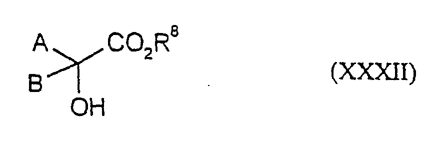 in welcher
- A, B und R⁸: die oben angegebenen Bedeutungen haben,
mit substituierten Phenylessigsäurehalogeniden der Formel (XXII) 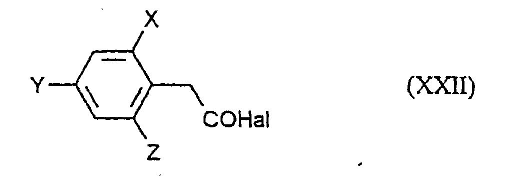 in welcher
- X, Y, Z und Hal: die oben angegebenen Bedeutungen haben,
acyliert (Chem. Reviews 52, 237-416 (1953)).

Weiterhin erhält man Verbindungen der Formel (III), wenn man substituierte Phenylessigsäuren der Formel (XXV) 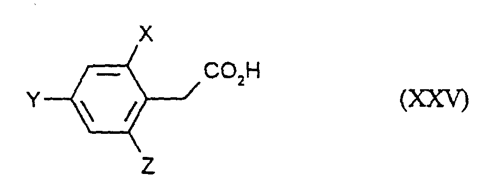 in welcher
- X, Y und Z: die oben angegebenen Bedeutungen haben,
mit α-Halogencarbonsäureestern der Formel (XXXIII) 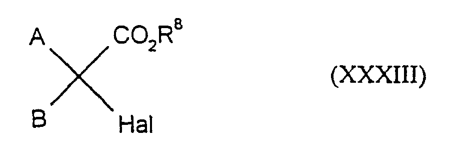 in welcher
- A, B und R⁸: die oben angegebenen Bedeutungen haben und
- Hal: für Chlor oder Brom steht,
alkyliert.

Die Verbindungen der Formel (XXXIII) sind käuflich.

Die bei dem obigen Verfahren (C) als Ausgangsstoffe benötigten Verbindungen der Formel (IV) 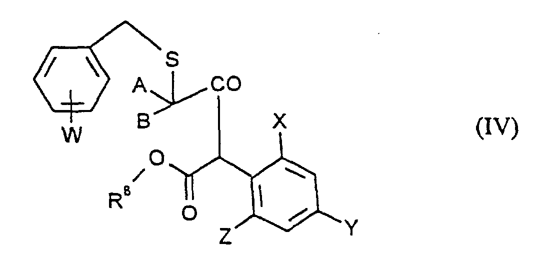 in welcher
- A, B, W, X, Y, Z und R⁸: die oben angegebenen Bedeutungen haben,
sind neu.

Sie lassen sich nach im Prinzip bekannten Methoden herstellen

Man erhält die Verbindungen der Formel (IV) beispielsweise, wenn man substituierte Phenylessigsäureester der Formel (XXVI) 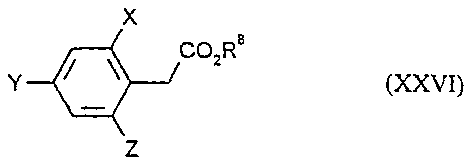 in welcher
- X, Y, R⁸ und Z: die oben angegebenen Bedeutungen haben,
mit 2-Benzylthio-carbonsäurehalogeniden der Formel (XXXIV) 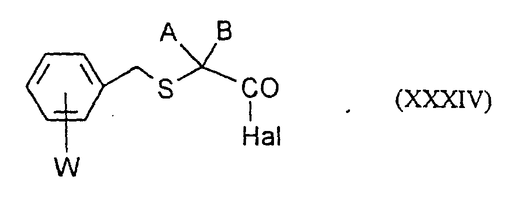 in welcher
- A, B und W: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXIV) sind teilweise bekannt und/oder lassen sich nach bekannten Verfahren herstellen (J. Antibiotics (1983). 26, 1589).

Die beim Verfahren (E) als Ausgangsstoffe benötigten Halogencarbonylketene der Formel (V) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). Man erhält die Verbindungen der Formel (V) 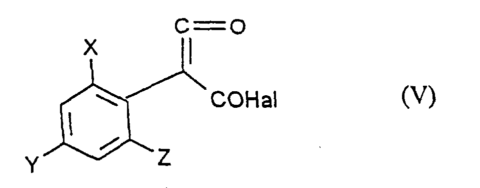 in welcher
- X, Y und Z: die oben angegebenen Bedeutungen haben und
- Hal: für Chlor oder Brom steht,
wenn man
substituierte Phenylmalonsäuren der Formel (XXXV) 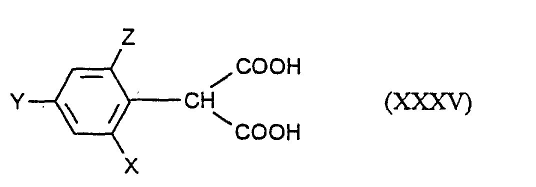 in welcher
- X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, bei einer Temperatur zwischen -20°C und 200°C, bevorzugt zwischen 0°C und 150°C, umsetzt.

Die substituierten Phenylmalonsäuren der Formel (XXXV) sind neu. Sie lassen sich aber in einfacher Weise nach bekannten Verfahren herstellen (vgl. z.B. Organikum, VEB Deutscher Verlag der Wissenschaften, Berlin 1977, S. 517 ff).

Die für das erfindungsgemäße Verfahren (E) als Ausgangsstoffe benötigten Carbonylverbindungen der Formel (VIII) oder deren Silylenolether der Formel (VIIIa) 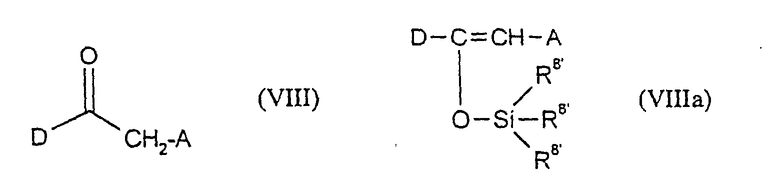 in welchen
- A, D und R^{8'}: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (F) als Ausgangsstoffe benötigten Ketensäurechloride der Formel (V) wurden bereits beim erfindungsgemäßen Verfahren (E) beschrieben. Die zur Durchführung des erfindungsgemäßen Verfahrens (F) benötigten Thioamide der Formel (IX) 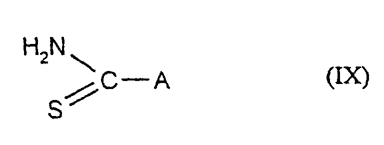 in welcher
- A: die oben angegebene Bedeutung hat,
sind allgemein in der Organischen Chemie bekannte Verbindungen.

Die beim Verfahren (G) als Ausgangsstoffe benötigten Verbindungen der Formel (I-4-a) sind bekannt und/oder lassen sich in einfacher Weise nach bekannten Methoden herstellen (vgl. and WO 92/16510).

Man erhält die Verbindungen der Formel (I-4-a) beispielsweise, wenn man
Verbindungen der Formel (V) 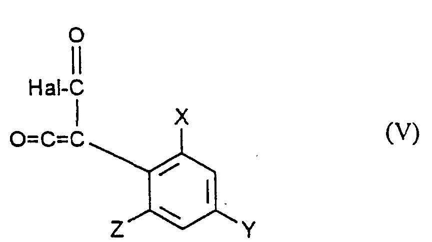 in welcher
- X, Y und Z: die oben angegebenen Bedeutungen haben
und
- Hal: für Halogen (insbesondere Chlor oder Brom) steht,
oder
Verbindungen der Formel (VI) 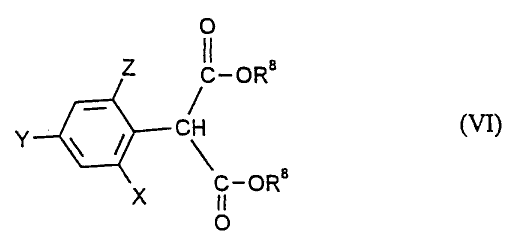 in welcher
- R⁸, X, Y und Z: die oben angegebenen Bedeutungen haben,
mit Hydrazinen der Formel (VII)

A-NH-NH-D (VII)

in welcher
- A und D: die oben angegebenen Bedeutungen haben,
gegebenenfalls in Gegenwart eines Verdünnungsmittels,
wobei verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie, nur im Fall, daß Verbindungen der Formel (VI) eingesetzt werden, Alkohole wie Methanol, Ethanol, Propanol, Iso-Propanol, Butanol, Iso-Butanol und tert.-Butanol, und gegebenenfalls in Gegenwart einer Base, wobei in dem Fall, daß Verbindungen der Formel (V) eingesetzt werden, anorganische Basen, insbesondere Alkali- oder Erdalkalicarbonate wie Natriumcarbonat, Kaliumcarbonat oder Calciumcarbonat sowie organische Basen wie beispielsweise Pyridin oder Triethylamin in Betracht kommen und in dem Fall, daß Verbindungen der Formel (VI) eingesetzt werden, Alkalimetall- und Erdalkalimetalloxide, -hydroxide und -carbonate, wie Natriumhydroxid, Kaliumhydroxid, Magnesiumoxid, Caiciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können, Alkalimetalle wie Natrium oder Kalium, Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat in Betracht kommen, bei Temperaturen zwischen -20°C und 250°C, vorzugsweise zwischen 0°C und 150°C umsetzt.

Die Malonsäureester der Formel (VI) 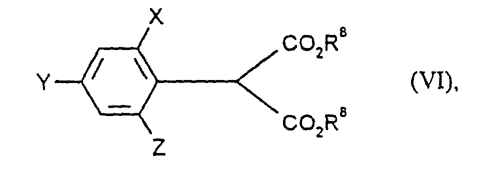 in welcher
- R⁸, X, Y und Z: die oben angegebenen Bedeutungen haben, sind neu.

Sie lassen sich nach allgemein bekannten Methoden der Organischen Chemie darstellen (vgl. z.B. Tetrahedron Lett. 27, 2763 (1986) und Organikum VEB Deutscher Verlag der Wissenschaften, Berlin 1977, S. 587 ff.).

Die Hydrazine der Formel (VII)

A-NH-NH-D (VII),

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), Ep 508 126).

Die zur Durchführung der erfindungsgemäßen Verfahren (G), (H), (I), (J), (K), (L) und (M) außerdem als Ausgangsstoffe benötigten Säurehalogenide der Formel (X), Carbonsäureanhydride der Formel (XI), Chlorameisensäureester oder Chlorameisensäurethioester der Formel (XII), Chlormonothioameisensäureester oder Chlordithioameisensäureester der Formel (XIII), Alkylhalogenide der Formel (XIV), Sulfonsäurechloride der Formel (XV), Phosphorverbindungen der Formel (XVI) und Metallhydroxide, Metallalkoxide oder Amine der Formel (XVII) und (XVIII) und Isocyanate der Formel (XIX) und Carbamidsäurechloride der Formel (XX) sind allgemein bekannte Verbindungen der organischen bzw. anorganischen Chemie.

Die Verbindungen der Formeln (VII), (VIII), (IX) bis (XXI), (XXIV) und (XXXII) bis (XXXIV) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, D, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß man Verbindungen der Formel (III), in welcher A, B, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle gegenüber den Reaktionsteilnehmern inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden, wie z.B. Halogenwasserstoffsäuren, Schwefelsäure, Alkyl-, Aryl- und Haloalkylsulfonsäuren, insbesondere werden halogenierte Alkylcarbonsäuren wie z.B. Trifluoressigsäure verwendet.

Die Reaktionstemperatur kann bei der Durchführung des erfindungsgemäßen Verfahrens (C) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure in katalytischen Mengen einzusetzen.

Das erfindungsgemäße Verfahren (E) ist dadurch gekennzeichnet, daß man Carbonylverbindungen der Formel (VIII) oder deren Silylenolether der Formel (VIIIa) mit Ketensäurehalogeniden der Formel (V) gegebenenfalls in Gegenwart eines Verdünnungsmittels und gegebenenfalls in Gegenwart eines Säureakzeptors umsetzt.

Als Verdünnungsmittel können bei dem erfindungsgemäßen Verfahren (E) alle gegenüber den Reaktionsteilnehmern inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base oder N,N-Dimethyl-anilin.

Die Reaktionstemperatur kann bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (E) wird vorzugsweise unter Normaldruck durchgeführt.

Bei der Durchführung des erfindungsgemäßen Verfahrens (E) setzt man die Reaktionskomponenten der Formeln (VIII) und (V) und gegebenenfalls den Säureakzeptor im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (F) ist dadurch gekennzeichnet, daß man Thioamide der Formel (IX) mit Ketensäurehalogeniden der Formel (V) gegebenenfalls in Gegenwart eines Verdünnungsmittels und gegebenenfalls in Gegenwart eines Säureakzeptors umsetzt.

Als Verdünnungsmittel können bei der erfindungsgemäßen Verfahrensvariante (F) alle inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (F) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperatur kann bei der Durchführung des erfindungsgemäßen Verfahrens (F) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (F) wird zweckmäßigerweise unter Normaldruck durchgeführt.

Bei der Durchführung des erfindungsgemäßen Verfahrens (F) setzt man die Reaktionskomponenten der Formeln (IX) und (V) und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (Gα) ist dadurch gekennzeichnet, daß man Verbindungen der Formeln (I-1-a) bis (I-6-a) jeweils mit Carbonsäurehalogeniden der Formel (X) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Gα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, Nitrile wie Acetonitril und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Gα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Gα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Gα) werden die Ausgangsstoffe der Formeln (I-1-a) bis (I-6-a) und das Carbonsäurehalogenid der Formel (X) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Gβ) ist dadurch gekennzeichnet, daß man Verbindungen der Formeln (I-1-a) bis (I-6-a) jeweils mit Carbonsäureanhydriden der Formel (XI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Gβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Gβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Gβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Gβ) werden die Ausgangsstoffe der Formeln (I-1-a) bis (I-6-a) und das Carbonsäureanhydrid der Formel (XI) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (H) ist dadurch gekennzeichnet, daß man Verbindungen der Formeln (I-1-a) bis (I-6-a) jeweils mit Chlorameisensäureestem oder Chlorameisensäurethiolestern der Formel (XII) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (H) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (H) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, Nitrile wie Acetonitril, darüber hinaus Carbonsäureester, wie Ethylacetat, und auch stark polare Solventien, wie Dimethylformamid, Dimethylsulfoxid und Sulfolan.

Die Reaktionstemperatur kann bei der Durchführung des erfindungsgemäßen Verfahrens (H) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (H) wird im allgemeinen unter Normaldruck durchgeführt.

Bei der Durchführung des erfindungsgemäßen Verfahrens (H) werden die Ausgangsstoffe der Formeln (I-1-a) bis (I-6-a) und der entsprechende Chlorameisensäureester bzw. Chlorameisensäurethiolester der Formel (XII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (I) ist dadurch gekennzeichnet, daß man Verbindungen der Formeln (I-1-a) bis (I-6-a) jeweils mit (Iα) Verbindungen der Formel (XIII) in Gegenwart eines Verdünnungsmittels und gegebenenfalls in Gegenwart eines Säurebindemittels oder (Iβ) Schwefelkohlenstoff und anschließend mit Alkylhalogeniden der Formel (XIV) gegebenenfalls in Gegennwart eines Verdünnungsmittels und gegebenenfalls in Gegenwart einer Base umsetzt.

Beim Herstellungsverfahren (Iα) setzt man pro Mol Ausgangsverbindung der Formeln (I-1-a) bis (I-6-a) ca. 1 Mol Chlormonothioameisensäureester bzw.

Chlordithioameisensäureester der Formel (XIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Carbonsäureester, Nitrile, Sulfone, Sulfoxide, aber auch Halogenalkane.

Vorzugsweise werden Dimethylsulfoxid, Ethylacetat, Acetonitril, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Iβ) setzt man pro Mol Ausgangsverbindungen der Formeln (I-1-a) bis (I-6-a) jeweils die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus den Verbindungen der Formeln (I-1-a) bis (I-6-a) durch Zusatz einer Base (wie z.B. Kaliumtertiärbutylat oder Natriumhydrid) das entsprechende Salz herzustellen. Man setzt die Verbindungen (I-1-a) bis (I-6-a) jeweils so lange mit Schwefelkohlenstoff um, bis die Bildung der Zwischenverbindung abgeschlossen ist, z.B. nach mehrstündigem Rühren bei Raumtemperatur.

Als Basen können beim Verfahren (Iβ) le üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise sind verwendbar aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Die weitere Umsetzung mit dem Alkylhalogenid der Formel (XIV) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Das erfindungsgemäße Verfahren (J) ist dadurch gekennzeichnet, daß man Verbindungen der Formeln (I-1-a) bis (I-6-a) jeweils mit Sulfonsäurechloriden der Formel (XV) gegebenenfalls in Gegenwart eines Verdünnungsmittels und gegebenenfalls in Gegenwart eines Säurebindemittels umsetzt.

Beim Herstellungsverfahren (J) setzt man pro Mol Ausgangsverbindung der Formel (I-1-a bis I-6-a) ca. 1 Mol Sulfonsäurechlorid der Formel (XV) bei -20 bis 150°C, vorzugsweise bei 20 bis 70°C um.

Das Verfahren (J) wird vorzugsweise in Gegenwart eines Verdünnungsmittels durchgeführt.

Als Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (K) ist dadurch gekennzeichnet, daß man Verbindungen der Formeln (I-1-a) bis (I-6-a) jeweils mit Phosphorverbindungen der Formel (XVI) gegebenenfalls in Gegenwart eines Verdünnungsmittels und gegebenenfalls in Gegenwart eines Säurebindemittels umsetzt.

Beim Herstellungsverfahren (K) setzt man zum Erhalt von Verbindungen der Formeln (I-1-e) bis (I-6-e) auf 1 Mol der Verbindungen (I-1-a) bis (I-6-a), 1 bis 2, vorzugsweise 1 bis 1,3 Mol der Phosphorverbindung der Formel (XVI) bei Temperaturen zwischen -40°C und 150°C, vorzugsweise zwischen -10 und 110°C um.

Das Verfahren (K) wird vorzugsweise in Gegenwart eines Verdünnungsmittels durchgeführt.

Als Verdünnungsmittel kommen alle inerten, polaren organischen Lösungsmittel in Frage wie Halogenkohlenwasserstoffe, Carbonsäureester, Ether, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (L) ist dadurch gekennzeichnet, daß man Verbindungen der Formeln (I-1-a) bis (I-6-a) jeweils mit Metallhydroxiden bzw. Metallalkoxiden der Formel (XVII) oder Aminen der Formel (XVIII), gegebenenfalls in Gegenwart eines Verdünnungsmittels, umsetzt.

Als Verdünnungsmittel können bei dem erfindungsgemäßen Verfahren (L) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (L) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (M) ist dadurch gekennzeichnet, daß man Verbindungen der Formeln (I-1-a) bis (I-6-a) jeweils mit (Mα) Verbindungen der Formel (XIX) gegebenenfalls in Gegenwart eines Verdünnungsmittels und gegebenenfalls in Gegenwart eines Katalysators oder (Mβ) mit Verbindungen der Formel (XX) gegebenenfalls in Gegenwart eines Verdünnungsmittels und gegebenenfalls in Gegenwart eines Säurebindemittels umsetzt.

Bei Herstellungsverfahren (Mα) setzt man pro Mol Ausgangsverbindung der Formeln (I-1-a) bis (I-6-a) ca. 1 Mol Isocyanat der Formel (XIX) bei 0 bis 100°C, vorzugsweise bei 20 bis 50°C um.

Das Verfahren (Mα) wird vorzugsweise in Gegenwart eines Verdünnungsmittels durchgeführt.

Als Verdünnungsmittel kommen alle inerten organischen Lösungsmittel in Frage, wie Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Mβ) setzt man pro Mol Ausgangsverbindung der Formeln (I-1-a) bis (I-6-a) ca. 1 Mol Carbamidsäurechlorid der Formel (XX) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvemichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:

Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindemia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp, Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze

wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen od mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octyli sothi azolin-3-on,
sein.

Die Herstellung und die Verwendung der erfindungsgemäßen Wirkstoffe gehen aus den nachfolgenden Beispielen hervor.

### Herstellungsbeispiele

### Beispiel (I-1-a-1)

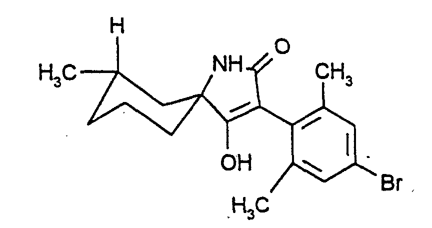

Zu 20,42 g (0,181 Mol) Kalium-tert.-butylat in 70 ml absolutem Tetrahydrofuran (THF) tropft man bei Rückflußtemperatur 32,6 g der Verbindung gemäß Beispiel (II-1), gelöst in 200 ml absolutem Toluol, und rührt noch 1,5 Stunden bei dieser Temperatur.

Zur Aufarbeitung verdünnt man mit Wasser, trennt die Phasen, extrahiert die Toluolphase mit Wasser und säuert die vereinten wäßrigen Phasen mit konz. HCI an. Das Produkt wird abgesaugt, gewaschen und getrocknet und schließlich in Methyl-tert.-butyl-(MTB)-ether/n-Hexan verrührt, abgesaugt und getrocknet.

Ausbeute: 20,6 g (68 % der Theorie); Fp.: >220°C.

Analog zu Beispiel (I-1-a-1) bzw. gemäß den allgemeinen Angaben zur Herstellung wurden die in der nachfolgenden Tabelle 21 aufgeführten Verbindungen der Formel (I-1-a) erhalten.

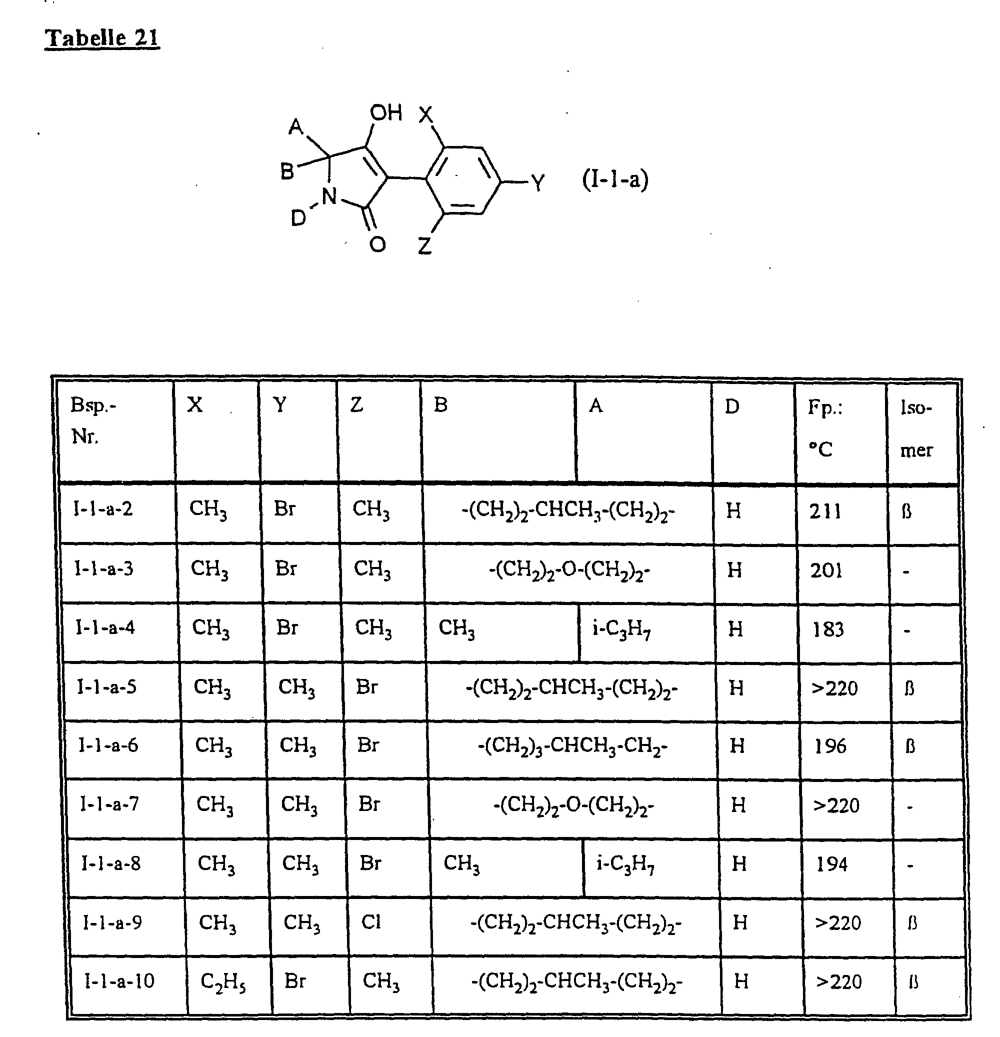

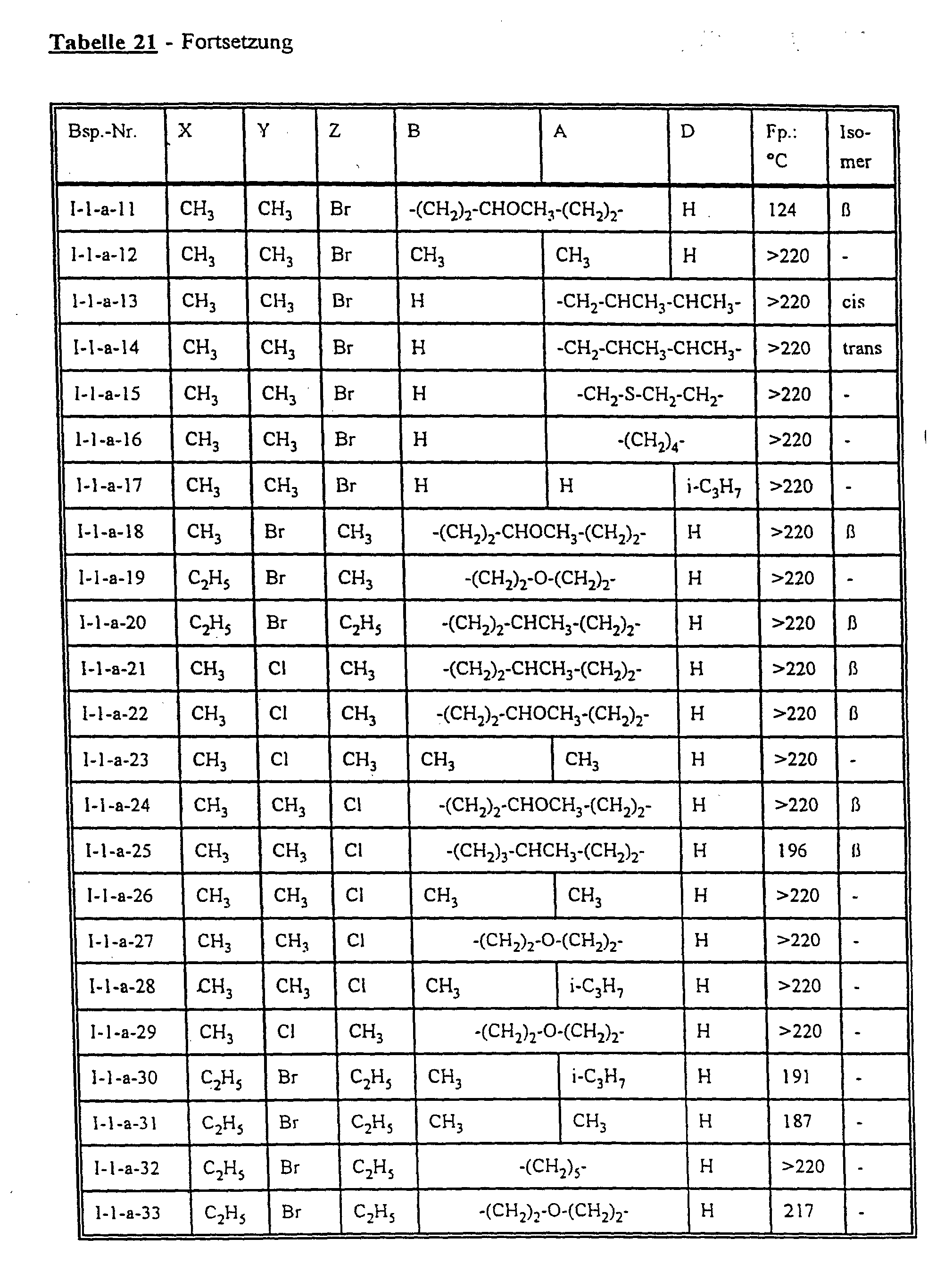

### Beispiel (I-1-b-1)

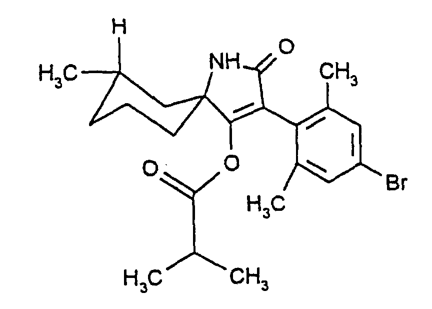

Zu 4,37 g der Verbindung gemäß Beispiel (I-1-a-1) in 70 ml absolutem Methylenchlorid und 2,52 ml (18 mMol) Triethylamin tropft man bei 0°C bis 10°C 1,9 ml (18 mMol) Isobuttersäurechlorid, gelöst in 5 ml absolutem Methylenchlorid. Man rührt bei Raumtemperatur, bis nach dünnschichtchromatographischer (DC) Kontrolle die Umsetzung beendet ist.

Zur Aufarbeitung wäscht man 2 mal mit 0.5 N NaOH, trocknet und dampft ein. Das Rohprodukt wird aus MTB-Ether/n-Hexan umkristallisiert.

Ausbeute: 1,70 g (32 % der Theorie); Fp.: 208°C.

Analog zu Beispiel (I-1-b-1) bzw. gemäß den allgemeinen Angaben zur Herstellung wurden die in der nachfolgenden Tabelle 22 aufgeführten Beispiele der Formel (I-1-b) erhalten.

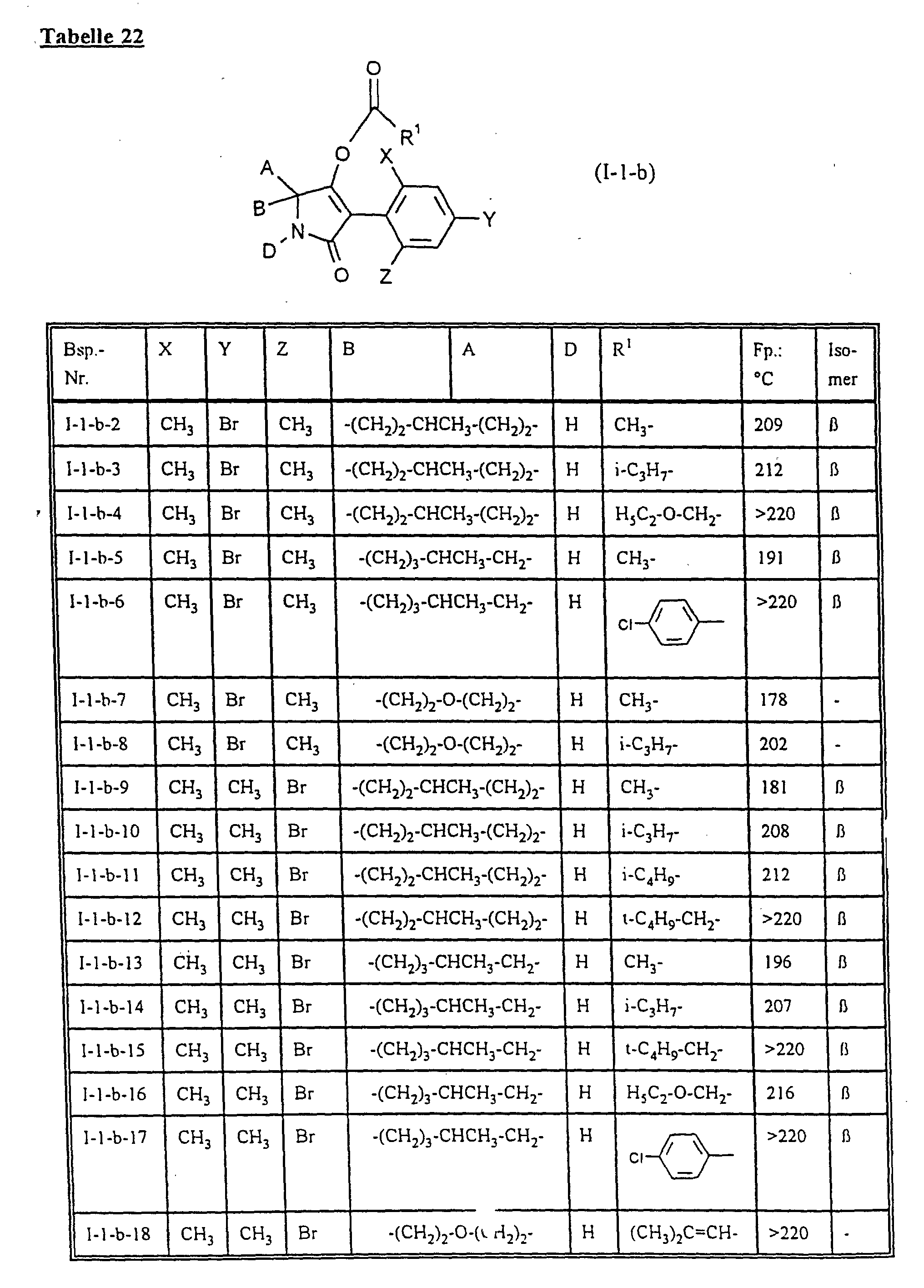

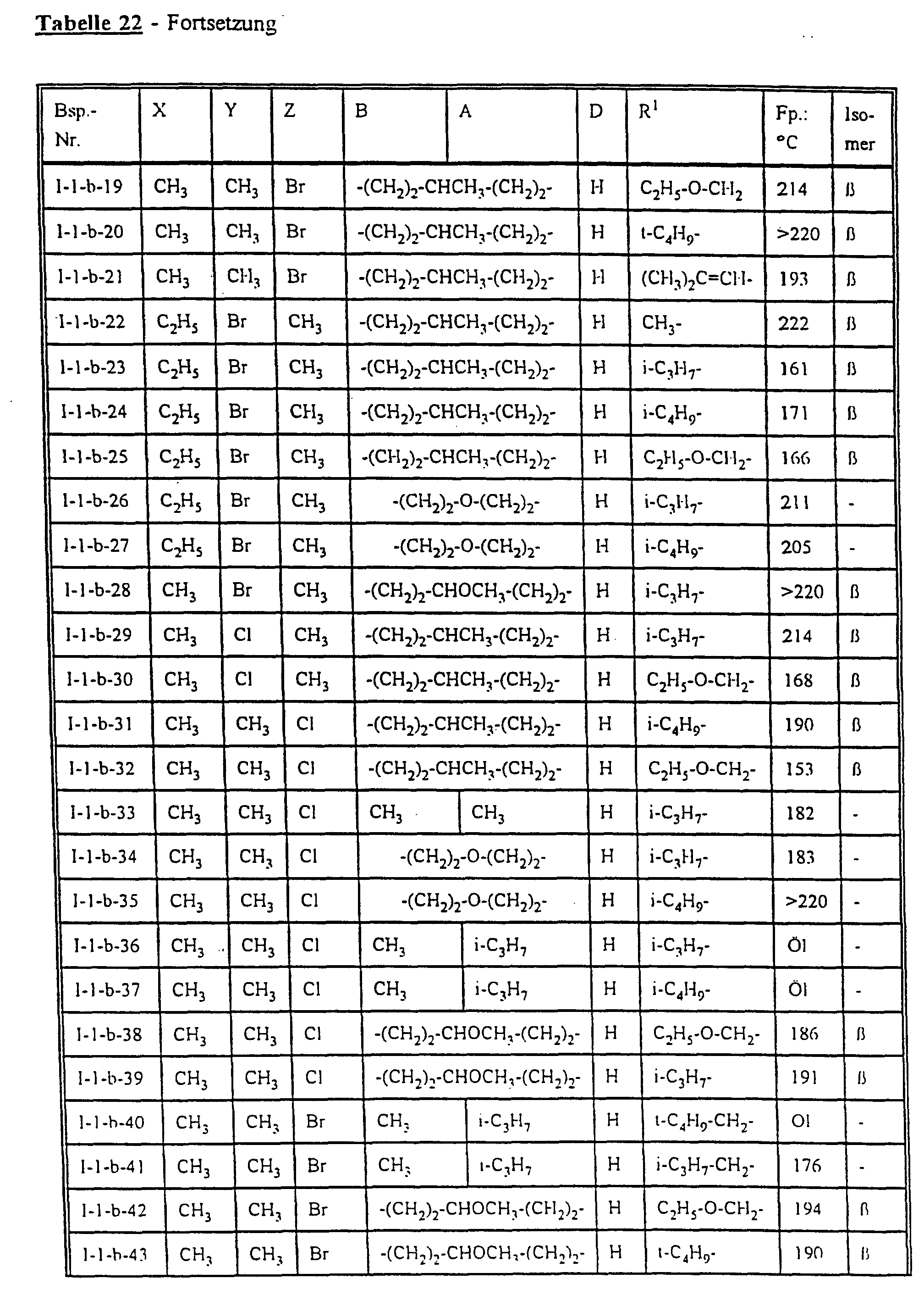

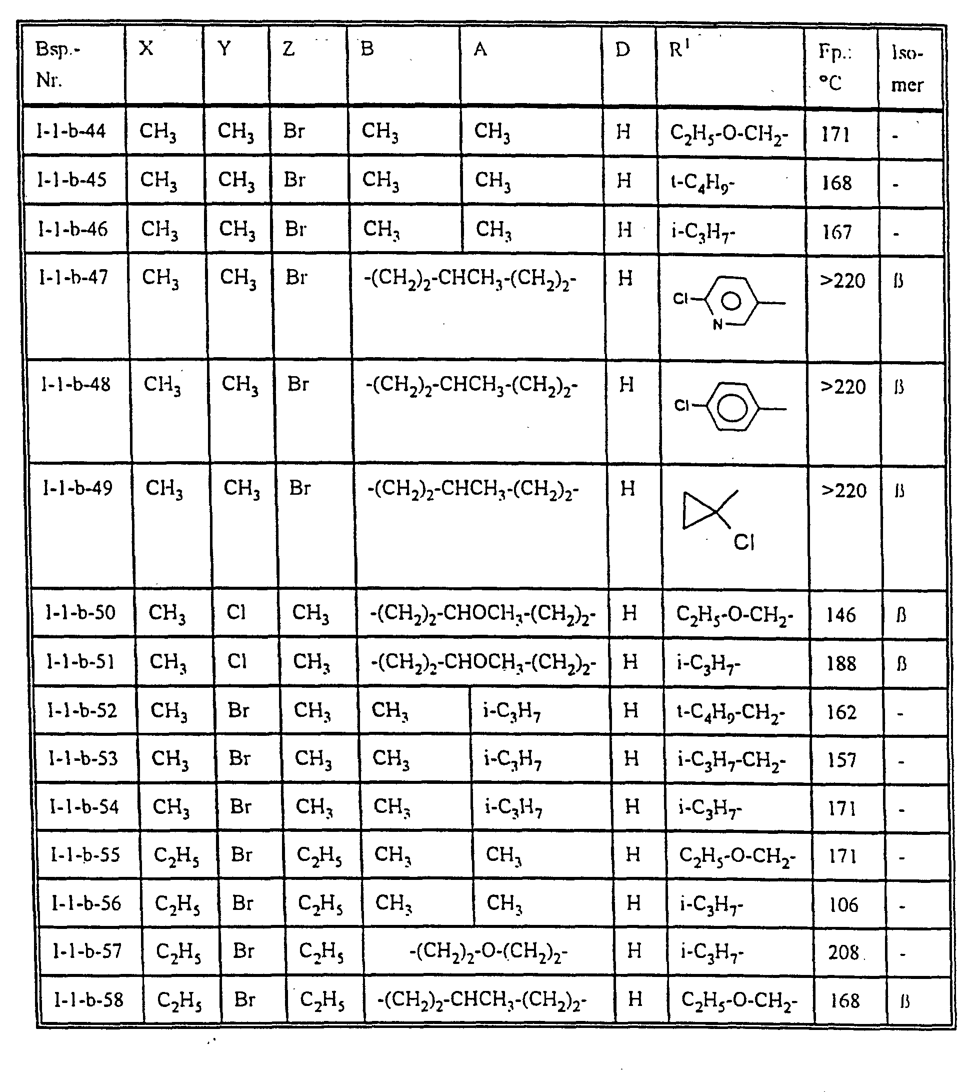

### Beispiel (I-1-c-1)

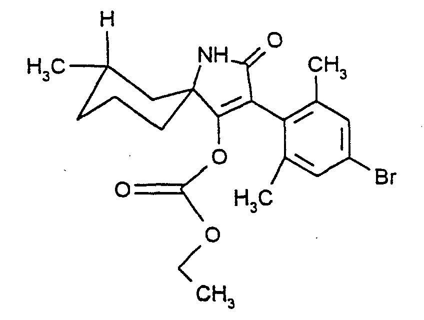

Zu 4,37 g der Verbindung gemäß Beispiel (I-1-a-1) in 70 ml absolutem Methylenchlorid und 1,7 ml (12 mMol) Triethylamin tropft man bei 0°C bis 10°C 1,2 ml Chlorameisensäureethylester in 3 ml absolutem Methylenchlorid. Man rührt bei Raumtemperatur, bis nach DC-Kontrolle die Umsetzung beendet ist.

Zur Aufarbeitung wäscht man 2 mal mit 0.5 N NaOH, trocknet und dampft ein. Das Rohprodukt wird aus MTB-Ether/n-Hexan umkristallisiert.

Ausbeute: 3,60 g (68 % der Theorie); Fp.: >220°C.

Analog zu Beispiel (I-1-c-1) bzw. gemäß den allgemeinen Angaben zur Herstellung wurden die in der nachfolgenden Tabelle 23 aufgeführten Verbindungen der Formel (I-1-c) hergestellt.

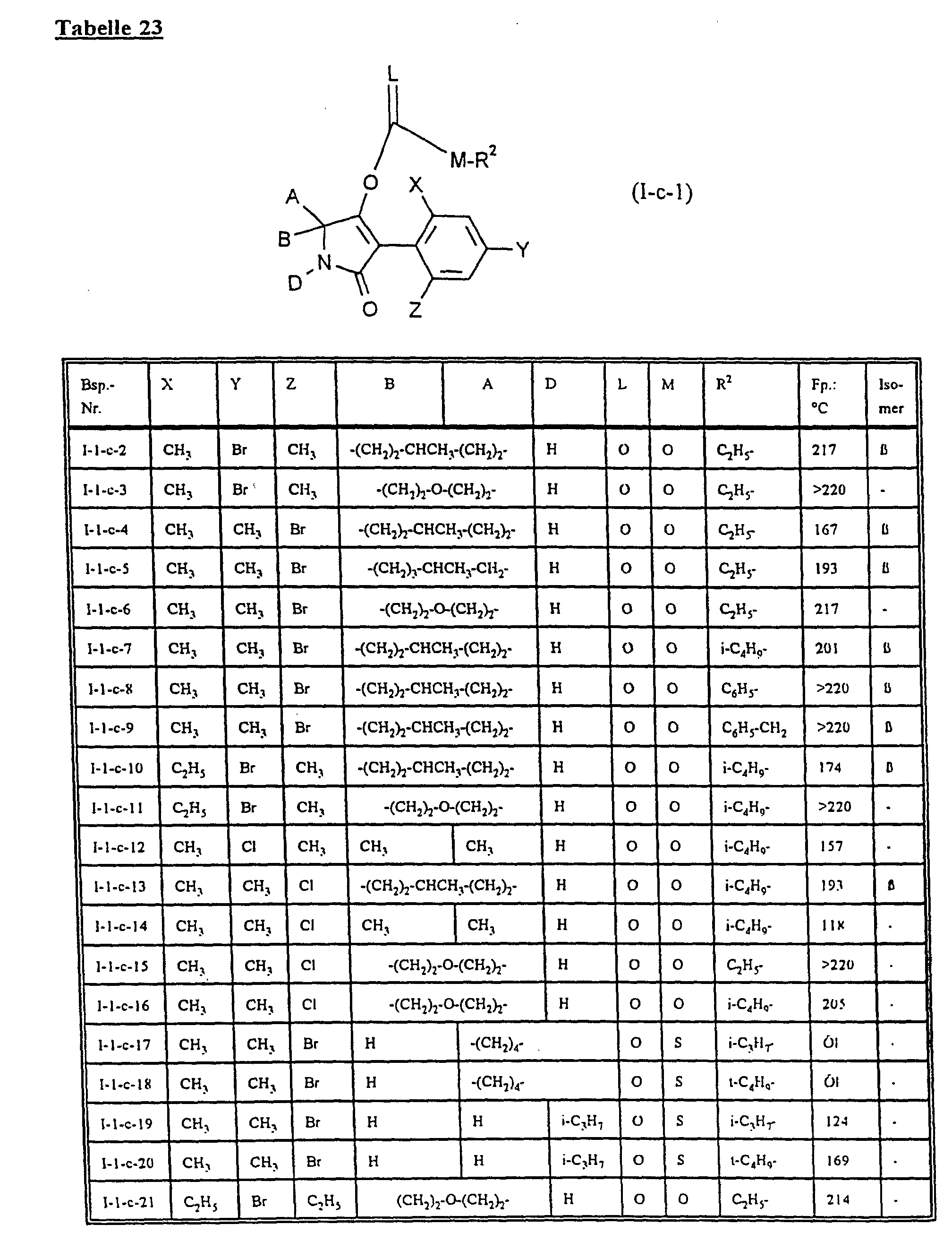

### Beispiel I-1-d-1

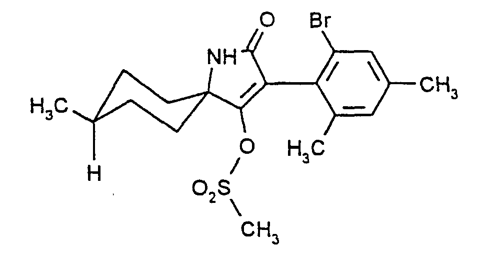

3,64 g der Verbindung gemäß Beispiel I-1-a-5 und 1,4 ml Triethylamin in 50 ml Abs. Methylenchlorid werden bei 0 bis 10°C mit 0,8 ml Methansulfonsäurechlorid in 5 ml abs. Methylenchlorid versetzt und anschließend bei Raumtemperatur gerührt. Nach beendeter Reaktion (Kontrolle mittels Dünnschichtchromatographie (DC)) wird 2 mal mit 50 ml 0,5 N NaOH gewaschen, über Magnesiumsulfat getrocknet, eingeengt und der Rückstand aus MTB-Ether/n-Hexan umkristallisiert. Ausbeute 2,90 g (65 % der Theorie), Fp. > 220°C.

### Beispiel (II-1)

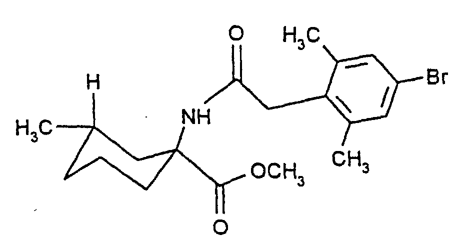

23,1 g 2,6-Dimethyl-4-brom-phenylessigsäure gemäß Beispiel (XXV-1) und 17,7 ml (0,24 Mol) Thionylchlorid werden bei 80°C bis zum Ende der Gasentwicklung gerührt. Bei 50°C wird überschüssiges Thionylchlorid abdestilliert und der Rückstand in 100 ml absolutem THF aufgenommen. Diese Lösung tropft man bei 0°C bis 10°C zum Gemisch von 20,9 g 1-Amino-3-methyl-cyclohexancarbonsäuremethylester in 200 ml absolutem THF und 30,8 ml (0,22 Mol) Triethylamin.

Zur Aufarbeitung wird abgesaugt, mit THF gewaschen, eingedampft und der Rückstand in Methylenchlorid aufgenommen. Man wäscht mit 0.5 N HCl, trocknet, dampft ein und kristallisiert den Rückstand aus MTB-Ether/n-Hexan um.

Ausbeute: 32,60 g (80 % der Theorie); Fp.: 137°C.

### Beispiel (II-2)

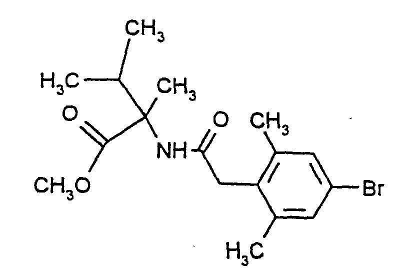

Zu 42 g (0,428 Mol) konz. Schwefelsäure gibt man bei 30 bis 40°C 28,8 g der Verbindung gemäß Beispiel (XXXI-1) in 170 ml Methylenchlorid und rührt noch 2 Stunden bei dieser Temperatur. Dann tropft man 57 ml absolutes Methanol so zu, daß sich eine Temperatur von 40°C einstellt. Nach beendeter Zugabe rührt man noch 6 Stunden bei 40 bis 70°C.

Zur Aufarbeitung gießt man auf Eis, extrahiert mit Methylenchlorid, wäscht mit NaHCO₃-Lösung, trocknet und dampft ein. Das Rohprodukt wird aus MTB-Ether/n-Hexan umkristallisiert.

Ausbeute: 20,7 g (65 % der Theorie); Fp.: 172°C.

Analog zu den Beispielen (II-1) und (II-2) bzw. gemäß den allgemeinen Angaben zur Herstellung wurden die in der nachfolgenden Tabelle 24 aufgeführten Verbindungen der Formel (II) hergestellt.

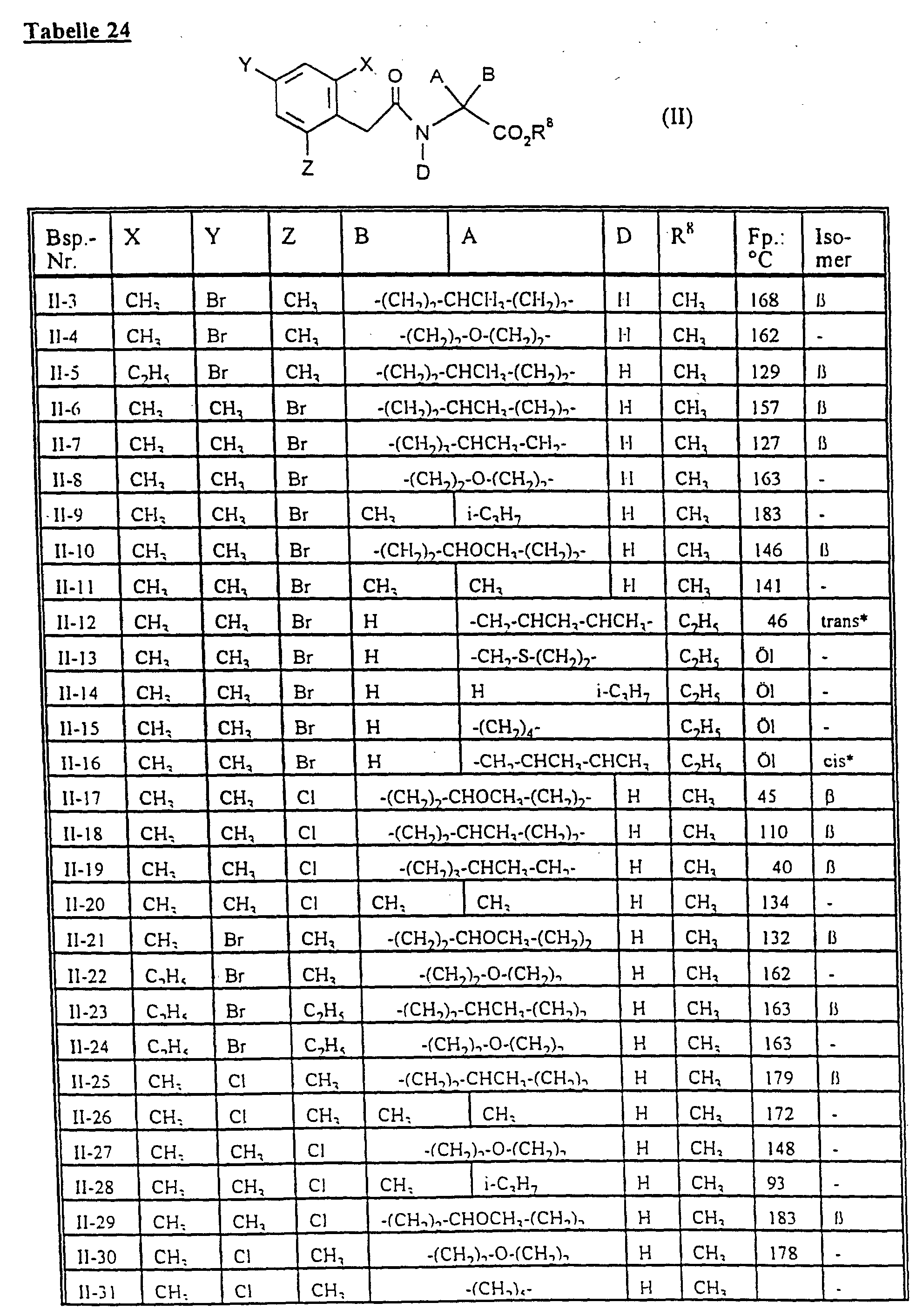

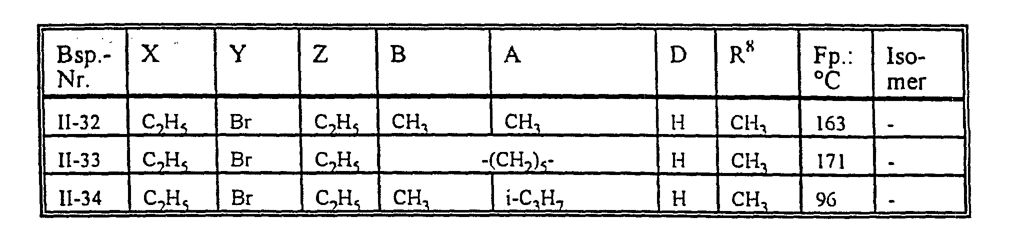

### Beispiel (XXXI-1)

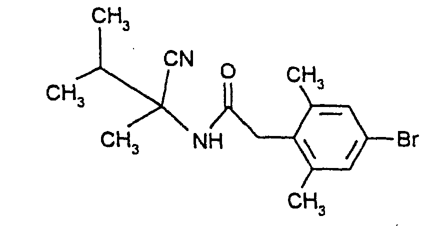

23,1 g 2,6-Dimethyl-4-bromphenylessigsäure gemäß Beispiel (XXV-1) und 17,7 ml Thionylchlorid werden bei 80°C gerührt, bis die Gasentwicklung beendet ist. Dann wird überschüssiges Thionylchlorid bei 50°C im Vakuum entfernt. Der Rückstand wird in 100 ml absolutem THF aufgenommen und bei 0 bis 10°C zu einer Mischung von 11,2 g des Amins der Formel (CH₃)₂CHC(CH₂)(CN)NH₂ und 14,4 ml (0,11 Mol) Triethylamin in 100 ml absolutem THF getropft. Anschließend wird noch eine Stunde bei Raumtemperatur gerührt.

Zur Aufarbeitung wird abgesaugt, eingeengt, der Rückstand in Methylenchlorid aufgenommen, in 0.5 N HCl gewaschen, getrocknet und eingeengt. Das Rohprodukt wird aus MTB-Ether/n-Hexan umkristallisiert.

Ausbeute: 28,8 g (85 % der Theorie); Fp.: 169°C.

Analog zu Beispiel (XXXI-1) wurden die in der nachfolgenden Tabelle 25 aufgeführten Verbindungen der Formel (XXXI) hergestellt.

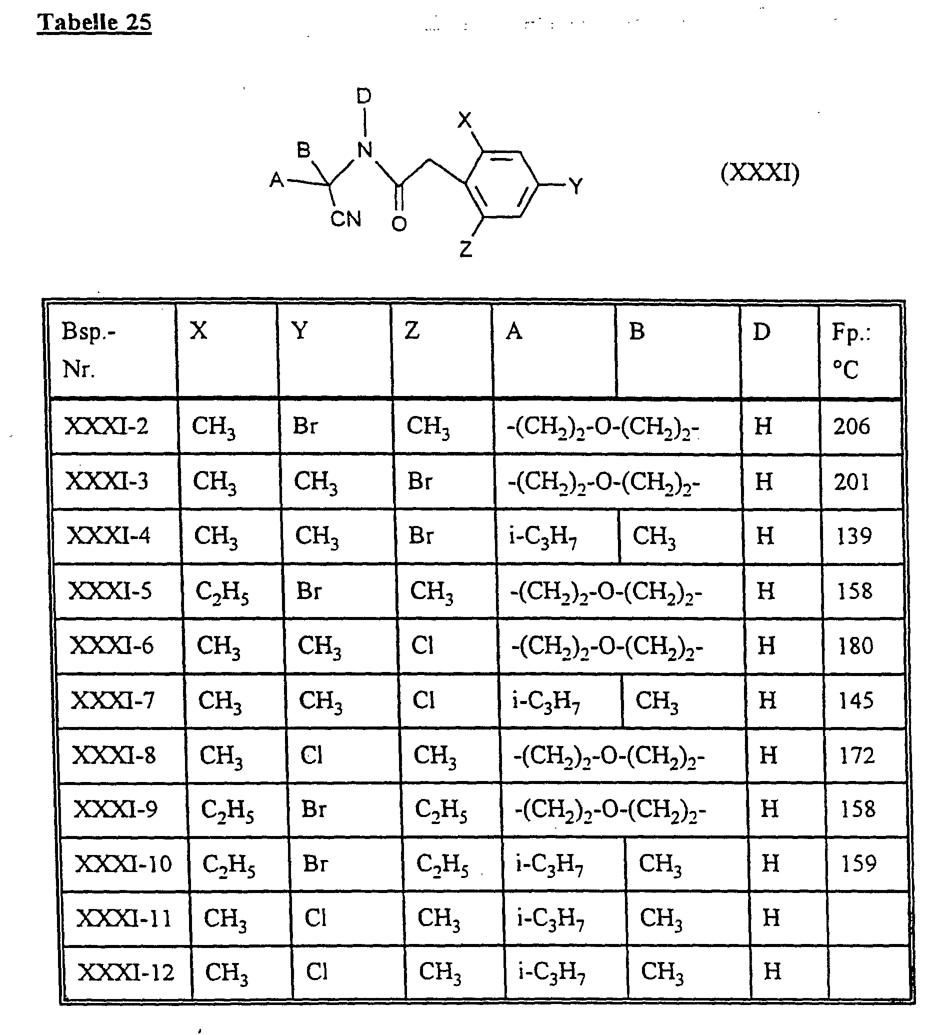

### Beispiel (I-2-a-1)

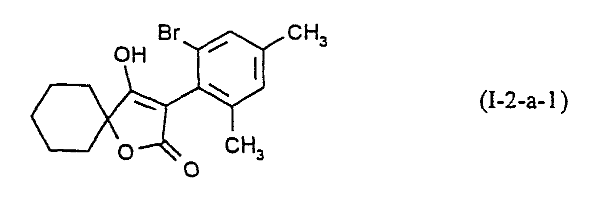

Zu 8,42 g (75 mMol) Kalium-tert.-butylat in 50 ml Dimethylformamid (DMF) tropft man bei 0 bis 10°C eine Lösung von 19,8 g (50 mMol) der Verbindung gemäß Beispiel (III-1) in 50 ml DMF und rührt über Nacht bei Raumtemperatur.

Zur Aufarbeitung tropft man das Reaktionsgemisch in 500 ml eiskalte 1 N HCl, saugt das ausgefallene Rohprodukt ab, wäscht mit Wasser und trocknet im Vakuumtrockenschrank. Zur weiteren Reinigung wird das Rohprodukt noch mit n-Hexan/Aceton ausgekocht.

Ausbeute: 13,6 g (77 % der Theorie); Fp.: >250°C.

Analog zu Beispiel (I-2-a-1) wurden die in der nachfolgenden Tabelle 26 aufgeführten Verbindungen der Formel (I-2-a) hergestellt.

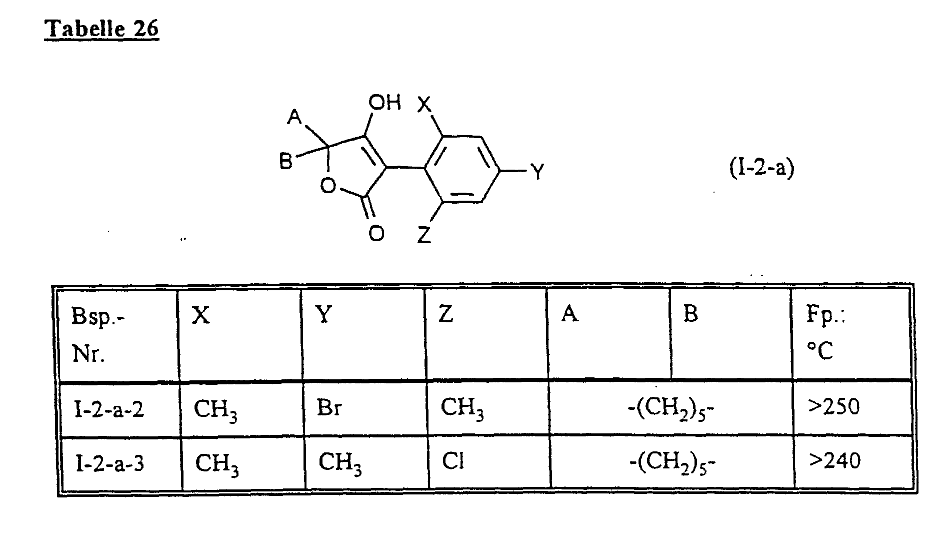

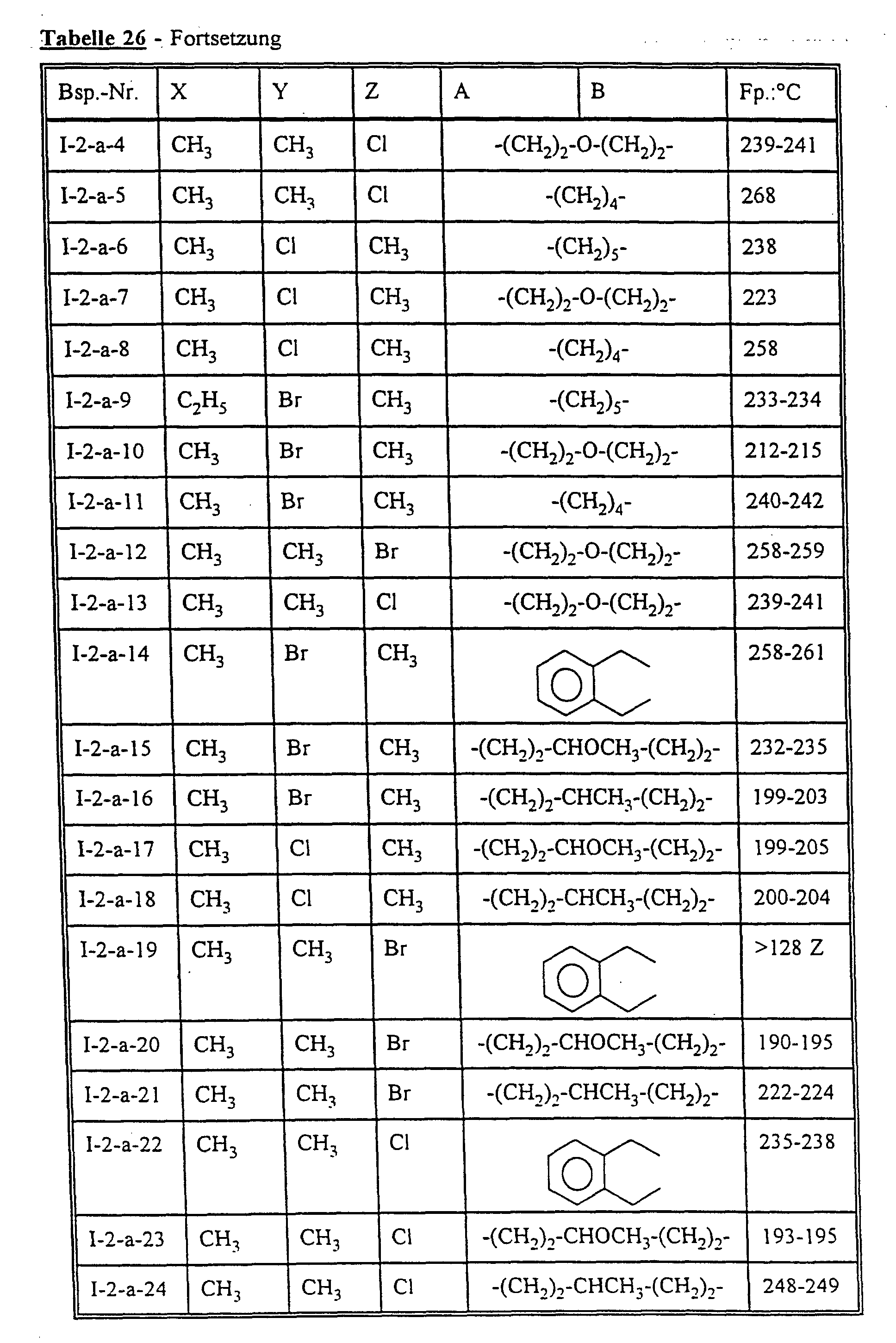

### Beispiel (I-2-b-1)

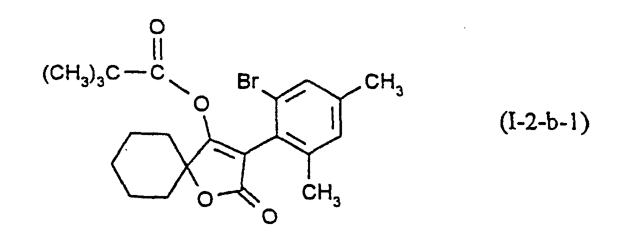

Zum Gemisch von 3,52 g (10 mMol) der Verbindung gemäß Beispiel (I-2-a-1) und 1,52 g (15 mMol) Triethylamin in 40 ml Methylenchlorid tropft man unter Eiskühlung eine Lösung von 1,57 g (13 mMol) Pivaloylchlorid in 40 ml Methylenchlorid und rührt 2 Stunden bei Raumtemperatur.

Zur Aufarbeitung wäscht man nacheinander mit 10 %iger Citronensäure, 1 N NaOH und NaCl-Lösung, trocknet und dampft ein. Zur weiteren Reinigung wird das Rohprodukt noch mit wenig Petrolether verrührt.

Ausbeute: 1,95 g (45 % der Theorie); Fp.: 107-109°C.

Analog zu Beispiel (I-2-b-1) wurden die in der nachfolgenden Tabelle 27 aufgeführten Verbindungen der Formel (I-2-b) hergestellt.

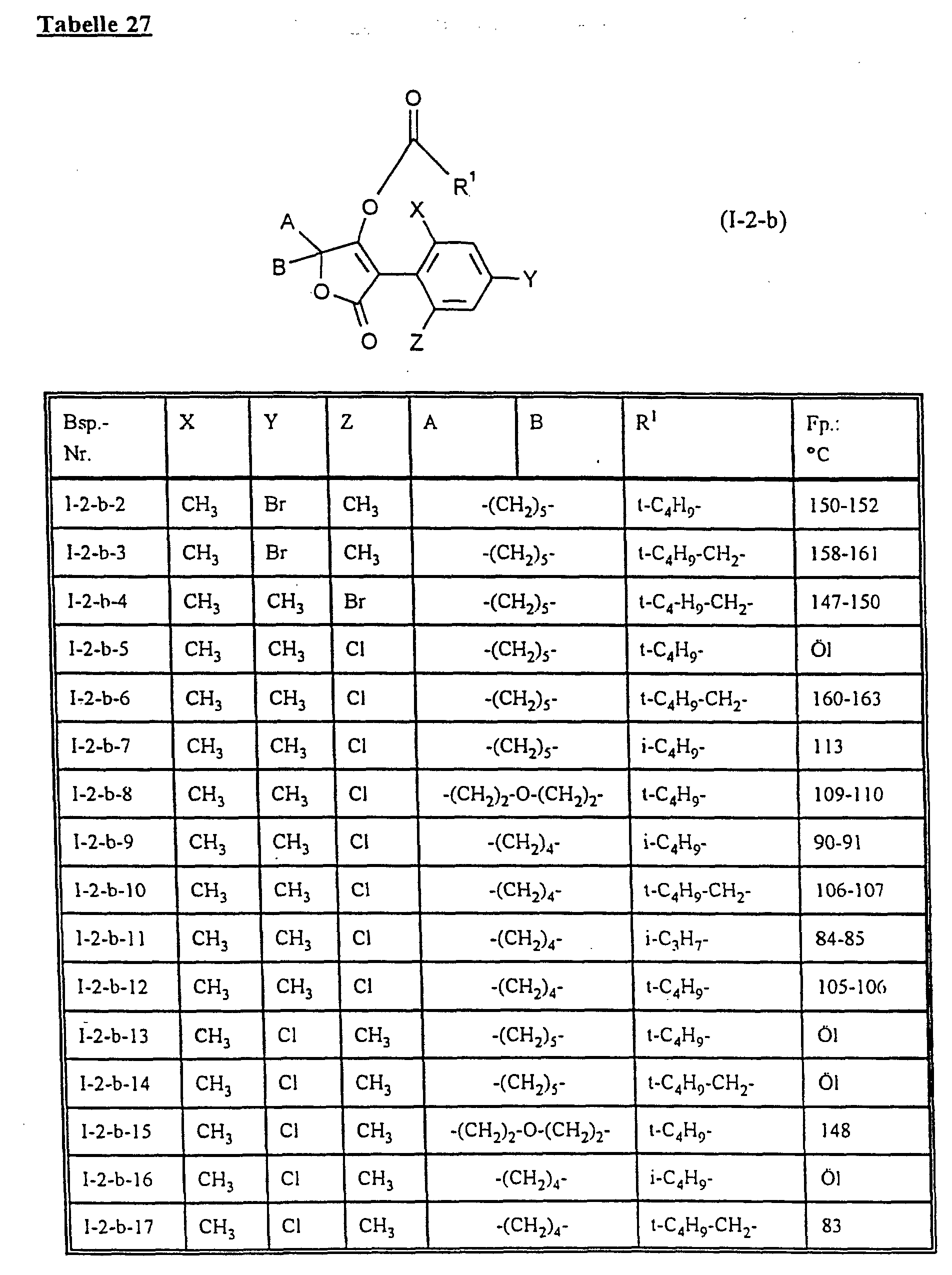

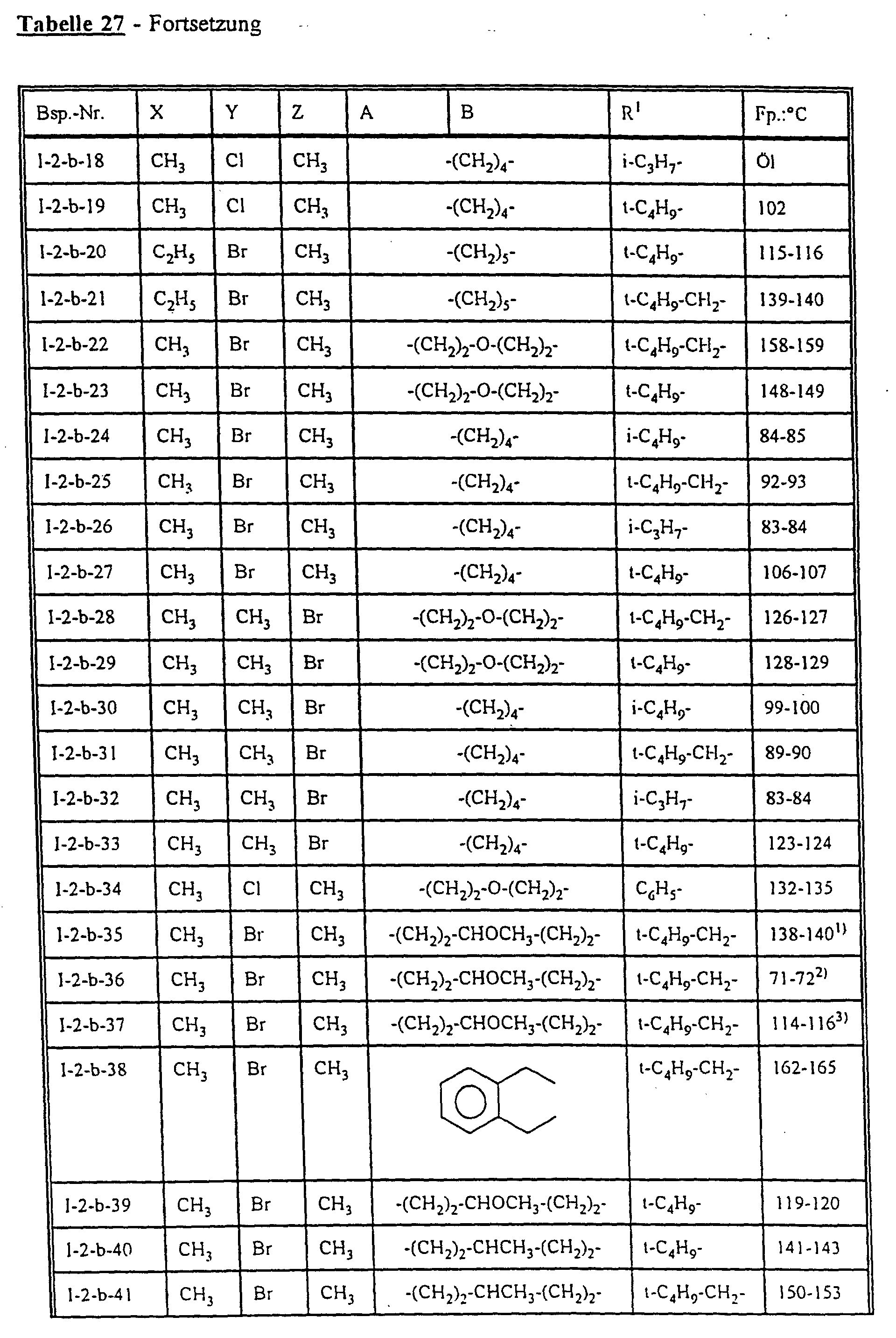

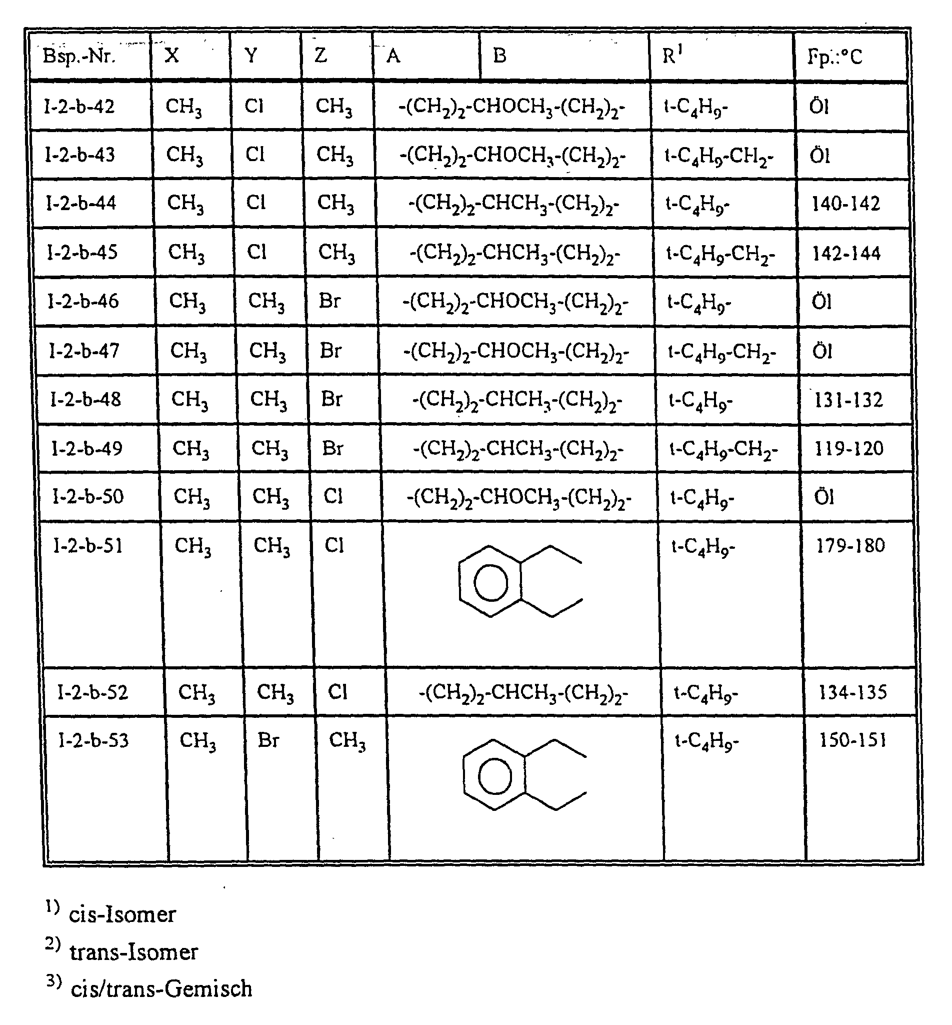

Analog zur Beispiel I-1-c-1 wurden die in der Tabelle 28 aufgeführten Verbindungen der Formel I-2-c erhalten.

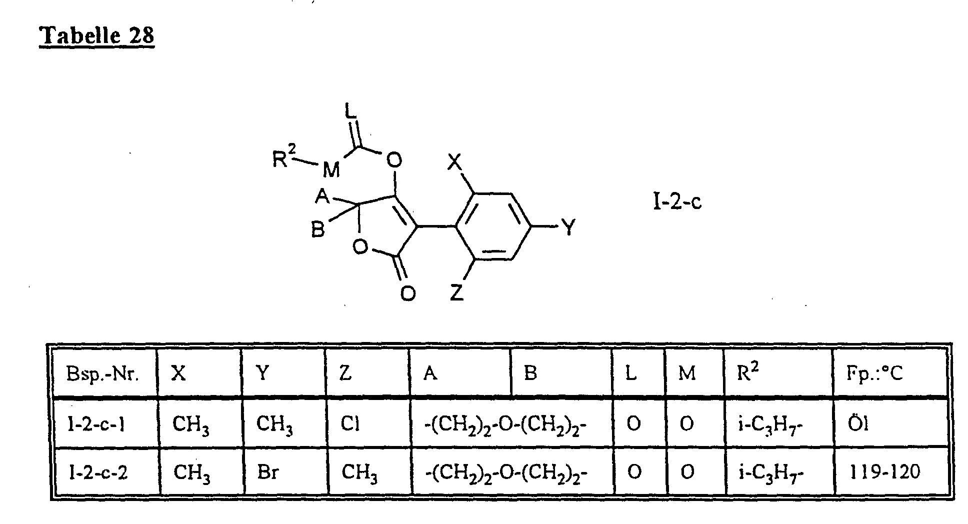

### Beispiel (III-1)

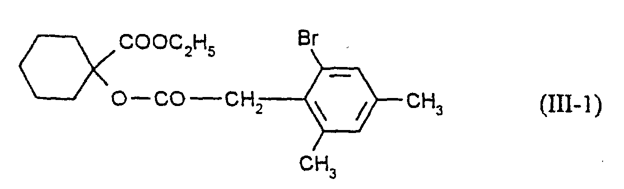

12,15 g (50 mMol) der Verbindung gemäß Beispiel (XXV-2) und 11,9 g (100 mMol) Thionylchlorid werden in 50 ml Toluol bei 80°C bis zum Ende der Gasentwicklung gerührt. Dann wird zur Trockne eingedampft und das so erhaltene rohe Säurechlorid zusammen mit 8,6 g (50 mMol) 1-Hydroxycyclohexancarbonsäureethylester über Nacht in 50 ml Toluol unter Rückfluß erhitzt. Anschließend wird eingeengt.

Ausbeute: 19,8 g (quantitativ); farbloses Öl.
- ¹H-NMR: δ =: 1.20 (t, 3H); 1.40-1.80 (m, 8H); 2.15 (m, 2H); 2.25 (s, 3H); 2.35 (s, 3H); 3.90 (s, 2H); 4.15 (q, 2H); 6.95 (m, 1H); 7.25 (m, 1H).

Analog bzw. gemäß den allgemeinen Angaben zur Herstellung erhält man die in der Tabelle 29 aufgeführten Verbindungen der Formel (III).

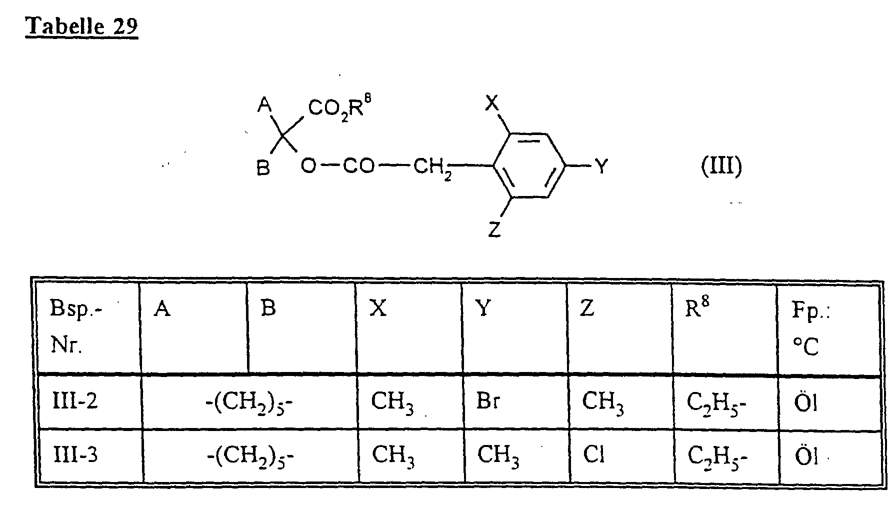

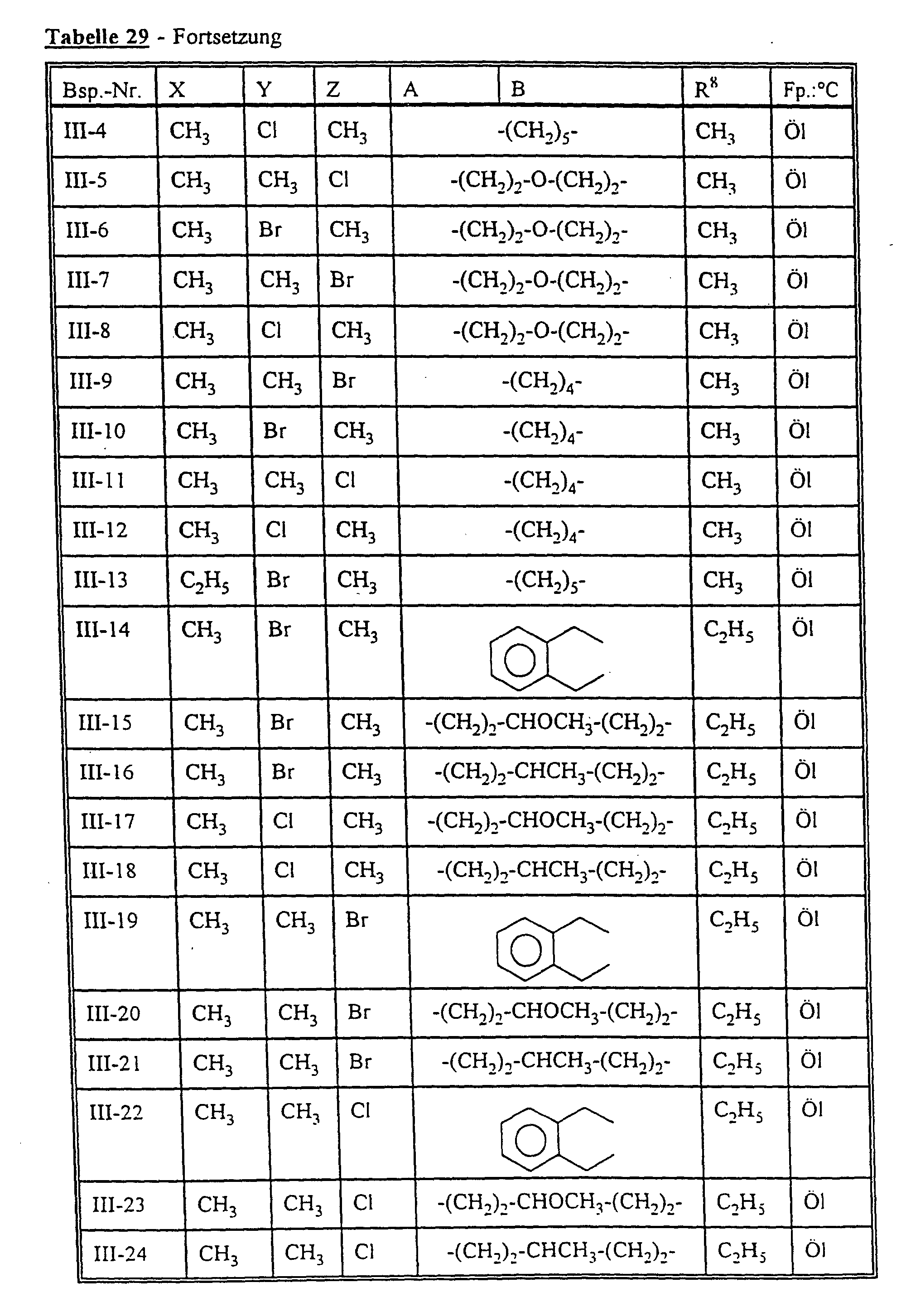

### Beispiel I-3-a-1

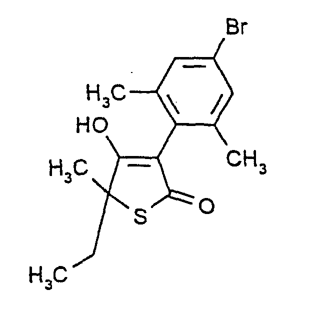

34,0 g (69 mmol) der Verbindung gemäß Beispiel (IV-1) werden in 70 ml Trifluoressigsäure und 140 ml Toluol 3 Stunden unter Rückfluß erhitzt. Anschließend wird die Trifluoressigsäure in Vakuum entfernt und der Rückstand mit 400 ml Wasser und 120 ml MTB-Ether versetzt. Durch Zugabe von NaOH wird pH 14 eingestellt, dann 2 mal mit MTB-Ether extrahiert. Die wäßrige Phase wird mit HCl angesäuert und 3 mal mit MTB-Ether extrahiert. Nach dem Trocknen wird die organische Phase eingeengt. Ausbeute 13,0 g (55 % der Theorie), Fp. 235-238°C.

Analog zu Beispiel I-3-a-1 wurden die in der Tabelle 30 aufgeführten Verbindungen der Formel I-3-a hergestellt.

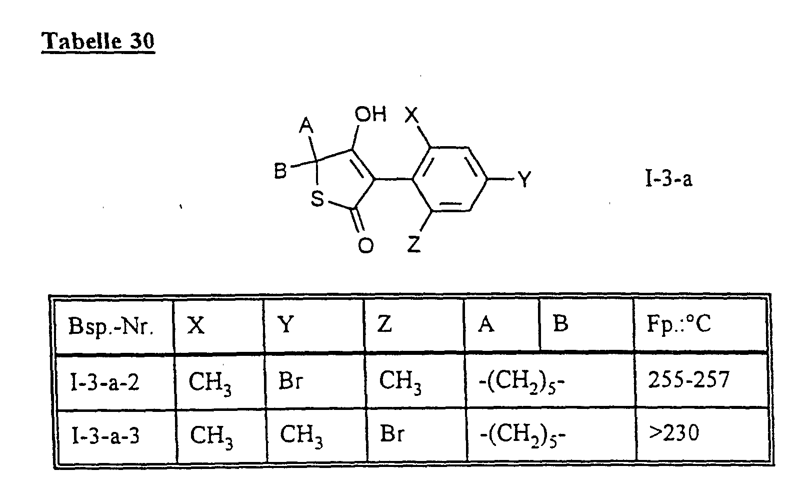

### Beispiel I-3-b-1

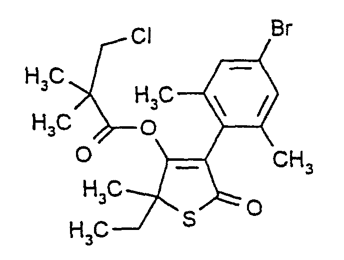

Unter Eiskühlung tropft man eine Lösung von 0,74 ml (0,89 g; 5,72 mmol) 3-Chlor-2,2-dimethylpropionsäurechlorid in 3 ml Methylenchlorid zum Gemisch von 1,5 g (4,4 mmol) der Verbindung gemäß Beispiel (I-3-a-1), 0,92 ml Triethylamin und 20 ml Methylenchlorid und rührt anschließend 2 Stunden bei Raumtemperatur.

Dann wäscht man 2 mal mit 10%iger Citronensäure und extrahiert die sauren wäßrigen Phasen mit Methylenchlorid. Die vereinigten organischen Phasen werden 2 mal mit 1N NaOH gewaschen, die wäßrigen alkalischen Phasen mit Methylenchlorid extrahiert. Die vereinigten organischen Phasen werden getrocknet und eingeengt. Ausbeute 1,65 g (81 % der Theorie), Öl.
¹H-NMR in CDCl₃, ppm
- δ =: 1,05 (t, 3H, CH₂CH₃)
1,18 (s, 6H, C(CH₃)₂)
1,62 (s, 3H, CCH₃)
1,95-2,05 (m, 2H, CH₂CH₃)
2,08 (s, 3H, ArCH₃)
2,10 (s, 3H, ArCH₃)
3,38 (s, 2H, CH₂Cl)
7,20 (s, 2H, ARH)
Analog zu Beispiel I-3-b-1 wurden die in der Tabelle 31 aufgeführten Verbindungen der Formel I-3-b hergestellt.

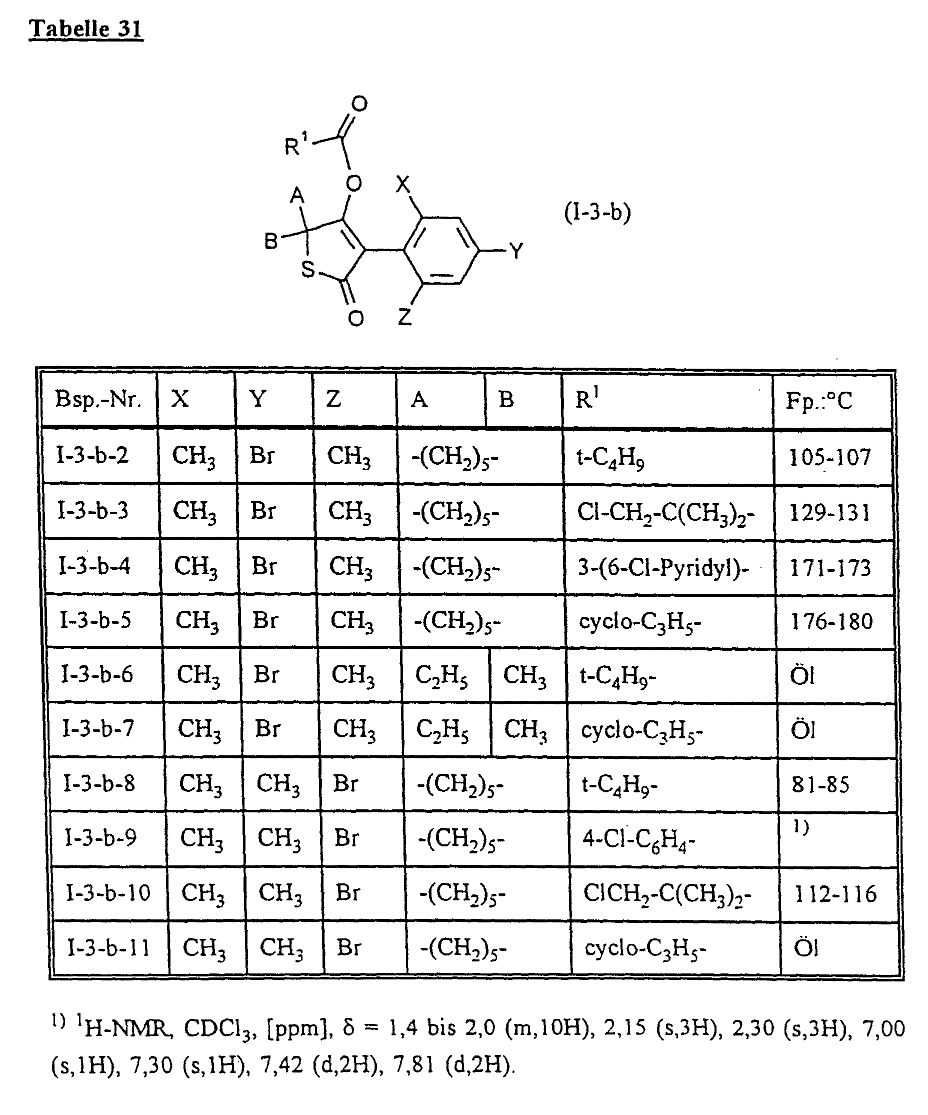

### Beispiel I-3-c-1

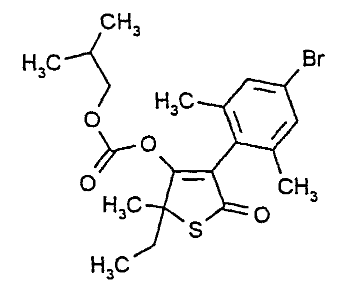

Unter Eiskühlung tropft man eine Lösung von 0,74 ml (5,72 mmol) Chlorameisensäureisobutylester in 3 ml Methylenchlorid zum Gemisch von 1,5 g (4,4 mmol) der Verbindung gemäß Beispiel (I-3-a-1), 0,92 ml Triethylamin und 20 ml Methylenchlorid. Man rührt 2 Stunden bei Raumtemperatur und arbeitet wie bei Beispiel I-3-b-1 beschrieben auf. Der am Ende verbleibende Rückstand wird mit Petrolether verrührt. Ausbeute 2,0 g (100 % der Theorie)
- ¹H-NMR, CDCl₃, [ppm], δ =: 0,68 (d, 6H, CH(CH₃)₂)
1,04 (t, 3H, CH₂CH₃)
1,5-1,6 (m, 1H, CH(CH₃)₂)
1,71 (m, 2H, CCH₃)
1,9-2,0 (m, 2H, CH₂CH₃)
2,08 (s, 3H, ArCH₃)
2,12 (s, 3H, ArCH₃)
3,61 (d, 2H, OCH₂)
7,12 (s, 2H, ArH)

Analog zu Beispiel I-3-c-1 wurden die in der Tabelle 32 aufgeführten Verbindungen der Formel I-3-c hergestellt.

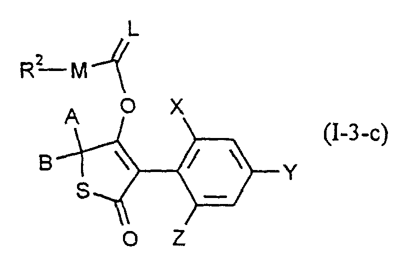

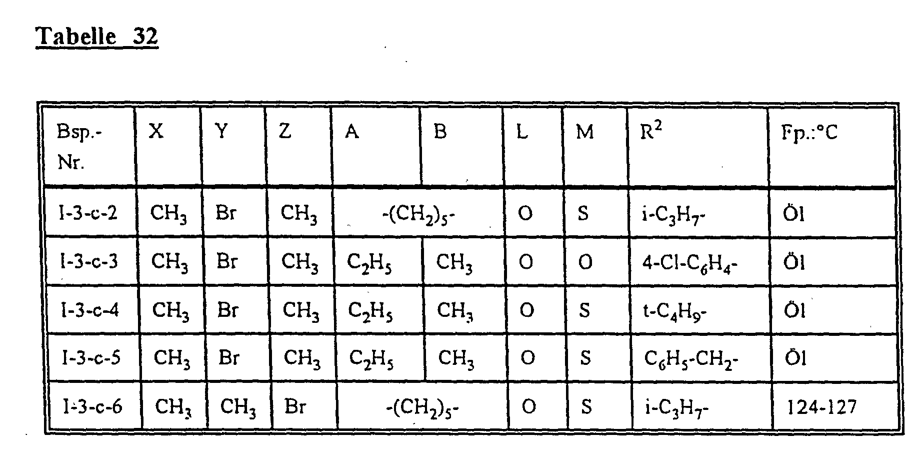

### Beispiel IV-1

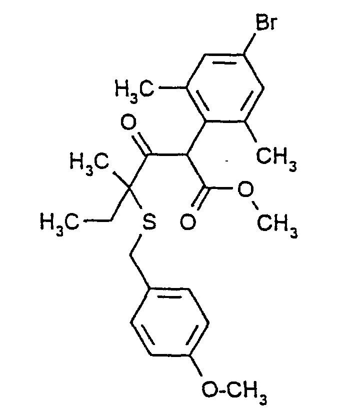

A: Das Gemisch von 25,0 g (98 mmol) der Verbindung der Formel 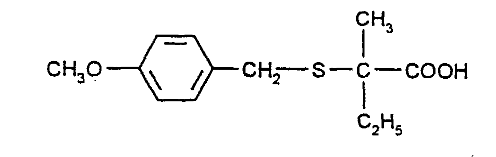 1 Tropfen DMF und 17,5 g (147 mmol) Thionylchlorid in 70 ml Toluol wird 5 Minuten bei Raumtemperatur und anschließend bei 100°C bis zum Ende der Gasentwicklung gerührt. Überschüssiges Thionylchlorid wird im Vakuum entfernt.
B: Zum Gemisch von 13,0 g (129 mmol) Diisopropylamin und 71,6 ml (118 mmol) Butyllithium (1,6 M in n-Hexan) in 100 ml THF tropft man bei 0°C 27,7 g der Verbindung gemäß Beispiel (XXVI-1) in 40 ml THF und rührt 30 Minuten. Dann tropft man das unter A hergestellte Säurechlorid gelöst in 40 ml THF bei 0°C zu und rührt 1 Stunde bei Raumtemperatur.
   Man gibt 350 ml MTB-Ether und einige Tropfen Wasser zu, wäscht 2 mal mit 10%iger Ammoniumchloridlösung, trocknet die organische Phase und engt ein. Das Rohprodukt wird an Kieselgel chromatographiert (Laufmittel Cyclohexan/Essigester 20/1 bis 5/1). Ausbeute 35,0 g (72 % der Theorie). ¹H-NMR, CDCl₃, [ppm]; δ = 0,9 bis 1,0 (m, 3H), 1,43 (s, 3H), 1,7 bis 2,0 (m, 2H), 2,3 bis 2,4 (s, 6H), 3,6 bis 3,8 (m, 8H), 6,7 bis 7,2 (m, 6H)

Analog zu Beispiel IV-1 und gemäß der allgemeinen Beschreibung wurden die in der nachfolgenden Tabelle aufgeführten Verbindungen der Formel IV erhalten.

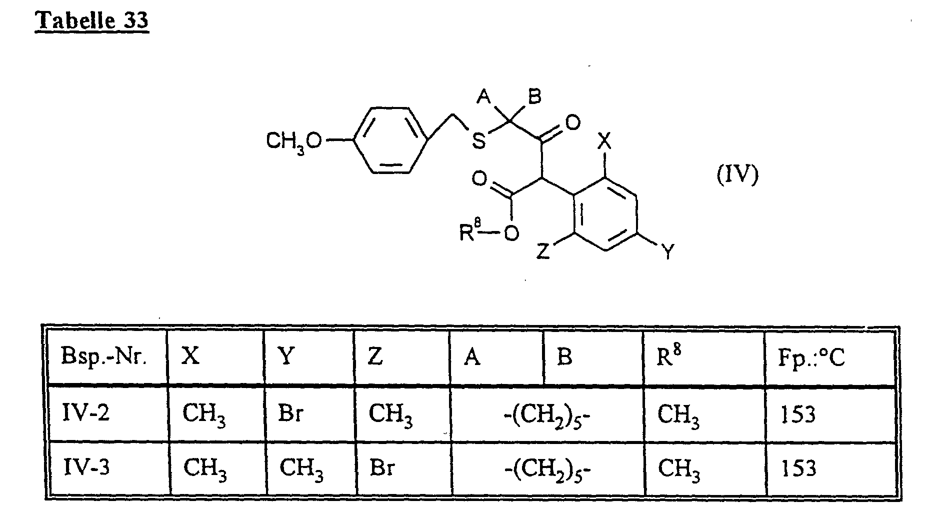

### Beispiel (I-5a-1)

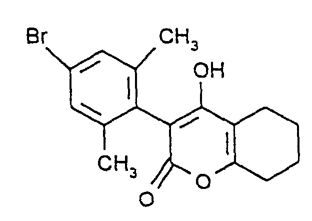

5,7 (20 mmol) 2-(4-Brom-2,6-dimethylphenyl)-chlorcarbonylketen werden mit 2,0 g (20 mmol) Cyclohexanon in 60 ml Xylol 8 h unter Rückfluß erhitzt. Der ausfallende Niederschlag wird abgetrennt, mit Cyclohexan gewaschen und getrocknet. Man erhält 5,0 g (72 % der Theorie) vom Schmelzpunkt 244 bis 245°C.

Analog bzw. gemäß den allgemeinen Angaben zur Herstellung erhält man die folgenden Verbindungen der Formel (I-5a):

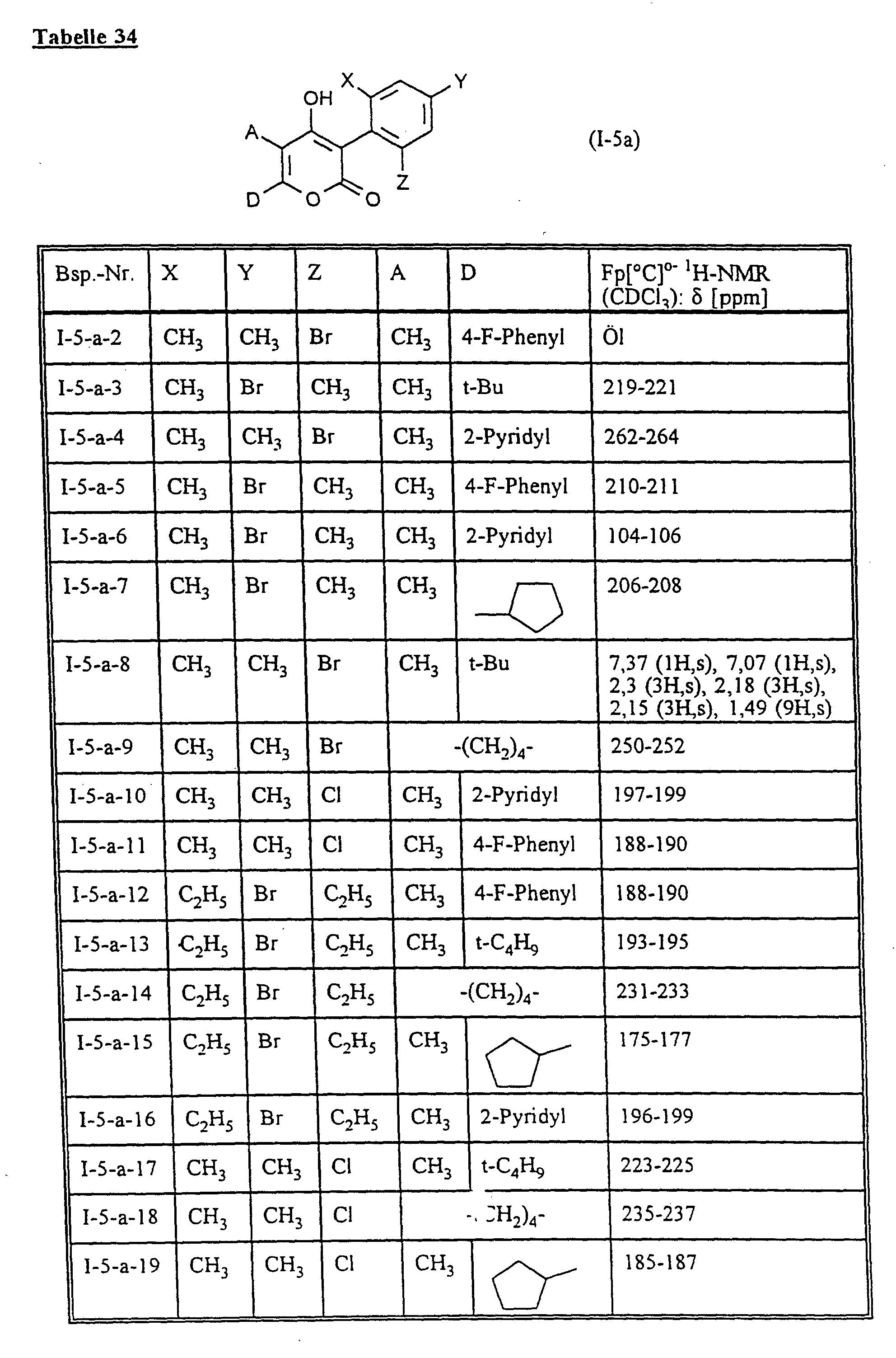

### Beispiel (I-5-b-1)

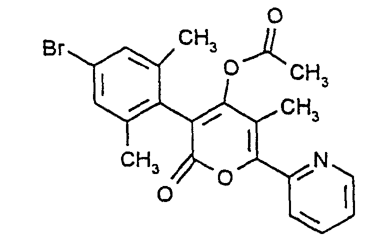

1,9 g (5 mmol) der Verbindung (1-5-a-6) werden in 20 ml Essigester vorgelegt, mit 0,5 g (5 mmol) Triethylamin versetzt und bei 0°C 0,4 g (5 mmol) Acetylchlorid in 5 ml Essigester zugetropft. Man rührt 20 h bei Raumtemperatur, trennt den Niederschlag ab, wäscht zweimal mit 50 ml halbkonzentrierter Natriumchloridlösung, trocknet über Natriumsulfat und dampft im Vakuum ein. Der Rückstand wird an Kieselgel mit Toluol/Aceton 30:1 chromatographiert. Ausbeute 1,2 g (56 % der Theorie) vom Schmelzpunkt 130 bis 132°C.

### Beispiel (I-5-c-1)

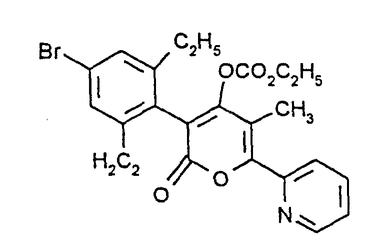

Unter Feuchtigkeitsausschluß legt man 1,8 g (4,2 mmol) 3-(4-Brom-2,6-diethylphenyl)-4-hydroxy-5-methyl-6-(pyrid-2-yl)-2-pyron in 10 ml abs. Pyridin und 15 ml abs. Ethylacetat vor und tropft unter Kühlung bei 0°C 0,46 g (4,2 mmol) Chlorameisensäureethylester in 5 ml abs. Ethylacetat zu. Anschließend wird bei 20°C 20 h nachgerührt und der Umsatz per DC verfolgt. Man dampft im Vakuum ein, nimmt den Rückstand in 50 ml Methylenchlorid auf, wäscht zweimal mit je 20 ml halb gesättigter Kochsalzlösung, trocknet über Magnesiumsulfat und dampft im Vakuum ein. Man erhält 1,8 g Rohprodukt. Durch "Flash"-Chromatographie an 200 g Kieselgel (35-70 µm) mit Toluol:Aceton 20:1 als Laufmittel, erhält man 1,2 g (59 % der Theorie) 4-Ethoxycarbonyloxy-3-(4-brom-2,6-diethylphenyl)-5-methyl-6-(pyrid-2-yl)-2-pyron vom Schmelzpunkt 114-117°C.

### Beispiel (I-6-a-1)

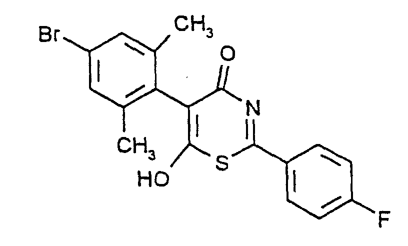

2,8 g (10 mmol) 2-(4-Brom-2,6-dimethylphenyl)-chlorcarbonylketen werden mit 1,6 g (10 mmol) 4-Fluorthiobenzamid in 80 ml Toluol 6 h auf 50°C erwärmt. Der Niederschlag wird abgetrennt, mit Cyclohexan gewaschen und getrocknet. Man erhält 3,0 g (74 % der Theorie) vom Schmelzpunkt 275 bis 276°C.

Analog bzw. gemäß den allgemeinen Angaben zur Herstellung erhält man die folgende Verbindung der Formel (I-6-a-2) vom Schmelzpunkt 235 bis 236°C.

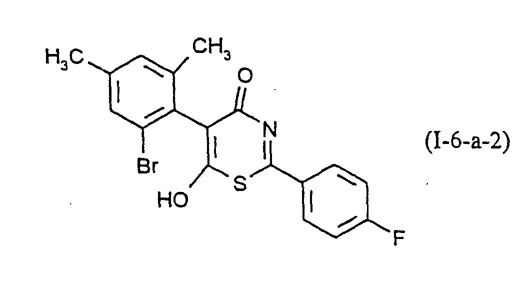

### Beispiel (XXII-1)

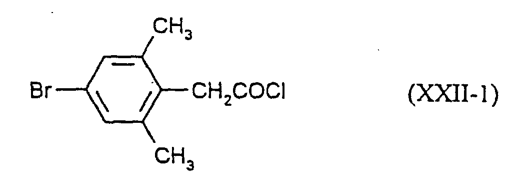

8 g der Verbindung gemäß Beispiel (XXV-1) werden mit 8,7 ml Thionylchlorid bis zum Ende der Gasentwicklung auf 80°C erhitzt. Überschüssiges Thionylchlorid wird im Vakuum entfernt und der Rückstand destilliert.

Ausbeute: 87 % der Theorie; Fp.: 69-71°C.

Analog bzw. gemäß den allgemeinen Angaben zur Herstellung erhält man die folgenden Verbindungen der Formel (XXII).

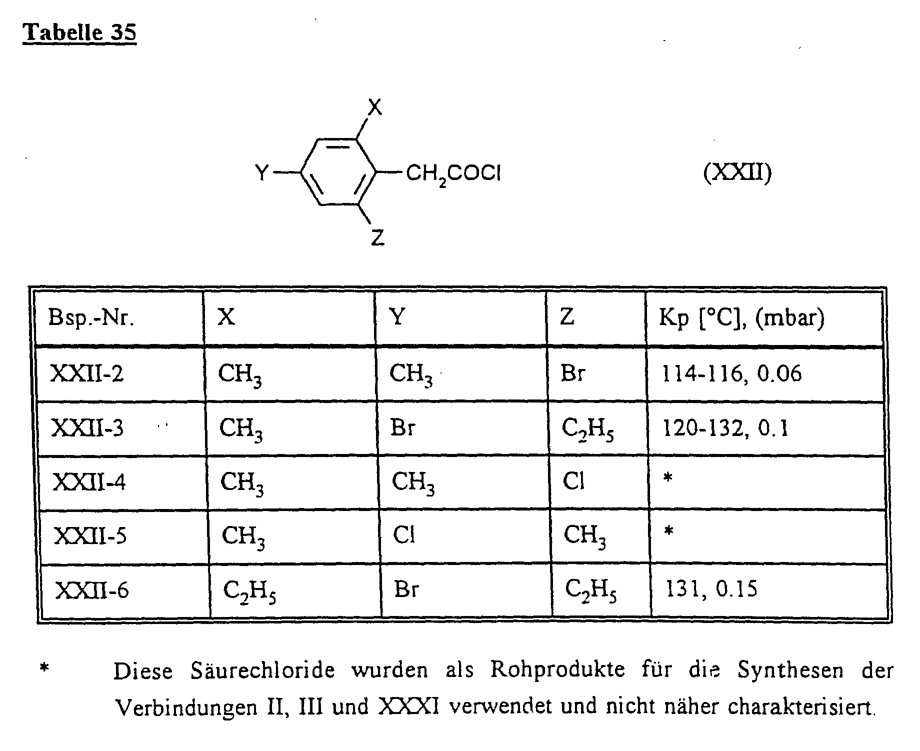

### Beispiel (XXV-1)

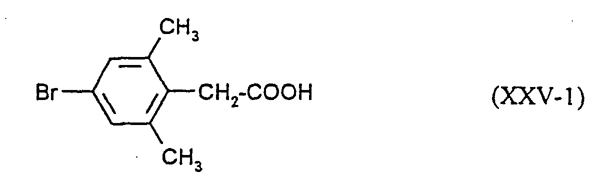

222,4 g (0,865 Mol) der Verbindung gemäß Beispiel (XXVI-1) und 80,56 g (1,438 Mol) Kaliumhydroxid in 210 ml Methanol und 105 ml Wasser werden 5 Stunden unter Rückfluß erhitzt. Nach dem Abkühlen engt man ein und löst den Rückstand in Wasser. Die wäßrige Phase wird mit Essigsäureethylester gewaschen und dann mit verdünnter Salzsäure gewaschen. Das ausgefallene Produkt wird abgeesaugt, mit Wasser gewaschen und getrocknet.

Ausbeute: 197,5 g (94 % der Theorie); Fp.: 185-187°C.

Analog bzw. gemäß den allgemeinen Angaben zur Herstellung erhält man die in der Tabelle 36 aufgeführten Verbindungen der Formel (XXV).

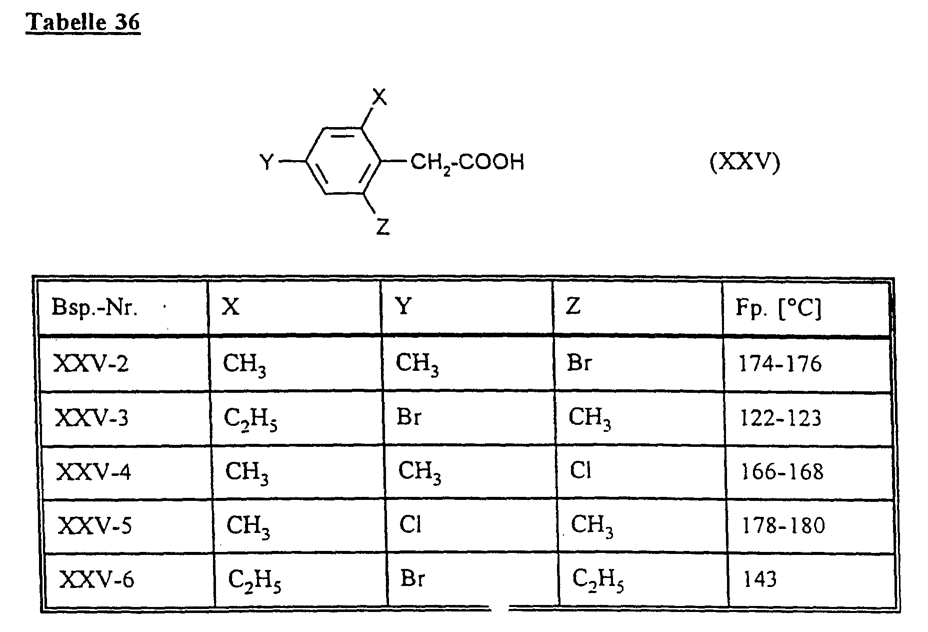

### Beispiel (XXVI-1)

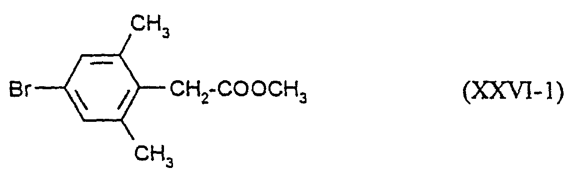

349,3 g (1,044 Mol) der Verbindung gemäß Beispiel (XXVII-1) (94,57 %ig), 475 ml Methanol und 842 ml 30 %ige Natriummethanolatlösung in Methanol werden 5 Stunden unter Rückfluß erhitzt. Dann fügt man bei Raumtemperatur 126 ml konz. Schwefelsäure zu und erhitzt 1 Stunde unter Rückfluß. das Lösungsmittel wird abdestilliert, der Rückstand mit Wasser versetzt und mit Methylenchlorid extrahiert. Nach dem Trocknen wird filtriert, eingeenngt und schließlich destilliert.

Ausbeute: 222,4 g (82,9 % der Theorie); Kp_{0.2} 98-100°C.

Analog bzw. gemäß den allgemeinen Vorschriften zur Herstellung erhält man die folgenden Verbindungen der Formel (XXVI).

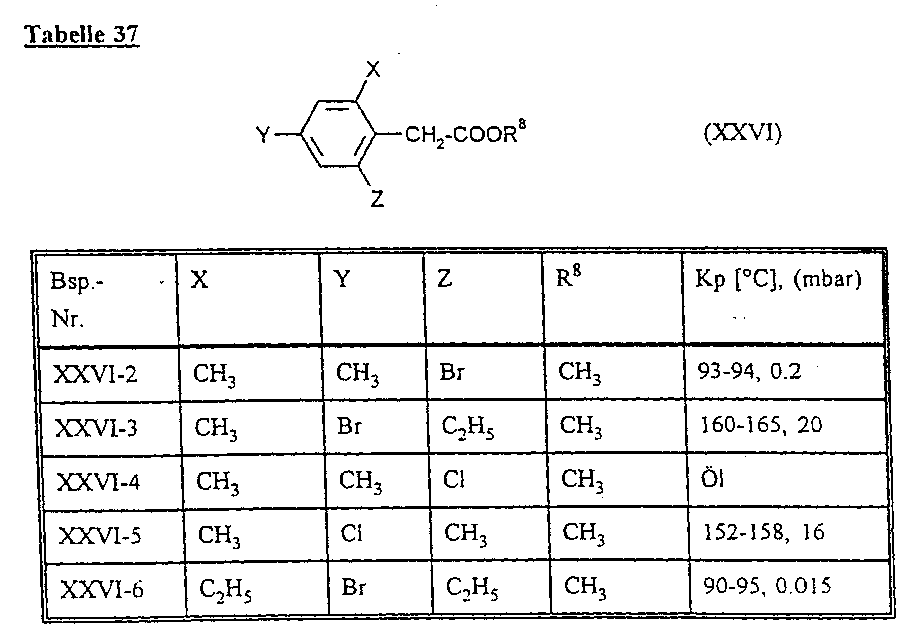

### Beispiel (XXVII-1)

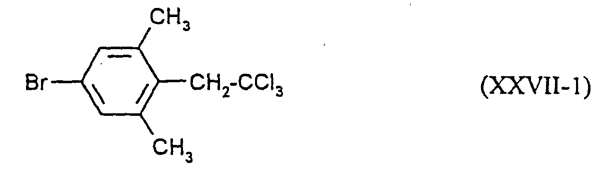

In eine Lösung von 326 g (3,175 Mol) tert.-Butylnitrit in 1270 ml tr. Acetonitril gibt man 326 g (2,673 Mol) wasserfreies Kupfer-II-chlorid. Zu der gut gekühlten Mischung tropft man 3130 g (32,27 m Δ 2580 ml) 1,1-Dichlorethan, wobei man mittels Eiskühlung auf unter 30°C hält. Bei unter 30°C tropft man dann eine Lösung von 424 g (2,12 Mol) 4-Brom-2,6-dimethylanilin in 2120 ml Acetonitril zu. Man rührt bei Raumtemperatur so lange nach, bis die Gasentwicklung (N₂) beendet ist (ca. 3 Stunden). Die fast schwarze Lösung wird vorsichtig in 9 l 20 %ige Salzsäure gegossen und mehrfach, insgesamt mit 9 l, Methyl-tert.-butylether extrahiert. Die vereinigten organischen Phasen werden mit 20 %iger Salzsäure gewaschen und über Magnesiumsulfat wasserfrei getrocknet. Abfiltrieren, eingengen. Das zurückbleibende Öl wird im Hochvakuum fraktioniert.

Ausbeute: 349,3 g (49 % der Theorie); Kp_{0.1} 130-137°C.

Analog bzw. gemäß den allgemeinen Angaben zur Herstellung wurden die folgenden Verbindungen der Formel (XXVII) hergestellt:

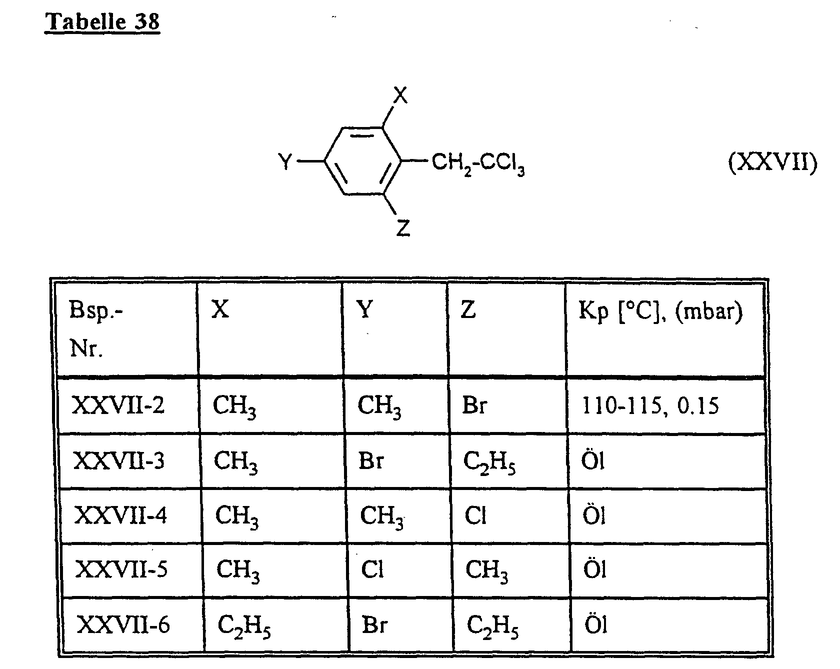

### Beispiel (VI-1)

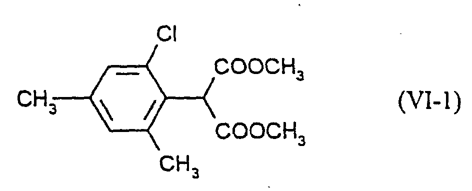

Es wurden 7,1 g NaH (80%ig) in 278 ml Dimethylcarbonat vorgelegt und auf 80 bis 90°C erwärmt. Anschließend wurden 39 g 2-Chlor-4,6-dimethylphenylessigsäuremethylester zugetropft und 20 h zum Rückfluß erhitzt. Es wurden weitere 3,4 g NaH (80%ig) zugegeben und nochmals 8 h zum Rückfluß erwärmt. Die Mischung wurde abgekühlt, noch vorhandenes NaH mit wenig Methanol zerstört und dann auf Eis gegossen. Nach dem Ansäuern mit halbkonzentrierter HCl wurde die organische Phase abgetrennt und die wäßrige Phase mehrfach mit Dichlormethan extrahiert. Die vereinigte organische Phase wurde getrocknet und eingeengt. Ausbeute: 35,1 g eines Feststoffs mit einem Schmelzpunkt von 67 bis 70°C.
¹H-NMR (CDCl₃): δ 7,12 (s,1H), 6,94 (s,1H), 5,36 (s,1H), 3,78 (s,6H), 2.31 (s,3H), 2,28 ppm (s,3H).

### Beispiel (XXXV-1)

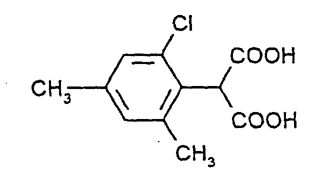

Es wurden 10 g 2-Chlor-4,6-dimethylphenylmalonsäuredimethylester gemaß Beispiel (VI-1) vorgelegt und nacheinander mit 20 ml Methanol und 6,8 g KOH gelöst und 9,1 ml Wasser versetzt. Nach einer Stunde wurde mit weiteren 20 ml Lösungsmittel (MeOH/Wasser 1:1) verdünnt. Die Mischung wurde 10 h zum Rückfluß erhitzt, dann abgekühlt und eingeengt. Der verbleibende Rückstand wurde in wenig Wasser aufgenommen und einmal mit Toluol gewaschen Anschließend wurde die wäßrige Phase weiter mit Wasser verdünnt, der gleiche Anteil Ether zugesetzt und auf ca. -10°C abgekühlt. Es wurde mit konzentrierter HCI angesäuert (pH 1), die organische Phase abgetrennt und noch 1 bis 2 mal nachextrahiert. Die vereinigten organischen Phasen wurden getrocknet und eingeengt. Der Rückstand wurde aus Toluol kristallisiert und ergab 7,6 g 2-Chlor-4,6-dimethylphenylmalonsäure mit einem Schmelzpunkt von 174 bis 176°C (Zersetzung).
¹H-NMR (CDCl₃) δ: 7,10 (s,1H), 6,95 (s,1H), 5,00 (s,1H), 2,36 (s,3H), 2,30 ppm (s,3H).

### Beispiel (V-1)

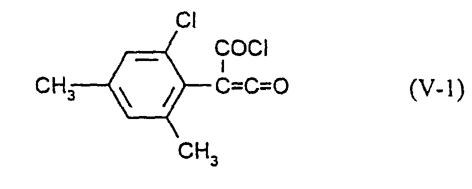

Es wurden 7,6 g (2-Chlor-4,6-dimethylphenylmalonsäure gemäß Beispiel (XXXV-1) in 22 ml Toluol suspendiert und mit 19,5 ml Thionylchlorid tropfenweise versetzt. Die Mischung wurde 9,5 h auf 95°C erhitzt, abgekühlt und durch Überleiten von Argon von den flüchtigen Bestandteilen befreit. Die Reste an Thionylchlorid und das Lösungsmittel wurden bei 45°C im Hochvakuum abdestilliert. Man erhielt 6,6 g 2-Chlor-4,6-dimethylphenylchlorcarbonylketen als ein Öl, das leicht verunreinigt war mit 2-Chlor-4,6-dimethylphenylessigsäurechlorid.
¹H-NMR (CDCl₃) δ: 7,16 (s,1H), 7,02 (s,1H), 2,33 (s,3H), 2,30 ppm (s,3H).

### Beispiel (VI-2)

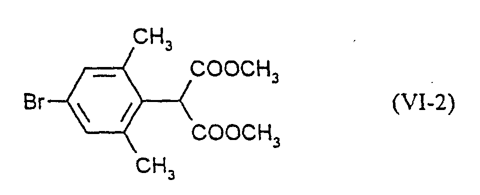

Es wurden in analoger Weise 70 g 4-Brom-2,6-dimethylphenylessigsäuremethylester mit 26,8 g NaH und 7,39 ml Dimethylcarbonat umgesetzt. Nach der Aufarbeitung wurden 95,4 g Rohprodukt (86,5%ig) erhalten.
¹H-NMR (CDCl₃) δ: 7,22 (s,2H), 5,00 (s,4H), 3,75 (s,6H), 2,33 ppm (s,6H).

### Beispiel (XXXV-2)

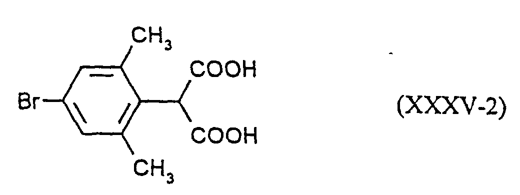

Es wurden in analoger Weise 85 g 4-Brom-2,6-dimethylphenylmalonsäuredimethylester gemäß Beispiel (VI-2) in 158 ml Methanol mit 49,6 g KOH in 66 ml H₂O eingesetzt. Nach der Aufarbeitung wurden 59,7 g der Malonsäure erhalten. Schmelzpunkt 164 bis 167°C (Zersetzung).
¹H-NMR (CDCl₃) δ: 7,20 (s,2H), 7,00 - 6,00 (OH), 4,83 (s,1H), 2,30 ppm (s,6H).

### Beispiel (V-2)

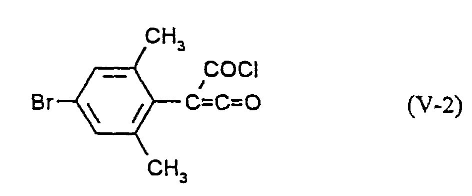

Es wurden in analoger Weise 59 g 4-Brom-2,6-dimethylphenylmalonsäure in 143 ml Toluol mit 128 ml Thionylchlorid umgesetzt und 49,5 g als Rohprodukt isoliert.
¹H-NMR (CDCl₃) δ: 7,31 (s,2H), 2,33 ppm (s,6H).

### Beispiel (VI-3)

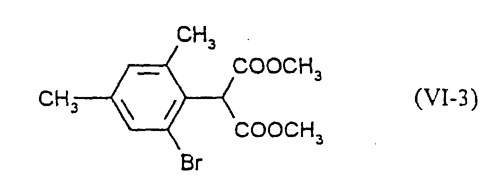

Es wurden in analoger Weise 23 g 2-Brom-4,6-dimethylphenylessigsäuremethylester mit 9,5 g NaH (80%ig) und 242 ml Dimethylcarbonat umgesetzt. Nach der Aufarbeitung wurden 31,2 g Rohprodukt (82%ig) erhalten.
¹H-NMR (CDCl₃) δ: 7,31 (s,1H), 6,98 (s,1H), 5,45 (s,1H), 3,88 (s,6H), 2,32 (s,3H), 2,28 ppm (s,3H).

### Beispiel (XXXV-3)

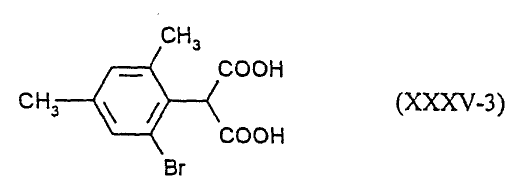

Es wurden in analoger Weise 27 g 2-Brom-4,6-dimethylphenylmalonsäuredimethylester gemäß Beispiel (VI-3) in 50 ml Methanol mit 15,7 g KOH in 21 ml Wasser umgesetzt. Nach der Aufarbeitung wurden 17,4 g Malonsäure erhalten. Schmelzpunkt 167 bis 169°C (Zersetzung).
¹H-NMR (CDCl₃) δ: 8,20 - 7,00 (OH), 7,26 (s,1H), 6,98 (s,1H), 5,07 (s,1H), 2,35 (s,3H), 2,28 ppm (s,3H).

### Beispiel (V-3)

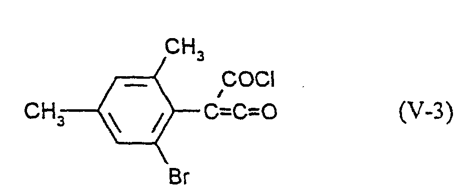

Es wurden in analoger Weise 17 g 2-Brom-4,6-dimethylphenylmalonsäure gemäß Beispiel (XXXV-3) in 41 ml Toluol mit 36,8 ml Thionylchlorid umgesetzt und 15,1 g als Rohprodukt isoliert.
IR : 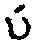 = 2130 (Keten)
¹H-NMR (CDCl₃) δ: 7,28 (s,1H), 7,00 (s,1H), 2,35 (s,3H), 2,29 ppm (s,3H).

### Beispiel (V-4)

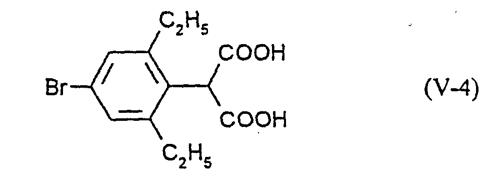

¹H-NMR (400 MHZ), CDCl₃): δ = 1.20, 2.64, 7.32

### Beispiel (XXXV-4)

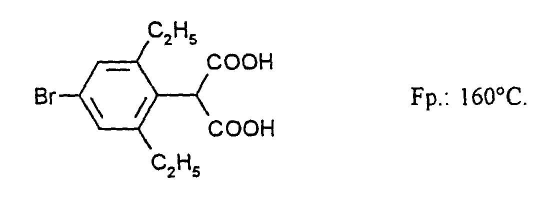

### Anwendungsbeispiele

### Beispiel A

| Phaedon-Larven-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach jeweils 3 Tagen wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen (I-2-a-2), (I-2-b-2), (I-2-a-1), (I-2-b-1), (I-2-b-4), (I-1-a-2), (I-1-a-1), (I-1-b-2) und (I-1-b-4) bei einer beispielhaften Wirkstoffkonzentration von 0,1 % eine Abtötung von 100 % nach 7 Tagen.

### Beispiel B

| Plutella-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet; daß keine Raupen abgetötet wurden.

In diesem Test bewirkten z.B. die Verbindungen gemäß den Herstellungsbeispielen (I-2-b-2), (I-2-b-1), (I-1-b-2), (I-1-b-4), (I-1-c-2 (I-1-a-5) und (I-1-a-6) bei einer beispielhaften Wirkstoffkonzentration von 0,1 % eine Abtötung von 100 % nach 7 Tagen.

### Beispiel C

| Nephotettix-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet; daß keine Zikaden abgetötet wurden.

In diesem Test bewirkten z.B. die Verbindungen gemäß den Herstellungsbeispielen (I-2-a-2), (I-2-a-1), (I-2-b-1), (I-1-a-2), (I-1-a-1), (I-1-b-2), (I-1-b-3), (I-1-b-4), (1-1-c-2), (I-1-a-5) und (I-1-a-6) bei einer beispielhaften Wirkstoffkonzentration von 0,1 % eine Abtötung von 100 % nach 6 Tagen.

### Beispiel D

| Myzus-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen (I-2-a-1), (I-2-a-2), (I-2-b-2), (I-1-b-3), (I-1-c-2) und (I-1-a-6) bei einer beispielhaften Wirkstoffkonzentration von 0,1 % eine Abtötung von 100 % nach 6 Tagen.

### Beispiel E

| Tetranychus-Test (OP-resistent/Tauchbehandlung) | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen gemäß den Herstellungsbeispielen (I-2-a-2), (I-2-b-2), (I-2-b-3), (I-2-a-1) und (I-2-b-4) bei einer beispielhaften Wirkstoffkonzentration von 0,01 % eine Wirkung von 100 % nach 13 Tagen.

### Beispiel F

| Pre-emergence-Test | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden im normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

| **Pre emergence Test / Gewächshaus** | | | | | | |
|---|---|---|---|---|---|---|
| Bsp.-Nr. | g/ha | Beta vulgaris | Alopecurus myosuroides | Avena fatua | Setaria viridis | Sinapis arvensis 1. |
| I-1-a-2 | 250 | 0 | 100 | 60 | 100 | 90 |
| I-1-b-2 | 250 | 0 | 100 | 100 | 100 | - |
| I-1-b-3 | 250 | 0 | - | 80 | 100 | 95 |
| I-1-b-4 | 250 | 0 | - | 100 | 100 | 95 |
| I-1-c-2 | 250 | 0 | 95 | 80 | 100 | 100 |
| I-1-a-5 | 250 | - | 100 | 100 | 100 | 100 |
| I-1-a-6 | 250 | - | 95 | 95 | 100 | 100 |

## Patentansprüche

1. Verbindungen der Formel (I) 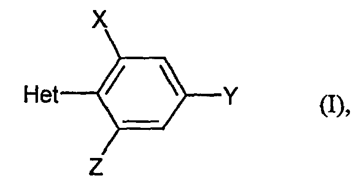 in welcher
X für Methyl, Ethyl, n-Propyl oder iso-Propyl steht,
Y für Fluor, Chlor, Brom, Methyl, Ethyl, n-Propyl oder iso-Propyl steht,
Z für Fluor, Chlor, Brom, Methyl, Ethyl, n-Propyl oder iso-Propyl steht,
wobei immer einer der Reste Y und Z für Halogen und der andere für Alkyl steht,
Het für eine der Gruppen 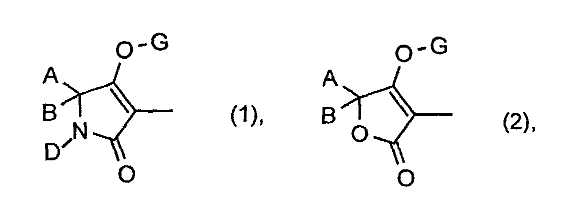 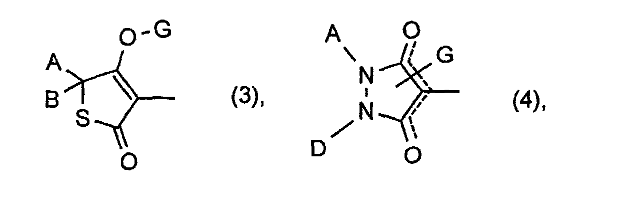 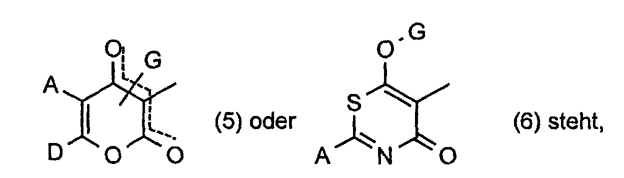
A für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄-alkyl, Poly-C₁-C₄-alkoxy-C₁-C₄-alkyl oder C₁-C₆-Alkylthio-C₁-C₄-alkyl, oder fiir gegebenenfalls durch Fluor, Chlor, Methyl oder Methoxy substituiertes C₃-C₆-Cycloalkyl, in welchem gegebenenfalls eine oder zwei Methylengruppen durch Sauerstoff und/oder Schwefel ersetzt sind oder fiir jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Thienyl, Pyridyl oder Benzyl steht,
B für Wasserstoff, C₁-C₈-Alkyl oder C₁-C₄-Alkoxy-C₁-C₂-alkyl steht oder
A, B und das Kohlenstoffatom an das sie gebunden sind, fiir C₃-C₈-Cycloalkyl oder C₅-C₈-Cycloalkenyl stehen, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
A, B und das Kohlenstoffatom, an das sie gebunden sind, fiir C₅-C₆-Cycloalkyl stehen, welches durch eine gegebenenfalls ein Sauerstoff- oder Schwefelatom enthaltende Alkylendiyl- oder durch eine Alkylendioxyl-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind, fiir C₃-C₆-Cycloalkyl oder C₅-C₆-Cycloalkenyl stehen, in dem zwei Substituenten gemeinsam mit den Kohlenstoffatomen, an die sie gebunden sind, für C₃-C₄-Alkandiyl, C₃-C₄-Alkendiyl oder Butadiendiyl stehen, worin jeweils gegebenenfalls eine Methylengruppe durch Sauerstoff oder Schwefel ersetzt ist,
D für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl steht,
oder
A und D gemeinsam fiir eine C₃-C₅-Alkandiyl- oder C₃-C₅-Alkendiylgruppe stehen, worin jeweils gegebenenfalls eine Methylengruppe durch Sauerstoff oder Schwefel ersetzt ist und welche gegebenenfalls durch Fluor, Chlor, Hydroxy, Mercapto oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy substituiert sind oder
worin gegebenenfalls eine der folgenden Gruppen 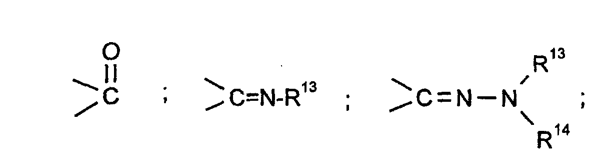 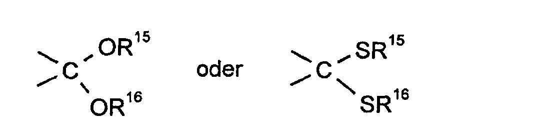 enthalten ist,
oder A und D im Fall der Verbindungen der Formel (I-1) gemeinsam mit den Atomen, an die sie gebunden sind, für eine der folgenden Gruppen 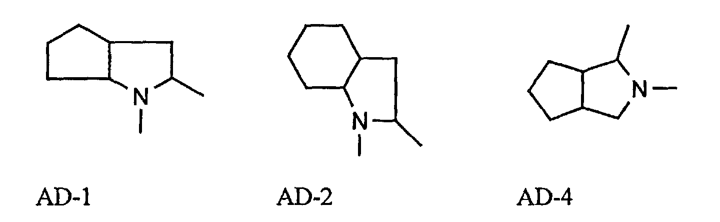 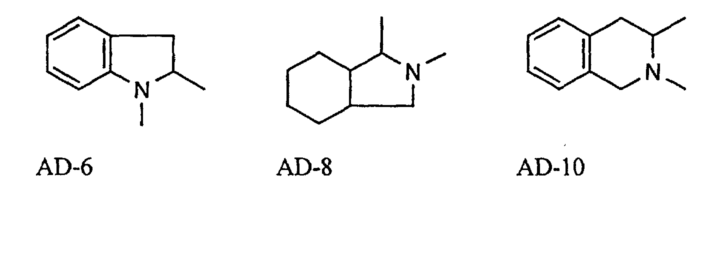 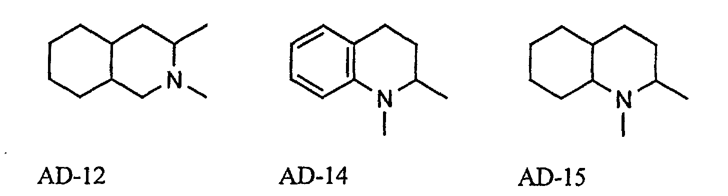 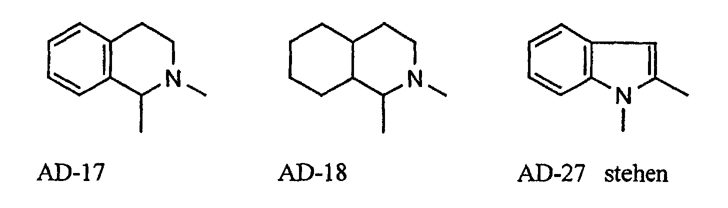
G im Fall, dass Het für einen der Reste (1), (2), (3), (5) oder (6) steht, für Wasserstoff (a) oder, im Fall, dass Het für einen der Reste (1), (2), (3), (4), (5) oder (6) steht, für eine der Gruppen 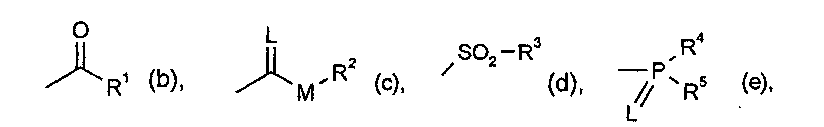 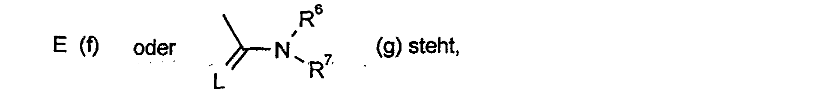 in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei Methylengruppen durch Sauerstoff und/ oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
fiir gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
fiir jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄alkoxy-C₂-C₆-alkyl, für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für jeweils gegebenenfalls durch Fluor oder Chlor substituiertes Methyl, Ethyl, Propyl, Isopropyl oder jeweils gegebenenfalls durch Fluor, Chlor, Brom, Methyl, Ethyl, Propyl, iso-Propyl, tert.-Butyl, Methoxy, Ethoxy, Isopropoxy, tert.-Butoxy, Trifluormethyl, Trifluormethoxy, Cyano oder Nitro substituiertes Phenyl oder Benzyl steht,
R⁴ und R⁵ unabhängig voneinander fiir jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebenenfalls eine Methylengruppe durch Sauerstoff oder Schwefel ersetzt ist,
R¹³ für Wasserstoff oder für jeweils gegebenenfalls durch Fluor oder Chlor substituiertes C₁-C₄-Alkyl oder C₁-C₄-Alkoxy, für C₃-C₆-Cycloalkyl oder für jeweils gegebenenfalls durch Fluor, Chlor, Brom, Methyl, Ethyl, iso-Propyl, tert.-Butyl, Methoxy, Ethoxy, iso-Propoxy, tert.-Butoxy, Trifluormethyl, Trifluormethoxy, Nitro oder Cyano substituiertes Phenyl, Phenyl-C₁-C₂-alkyl oder Benzyloxy steht,
R¹⁴ für Wasserstoff oder C₁-C₄-Alkyl steht oder
R¹³ und R¹⁴ gemeinsam für C₄-C₆-Alkandiyl stehen,
R¹⁵ und R¹⁶ gleich oder verschieden sind und für Methyl oder Ethyl stehen oder
R¹⁵ und R¹⁶ zusammen für einen C₂-C₃-Alkandiylrest stehen, der gegebenenfalls durch Methyl, Ethyl, n-Propyl, iso-Propyl, n-Butyl, iso-Butyl, sek.-Butyl, tert.-Butyl oder durch gegebenenfalls durch Fluor, Chlor, Methoxy, Trifluormethyl, Trifluormethoxy, Nitro oder Cyano substituiertes Phenyl substituiert ist.

2. Verbindungen der Formel (I) gemäß Anspruch 1, in welcher die Substituenten die in der folgenden Tabelle aufgeführten Definitionen besitzen:

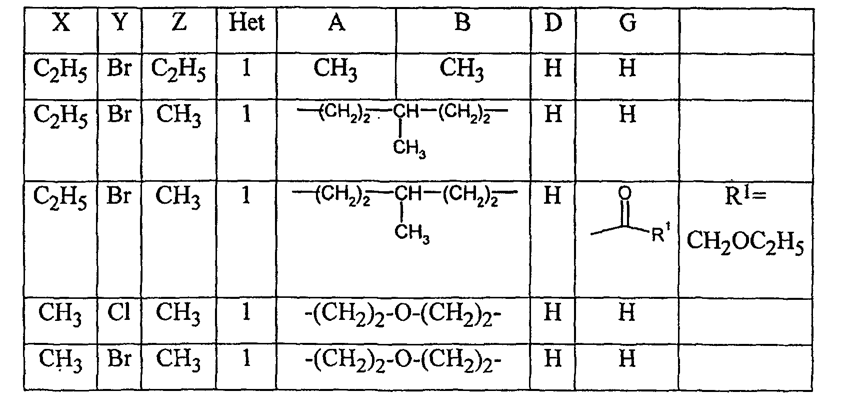

3. Verfahren zur Herstellung von Verbindungen der Formel (I) gemäß Anspruch 1, **dadurch gekennzeichnet, dass** man
(A) Verbindungen der Formel (I-1-a) 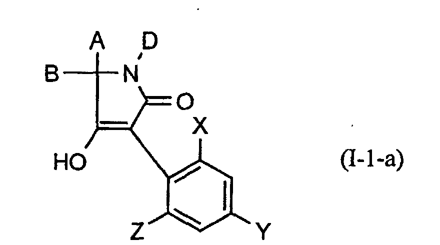 in welcher
A, B, D, X, Y und Z die in Anspruch 1 angegebenen Bedeutungen haben,
erhält, wenn man
Verbindungen der Formel (II) 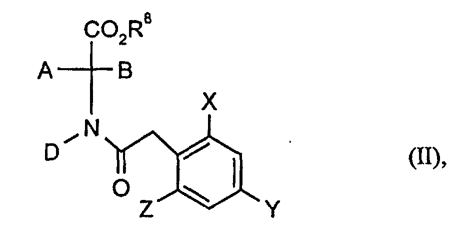 in welcher
A, B, D, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 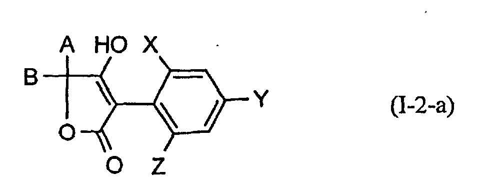 in welcher
A, B, X, Y und Z die oben angegebenen Bedeutungen haben,
erhält, wenn man
Verbindungen der Formel (III) 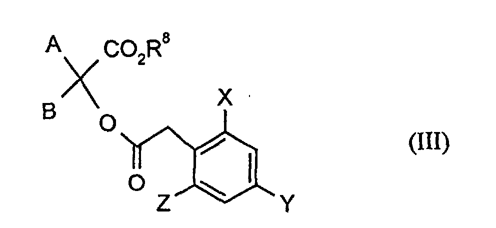 in welcher
A, B, X, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-3-a) 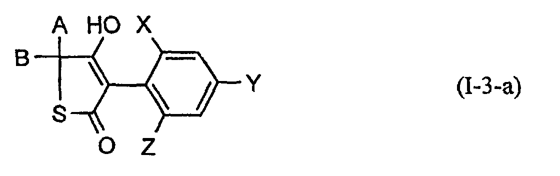 in welcher
A, B, X, Y und Z die oben angegebenen Bedeutungen haben,
erhält, wenn man
Verbindungen der Formel (IV) 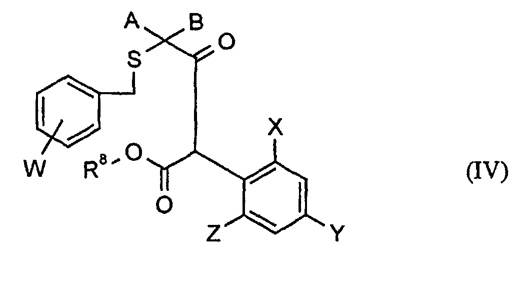 in welcher
A, B, X, Y, Z und R⁸ die oben angegebenen Bedeutungen haben und
W für Wasserstoff, Halogen, Alkyl oder Alkoxy steht,
gegebenenfalls in Gegenwart eines Verdünnungsmittels und in Gegenwart einer Säure intramolekular cyclisiert,
(E) die Verbindungen der Formel (I-5-a) 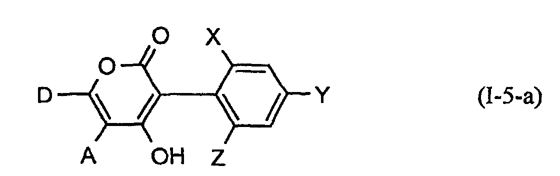 in welcher
A, D, X, Y und Z die oben angegebenen Bedeutungen haben,
erhält, wenn man
Verbindungen der Formel (VIII) 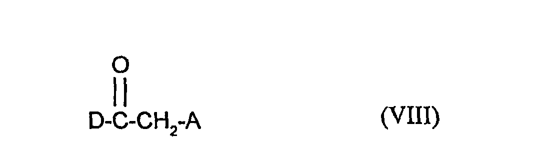 in welcher
A und D die oben angegebenen Bedeutungen haben,
mit Verbindungen der Formel (V) 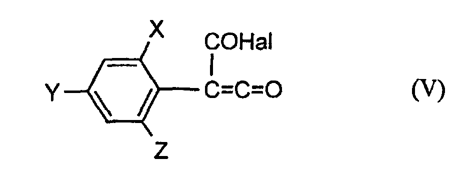 in welcher
X, Y und Z die oben angegebenen Bedeutungen haben und
Hal für Halogen steht,
gegebenenfalls in Gegenwart eines Verdünnungsmittels und geg nenfalls in Gegenwart eines Säureakzeptors umsetzt,
(F) die Verbindungen der Formel (I-6-a) 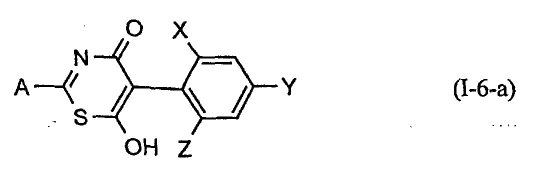 in welcher
A, X, Y und Z die oben angegebenen Bedeutungen haben,
erhält, wenn man Verbindungen der Formel (IX) 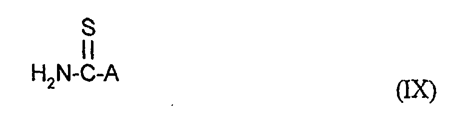 in welcher
A die oben angegebene Bedeutung hat,
mit Verbindungen der Formel (V) 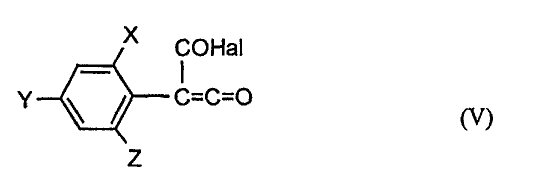 in welcher
Hal, X, Y und Z die oben angegebenen Bedeutungen haben,
gegebenenfalls in Gegenwart eines Verdünnungsmittels und gegebenenfalls in Gegenwart eines Säureakzeptors umsetzt und gegebenenfalls anschließend die so erhaltenen Verbindungen der oben gezeigten Formeln (I-1-a) bis (I-3-a), (I-5-a) und (I-6-a) oder Verbindungen der Formel (I-4-a) 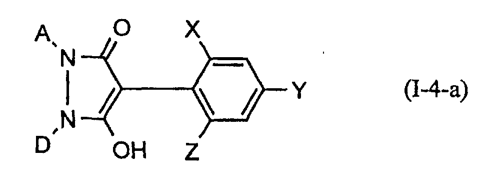 in welcher
A, D, X, Y und Z die oben angegebene Bedeutung haben, jeweils
(G)
α) mit Säurehalogeniden der Formel (X) 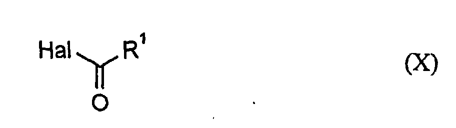 in welcher
R¹ die oben angegebene Bedeutung hat und
Hal für Halogen steht
oder
β) mit Carbonsäureanhydriden der Formel (XI)
R¹-CO-O-CO-R¹ (XI)
in welcher
R¹ die oben angegebene Bedeutung hat,
gegebenenfalls in Gegenwart eines Verdünnungsmittels und gegebenenfalls in Gegenwart eines Säurebindemittels umsetzt,
(H) mit Chlorameisensäureestern oder Chlorameisensäurethioestern der Formel (XII)
R²-M-CO-Cl (XII)
in welcher
R² und M die oben angegebenen Bedeutungen haben,
gegebenenfalls in Gegenwart eines Verdünnungsmittels und gegebenenfalls in Gegenwart eines Säurebindemittels umsetzt,
oder
(I)
α) mit Chlormonothioameisensäureestern oder Chlordithioameisensäureestern der Formel (XIII) 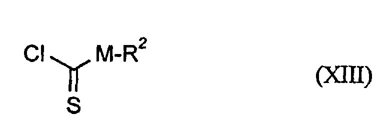 in welcher
M und R² die oben angegebenen Bedeutungen haben,
gegebenenfalls in Gegenwart eines Verdünnungsmittels und gegebenenfalls in Gegenwart eines Säurebindemittels umsetzt
oder
β) mit Schwefelkohlenstoff und anschließend mit Verbindungen der Formel (XIV)
R²-Hal (VIX)
in welcher
R² die oben angegebene Bedeutung hat und
Hal für Chlor, Brom oder Iod steht,
gegebenenfalls in Gegenwart eines Verdünnungsmittels und gegebenenfalls in Gegenwart einer Base umsetzt,
oder
(J) mit Sulfonsäurechloriden der Formel (XV)
R³-SO₂-Cl (XV)
in welcher
R³ die oben angegebene Bedeutung hat,
gegebenenfalls in Gegenwart eines Verdünnungsmittels und gegebenenfalls in Gegenwart eines Säurebindemittels umsetzt,
oder
(K) mit Phosphorverbindungen der Formel (XVI) 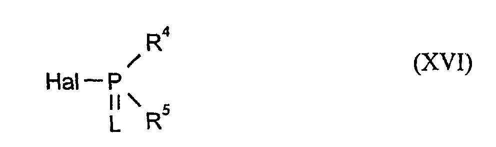 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
oder
(L) mit Metallverbindungen oder Aminen der Formeln (XVII) oder (XVIII)
Me(OR¹⁰)ₜ (XVII) 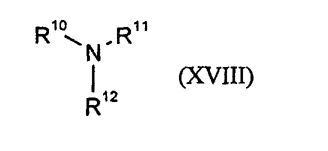
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt
oder
(M)
α) mit Isocyanaten oder Isothiocyanaten der Formel (XIX)
R⁶-N=C=L (XIX)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XX) 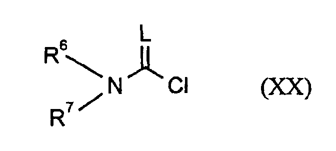 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

4. Verbindungen der Formel (II) 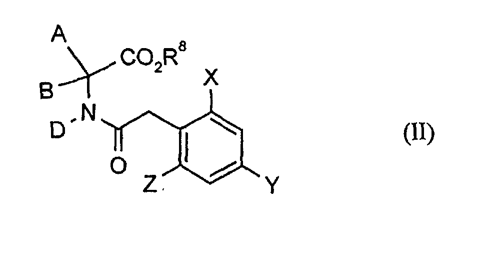 in welcher
A, B, D, X, Y und Z die in Anspruch 1 angegebene Bedeutungen haben und
R⁸ für Alkyl steht.

5. Verbindungen der Formel (XXIII) 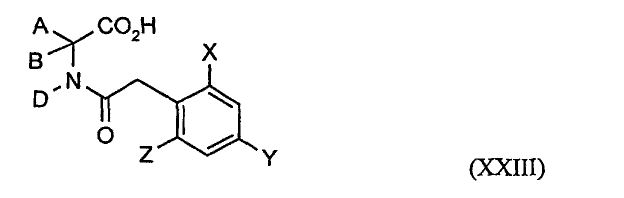 in welcher
A, B, D, X, Y und Z die in Anspruch 1 angegebenen Bedeutungen haben.

6. Verbindungen der Formel (XXII) 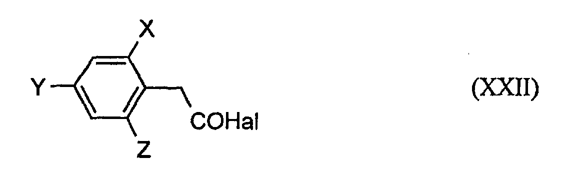 in welcher
X, Y und Z die in Anspruch 1 angegebenen Bedeutungen haben und
Hal für Chlor oder Brom steht.

7. Verbindungen der Formel (XXV) 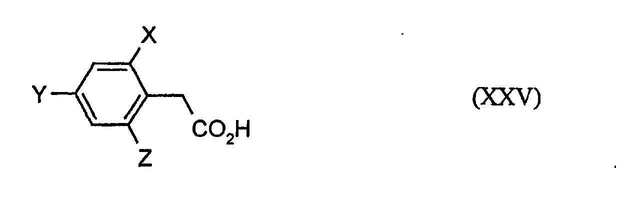 in welcher
X, Y und Z die in Anspruch 1 angegebene Bedeutung haben.

8. Verbindungen der Formel (XXVI) 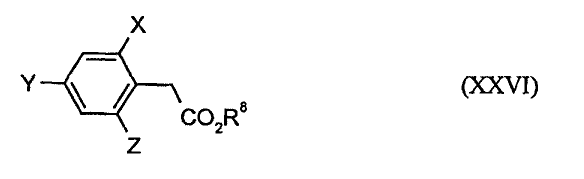 in welcher
X, Y und Z die oben angegebene Bedeutung haben, und
R⁸ für Alkyl steht.

9. Verbindungen der Formel (XXVII) 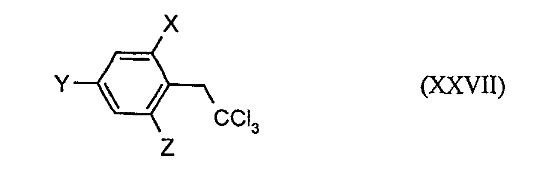 in welcher
X, Y und Z die in Anspruch 1 angegebene Bedeutung haben.

10. Verbindungen der Formel (XXXI) 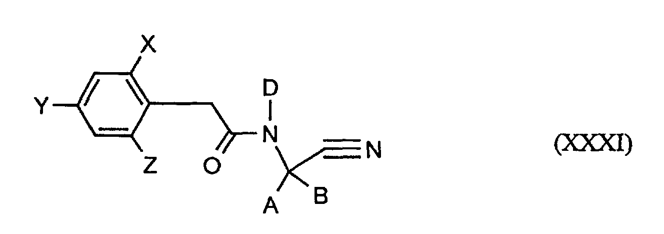 in welcher
A, B, D, X, Y und Z die in Anspruch 1 angegebenen Bedeutungen haben.

11. Verbindungen der Formel (III) 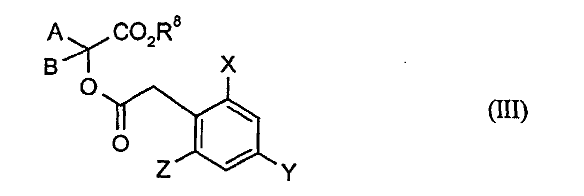 in welcher
A, B, X, Y und Z die oben angegebenen Bedeutungen haben, und
R⁸ für Alkyl steht.

12. Verbindungen der Formel (IV) 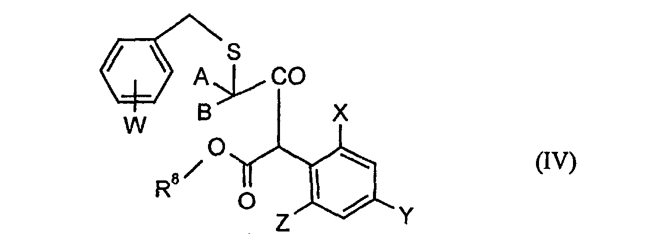 in welcher
A, B, X, Y und Z die in Anspruch 1 angegebenen Bedeutungen haben,
W für Wasserstoff, Halogen, Alkyl oder Alkoxy steht und
R⁸ für Alkyl steht.

13. Verbindungen der Formel (V) 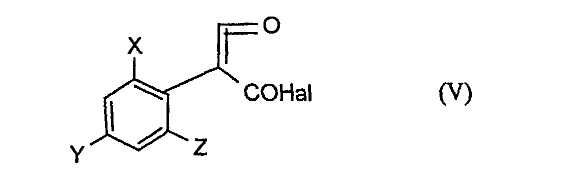 in welcher
X, Y und Z die in Anspruch 1 angegebenen Bedeutungen haben und
Hal für Chlor oder Brom steht.

14. Verbindungen der Formel (XXXV) 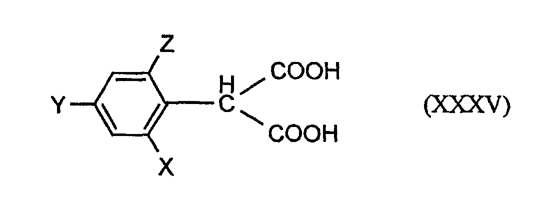 in welcher
X, Y und Z die in Anspruch 1 angegebenen Bedeutungen haben.

15. Verbindungen der Formel (VI) 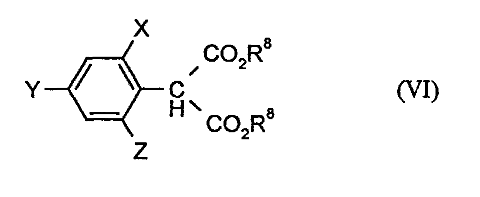 in welcher
X, Y und Z die in Anspruch 1 angegebenen Bedeutungen haben und
R⁸ für Alkyl steht.

16. Schädlingsbekämpfungsmittel und Unkrautbekämpfungsmittel, **gekennzeichnet durch** einen Gehalt an mindestens einer Verbindung der Formel (I) gemäß Anspruch 1.

17. Verwendung von Verbindungen der Formel (I) gemäß Anspruch 1 zur Bekämpfung von Schädlingen und Unkräutern.

18. Verfahren zur Bekämpfung von Schädlingen und Unkräutern, **dadurch gekennzeichnet, dass** man Verbindungen der Formel (I) gemäß Anspruch 1 auf Schädlinge und/oder ihren Lebensraum einwirken lässt oder auf Unkräuter und/oder ihren Lebensraum einwirken lässt.

19. Verfahren zur Herstellung von Schädlingsbekämpfungsmitteln und Unkrautbekämpfungsmitteln, **dadurch gekennzeichnet, dass** man Verbindungen der Formel (I) gemäß Anspruch 1 mit Streckmitteln und/oder oberflächenaktiven Mitteln vermischt.

20. Verwendung von Verbindungen der Formel (I) gemäß Anspruch 1 zur Herstellung von Schädlingsbekämpfungsmitteln und Unkrautbekämpfungsmitteln.

## Claims

1. Compounds of the formula (I) 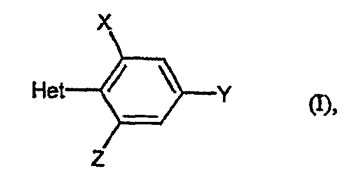 in which
X represents methyl, ethyl, n-propyl or iso-propyl,
Y represents fluorine, chlorine, bromine, methyl, ethyl, n-propyl or isopropyl,
Z represents fluorine, chlorine, bromine, methyl, ethyl, n-propyl or isopropyl,
in this case one of the radicals Y and Z always represents halogen and the other alkyl,
Het represents one of the groups 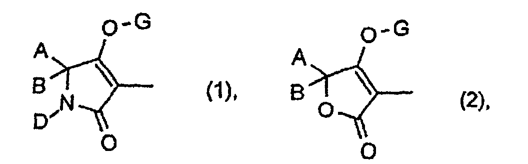 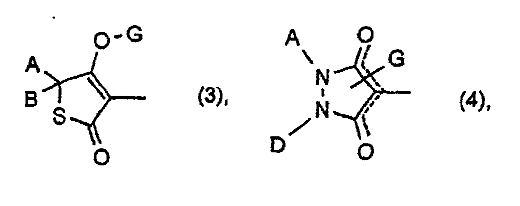 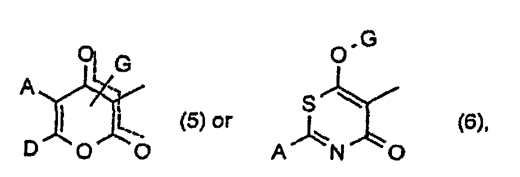
A represents hydrogen, or C₁-C₈-alkyl, C₂-C₄-alkenyl, C₁-C₆-alkoxy-C₁-C₄-alkyl, poly-C₁-C₄-alkoxy-C₁-C₄-alkyl or C₁-C₆-alkylthio-C₁-C₄-alkyl, each of which is optionally substituted by fluorine or chlorine, or C₃-C₆-cycloalkyl which is optionally substituted by fluorine, chlorine, methyl or methoxy and in which one or two methylene groups are optionally replaced by oxygen and/or sulphur, or phenyl, furanyl, thienyl, pyridyl or benzyl, each of which is optionally substituted by fluorine, chlorine, bromine, methyl, ethyl, n-propyl, iso-propyl, methoxy, ethoxy, trifluoromethyl, trifluoromethoxy, cyano or nitro,
B represents hydrogen, C₁-C₈-alkyl or C₁-C₄-alkoxy-C₁-C₂-alkyl or
A, B and the carbon atom to which they are bonded represent C₃-C₈-cycloalkyl or C₅-C₈-cycloalkenyl, in which in each case a methylene group is optionally replaced by oxygen or sulphur and each of which is optionally substituted by methyl, ethyl, n-propyl, isopropyl, butyl, iso-butyl, sec-butyl, tert-butyl, cyclohexyl, trifluoromethyl, methoxy, ethoxy, n-propoxy, iso-propoxy, butoxy, iso-butoxy, sec-butoxy, tert-butoxy, methylthio, ethylthio, fluorine, chlorine or phenyl, or
A, B and the carbon atom to which they are bonded very particularly preferably represent C₅-C₆-cycloalkyl which is substituted by an alkylenediyl group optionally containing an oxygen or sulphur atom or by an alkylenedioxyl group which, with the carbon atom to which it is bonded, forms a further five- or six-membered ring, or
A, B and the carbon atom to which they are bonded represent C₃-C₆-cycloalkyl or C₅-C₆-cycloalkenyl, in which two substituents, together with the carbon atoms to which they are bonded, represent C₃-C₄-alkanediyl, C₃-C₄-alkenediyl or butadienediyl, in which in each case a methylene group is optionally replaced by oxygen or sulphur,
D represents hydrogen, or C₁-C₈-alkyl, C₃-C₄-alkenyl, C₃-C₄-alkinyl, C₁-C₆-alkoxy-C₂-C₄-alkyl, poly-C₁-C₄-alkoxy-C₂-C₄-alkyl, C₁-C₄-alkylthio-C₂-C₄-alkyl or C₃-C₆-cycloalkyl, each of which is optionally substituted by fluorine or chlorine, and in which one or two non-directly adjacent methylene groups are optionally replaced by oxygen and/or sulphur, or phenyl, furanyl, pyridyl, thienyl or benzyl, each of which is optionally substituted by fluorine, chlorine, bromine, methyl, ethyl, n-propyl, iso-propyl, methoxy, ethoxy, trifluoromethyl, trifluoromethoxy, cyano or nitro,
or
A and D together represent a C₃-C₅-alkanediyl or C₃-C₅-alkenediyl group, in which in each case a methylene group is optionally replaced by oxygen or sulphur and each of which is optionally substituted by fluorine, chlorine, hydroxyl, mercapto or by C₁-C₆-alkyl, C₁-C₄-alkoxy, C₁-C₄-alkylthio, C₃-C₆-cycloalkyl, phenyl or benzyloxy, each of which is optionally substituted by fluorine or chlorine, or
which optionally contains one of the following groups 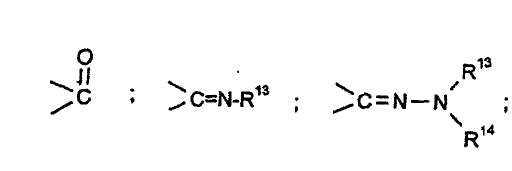 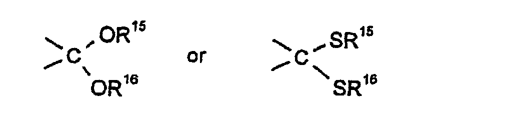 or A and D represent, in the case of the compounds of the formula (I-1), together with the atoms to which they are bonded, one of the following groups: 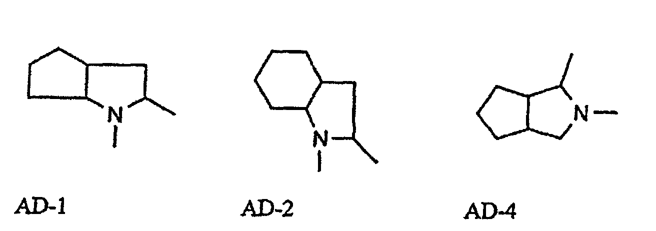 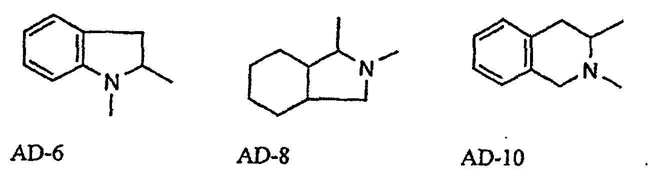 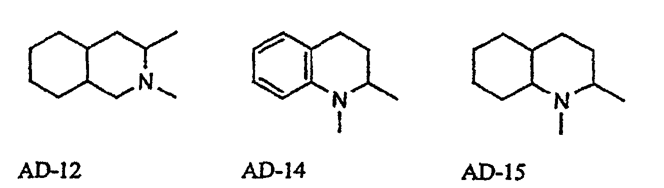 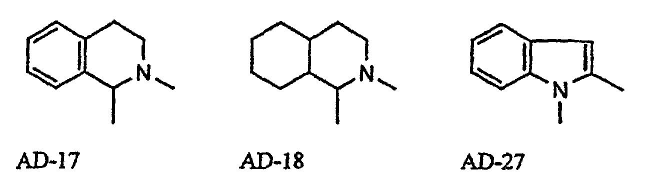
G represents, in the case in which Het represents one of the radicals (1), (2), (3), (5) or (6), hydrogen (a) or, in the case in which Het represents one of the radicals (1), (2), (3), (4), (5) or (6), one of the groups 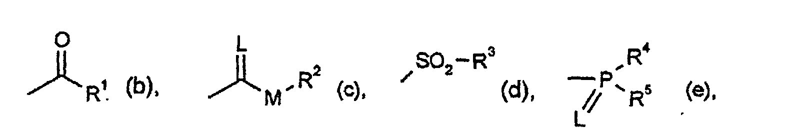 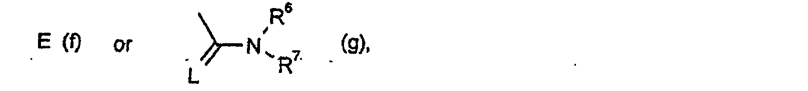 in which
E represents a metal ion equivalent or an ammonium ion,
L represents oxygen or sulphur and
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl, poly-C₁-C₄-alkoxy-C₁-C₄-alkyl, each of which is optionally substituted by fluorine or chlorine, or C₃-C₆-cycloalkyl which is optionally substituted by fluorine, chlorine, methyl, ethyl, n-propyl, i-propyl, n-butyl, i-butyl, tert-butyl, methoxy, ethoxy, n-propoxy or iso-propoxy and in which one or two methylene groups are optionally replaced by oxygen and/or sulphur,
phenyl which is optionally substituted by fluorine, chlorine, bromine, cyano, nitro, methyl, ethyl, n-propyl, i-propyl, methoxy, ethoxy, trifluoromethyl, trifluoromethoxy, methylthio, ethylthio, methylsulphonyl or ethylsulphonyl,
benzyl which is optionally substituted by fluorine, chlorine, bromine, methyl, ethyl, n-propyl, i-propyl, methoxy, ethoxy, trifluoromethyl or trifluoromethoxy,
furanyl, thienyl or pyridyl, each of which is optionally substituted by fluorine, chlorine, bromine, methyl or ethyl,
phenoxy-C₁-C₄-alkyl which is optionally substituted by fluorine, chlorine, methyl or ethyl, or
pyridyloxy-C₁-C₄-alkyl, pyrimidyloxy-C₁-C₄-alkyl or thiazolyloxy-C₁-C₄-alkyl, each of which is optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl, each of which is optionally substituted by fluorine or chlorine,
C₃-C₆-cycloalkyl which is optionally substituted by fluorine, chlorine, methyl, ethyl, n-propyl, iso-propyl or methoxy,
or phenyl or benzyl, each of which is optionally substituted by fluorine, chlorine, cyano, nitro, methyl, ethyl, n-propyl, i-propyl, methoxy, ethoxy, trifluoromethyl or trifluoromethoxy,
R³ represents methyl, ethyl, propyl, isopropyl, each of which is optionally substituted by fluorine or chlorine, or phenyl or benzyl, each of which is optionally substituted by fluorine, chlorine, bromine, methyl, ethyl, propyl, iso-propyl, tert-butyl, methoxy, ethoxy, isopropoxy, tert-butoxy, trifluoromethyl, trifluoromethoxy, cyano or nitro,
R⁴ and R⁵ independently of one another represent C₁-C₄-alkyl, C₁-C₄-alkoxy, C₁-C₄-alkylamino, di-(C₁-C₄-alkyl)amino or C₁-C₄-alkylthio, each of which is optionally substituted by fluorine or chlorine, or phenyl, phenoxy or phenylthio, each of which is optionally substituted by fluorine, chlorine, bromine, nitro, cyano, methyl, methoxy, trifluoromethyl or trifluoromethoxy,
R⁶ and R⁷ independently of one another represent hydrogen, or C₁-C₄-alkyl, C₃-C₆-cycloalkyl, C₁-C₄-alkoxy, C₃-C₄-alkenyl or C₁-C₄-alkoxy-C₂-C₄-alkyl, each of which is optionally substituted by fluorine or chlorine, or phenyl or benzyl, each of which is optionally substituted by fluorine, chlorine, bromine, methyl, methoxy or trifluoromethyl, or together represent a C₅-C₆-alkylene radical which is optionally substituted by methyl or ethyl and in which a methylene group is optionally replaced by oxygen or sulphur,
R¹³ represents hydrogen or C₁-C₄-alkyl or C₁-C₄-alkoxy, each of which is optionally substituted by fluorine or chlorine, C₃-C₆-cycloalkyl, or phenyl, phenyl-C₁-C₂-alkyl or benzyloxy, each of which is optionally substituted by fluorine, chlorine, bromine, methyl, ethyl, iso-propyl, tert-butyl, methoxy, ethoxy, iso-propoxy, tert-butoxy, trifluoromethyl, trifluoromethoxy, nitro or cyano,
R¹⁴ represents hydrogen or C₁-C₄-alkyl, or
R¹³ and R¹⁴ together represent C₄-C₆-alkanediyl,
R¹⁵ and R¹⁶ are identical or different and represent methyl or ethyl, or
R¹⁵ and R¹⁶ together represent a C₂-C₃-alkanediyl radical which is optionally substituted by methyl, ethyl, n-propyl, iso-propyl, n-butyl, iso-butyl, sec-butyl, tert-butyl or by phenyl which is optionally substituted by fluorine, chlorine, methoxy, trifluoromethyl, trifluoromethoxy, nitro or cyano.

2. Compounds of the formula (I) according to Claim 1, in which the substituents have the definitions listed in the following table:

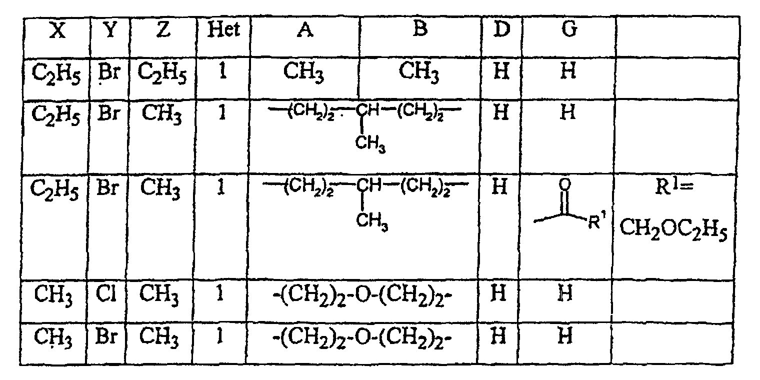

3. Process for the preparation of compounds of the formula (I) according to Claim 1, **characterized in that**
(A) compounds of the formula (I-1-a) 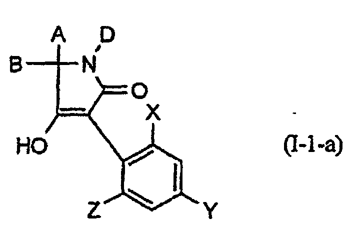 in which
A, B, D, X, Y and Z have the meanings given in Claim 1,
are obtained when
compounds of the formula (II) 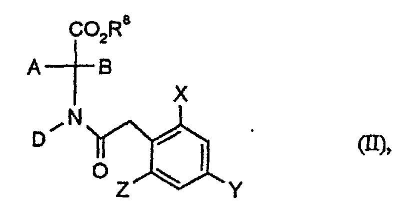 in which
A, B, D, X, Y and Z have the meanings given above,
and
R⁸ represents alkyl,
are intramolecularly condensed in the presence of a diluent and in the presence of a base,
(B) compounds of the formula (I-2-a) 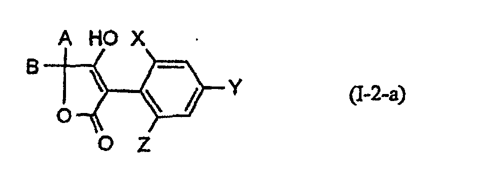 in which
A, B, X, Y and Z have the meanings given above,
are obtained when
compounds of the formula (III) 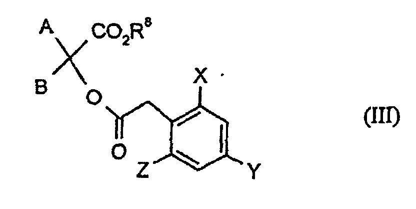 in which
A, B, X, Y, Z and R⁸ have the meanings given above,
are intramolecularly condensed in the presence of a diluent and in the presence of a base,
(C) compounds of the formula (I-3-a) 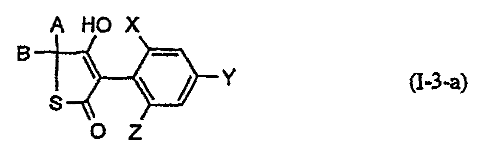 in which
A, B, X, Y and Z have the meanings given above,
are obtained when
compounds of the formula (IV) 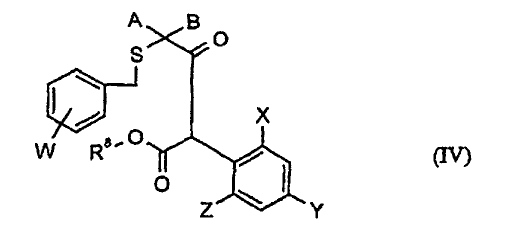 in which
A, B, X, Y, Z and R⁸ have the meanings given above and
W represents hydrogen, halogen, alkyl or alkoxy,
are cyclized, if appropriate in the presence of a diluent and in the presence of an acid,
(E) the compounds of the formula (I-5-a) 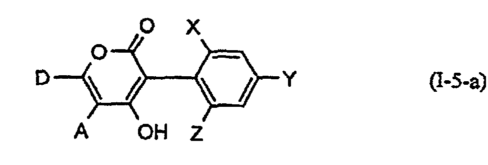 in which
A, D, X, Y and Z have the meanings given above,
are obtained when
compounds of the formula (VIII) 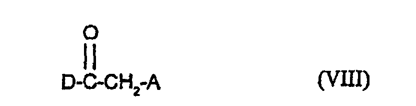 in which
A and D have the meanings given above,
are reacted with compounds of the formula (V) 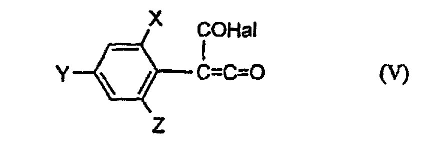 in which
X, Y and Z have the meanings given above and
Hal represents halogen,
if appropriate in the presence of a diluent and if appropriate in the presence of an acid acceptor,
(F) the compounds of the formula (I-6-a) 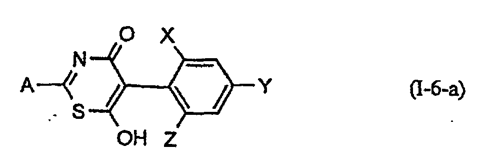 in which
A, X, Y and Z have the meanings given above,
are obtained when compounds of the formula (IX) 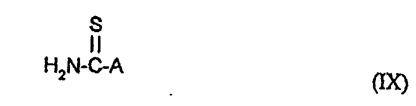 in which
A has the meaning given above,
are reacted with compounds of the formula (V) 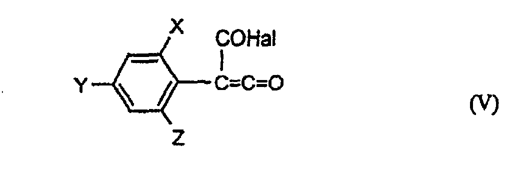 in which
Hal, X, Y and Z have the meanings given above,
if appropriate in the presence of a diluent and if appropriate in the presence of an acid acceptor, and optionally then the compounds thus obtained of the formulae (I-1-a) to (I-3-a), (I-5-a) and (I-6-a) shown above, compounds of the formula (I-4-a) 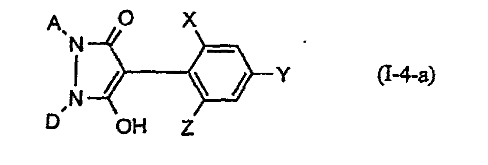 in which
A, D, X, Y and Z have the meanings given above, in each case are reacted
(G)
α) with acid halides of the formula (X) 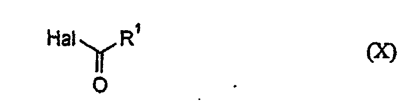 in which
R¹ has the meaning given above and
Hal represents halogen,
or
β) with carboxylic anhydrides of the formula (XI)
R¹-CO-O-CO-R¹ (XI)
in which
R¹ has the meaning given above,
if appropriate in the presence of a diluent and if appropriate in the presence of an acid-binding agent,
(H) with chloroformic acid esters or chloroformic acid thioesters of the formula (XII)
R²-M-CO-Cl (XII)
in which
R² and M have the meanings given above,
if appropriate in the presence of a diluent and if appropriate in the presence of an acid-binding agent,
or
(I)
α) with chloromonothioformic acid esters or chlorodithioformic acid esters of the formula (XIII) 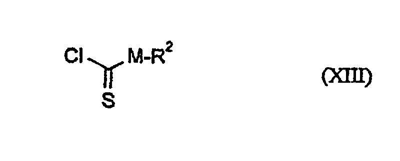 in which
M and R² have the meanings given above,
if appropriate in the presence of a diluent and if appropriate in the presence of an acid-binding agent
or
β) with carbon disulphide and then with compounds of the formula (XIV)
R²-Hal (XIV)
in which
R² has the meaning given above and
Hal represents chlorine, bromine or iodine,
if appropriate in the presence of a diluent and if appropriate in the presence of a base,
(J) with sulphonyl chlorides of the formula (XV)
R³-SO₂-Cl (XV)
in which
R³ has the meaning given above,
if appropriate in the presence of a diluent and if appropriate in the presence of an acid-binding agent,
or
(K) with phosphorus compounds of the formula (XVI) 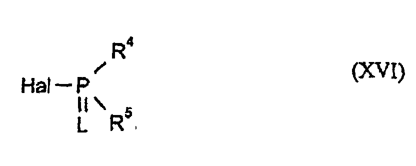 in which
L, R⁴ and R⁵ have the meanings given above and
Hal represents halogen,
if appropriate in the presence of a diluent and if appropriate in the presence of an acid-binding agent,
or
(L) with metal compounds or amines of the formula (XVII) or (XVIII)
Me(OR¹⁰)ₜ (XVII) 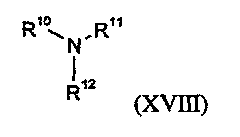
in which
Me represents a mono- or divalent metal,
t represents the number 1 or 2 and
R¹⁰, R¹¹, R¹² independently of one another represent hydrogen or alkyl,
if appropriate in the presence of a diluent,
or
(M)
α) with isocyanates or isothiocyanates of the formula (XIX)
R⁶-N=C=L (XIX)
in which
R⁶ and L have the meanings given above,
if appropriate in the presence of a diluent and if appropriate in the presence of a catalyst, or
β) with carbamoyl chlorides or thiocarbamoyl chlorides of the formula (XX) 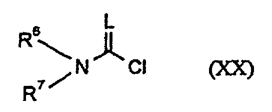 in which
L, R⁶ and R⁷ have the meanings given above,
if appropriate in the presence of a diluent and if appropriate in the presence of an acid-binding agent.

4. Compounds of the formula (II) 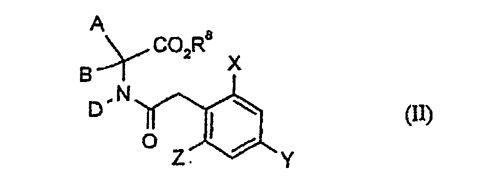 in which
A, B, D, X, Y and Z have the meanings given in Claim 1 and
R⁸ represents alkyl.

5. Compounds of the formula (XXIII) 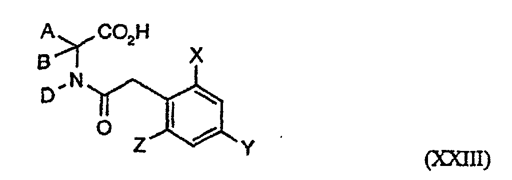 in which
A, B, D, X, Y and Z have the meanings given in Claim 1.

6. Compounds of the formula (XXII) 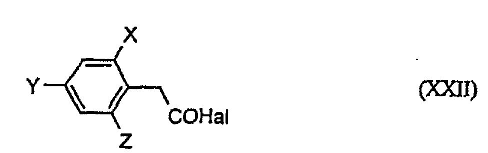 in which
X, Y and Z have the meanings given in Claim 1 and
Hal represents chlorine or bromine.

7. Compounds of the formula (XXV) 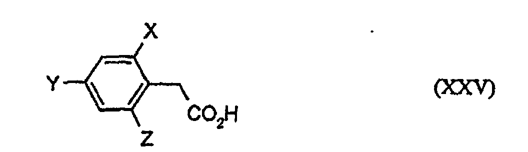 in which
X, Y and Z have the meanings given in Claim 1.

8. Compounds of the formula (XXVI) 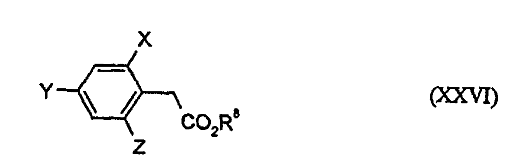 in which
X, Y and Z have the meanings given above, and
R⁸ represents alkyl.

9. Compounds of the formula (XXVII) 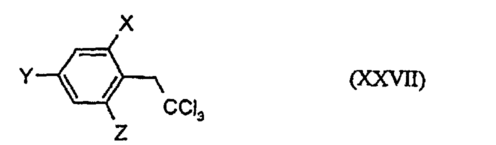 in which
X, Y and Z have the meanings given in Claim 1.

10. Compounds of the formula (XXXI) 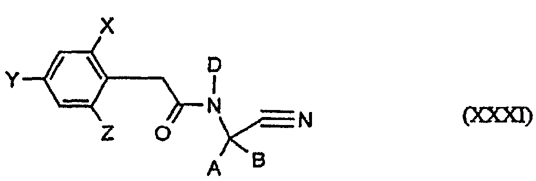 in which
A, B, D, X, Y and Z have the meanings given in Claim 1.

11. Compounds of the formula (III) 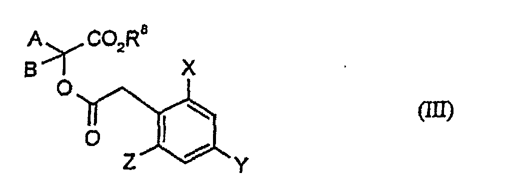 in which
A, B, X, Y and Z have the meanings given above, and
R⁸ represents alkyl.

12. Compounds of the formula (IV) 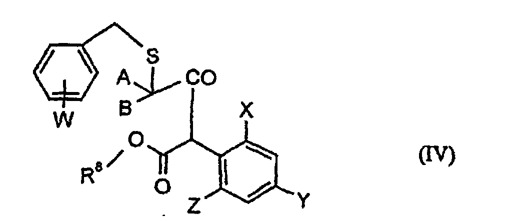 in which
A, B, X, Y and Z have the meanings given in Claim 1,
W represents hydrogen, halogen, alkyl or alkoxy and
R⁸ represents alkyl.

13. Compounds of the formula (V) 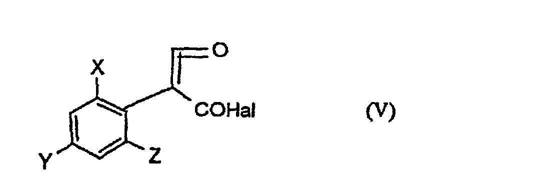 in which
X, Y and Z have the meanings given in Claim 1 and
Hal represents chlorine or bromine.

14. Compounds of the formula (XXXV) 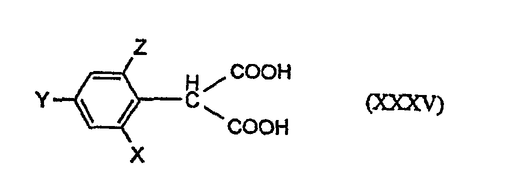 in which
X, Y and Z have the meanings given in Claim 1.

15. Compounds of the formula (VI) 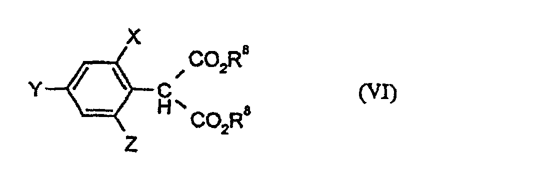 in which
X, Y and Z have the meanings given in Claim 1 and
R⁸ represents alkyl.

16. Pesticides and weedkillers, **characterized in that** they contain at least one compound of the formula (I) according to Claim 1.

17. Use of compounds of the formula (I) according to Claim 1 for the control of pests and weeds.

18. Method for the control of pests and weeds, **characterized in that** compounds of the formula (I) according to Claim 1 are allowed to act on pests and/or their habitat or on weeds and/or their habitat.

19. Process for the production of pesticides and weedkillers, **characterized in that** compounds of the formula (I) according to Claim 1 are mixed with extenders and/or surface-active agents.

20. Use of compounds of the formula (I) according to Claim 1 for the production of pesticides and weedkillers.

## Revendications

1. Composés de formule (I) 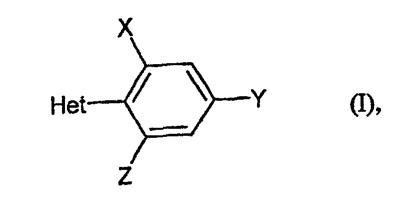 dans laquelle
X est un reste méthyle, éthyle, n-propyle ou isopropyle,
Y est le fluor, le chlore, le brome, un reste méthyle, éthyle, n-propyle ou isopropyle,
Z est le fluor, le chlore, le brome, un reste méthyle, éthyle, n-propyle ou isopropyle,
l'un des restes Y et Z étant toujours un halogène et l'autre un reste alkyle,
Het représente l'un des groupes 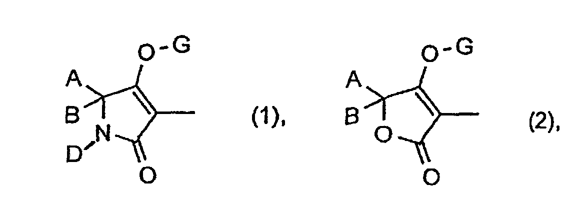 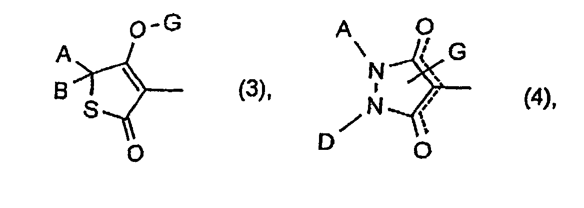 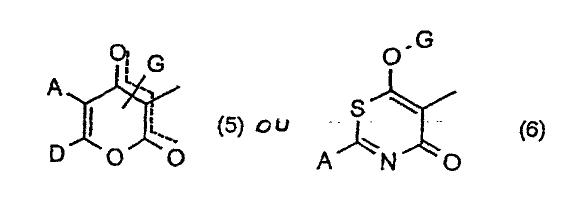
A représente l'hydrogène, un reste alkyle en C₁ à C₈, alcényle en C₂ à C₄, (alkoxy en C₁ à C₆)-(alkyle en C₁ à C₄), poly(alkoxy en C₁ à C₄) (alkyle en C₁ à C₄) ou (alkylthio en C₁ à C₆)-(alkyle en C₁ à C₄), chacun étant éventuellement substitué par du fluor ou du chlore, ou
un reste cycloalkyle en C₃ à C₆ éventuellement substitué par du fluor, du chlore, un radical méthyle ou méthoxy et dans lequel, le cas échéant, un ou deux groupes méthylène sont remplacés par de l'oxygène et/ou du soufre, ou bien
un reste phényle, furannyle, thiényle, pyridyle ou benzyle dont chacun est éventuellement substitué par du fluor, du chlore, du brome, un radical méthyle, éthyle, n-propyle, isopropyle, méthoxy, éthoxy, trifluoro-méthyle, trifluorométhoxy, cyano ou nitro,
B représente l'hydrogène, un reste alkyle en C₁ à C₈ ou un reste (alkoxy en C₁ à C₄)-(alkyle en C₁ ou C₂), ou bien
A, B et l'atome de carbone auquel ils sont liés forment un reste cycloalkyle en C₃ à C₈ ou cycloalcényle en C₅ à C₈, dans chacun desquels, le cas échéant, un groupe méthylène est remplacé par de l'oxygène ou du soufre et qui sont éventuellement substitués par un radical méthyle, éthyle, n-propyle, isopropyle, butyle, isobutyle, sec.-butyle, tertio-butyle, cyclohexyle, trifluorométhyle, méthoxy, éthoxy, n-propoxy, isopropoxy, butoxy, isobutoxy, sec.-butoxy, tertio-butoxy, méthylthio, éthylthio, fluoro, chloro ou phényle, ou bien
A, B et l'atome de carbone auquel ils sont liés forment un reste cycloalkyle qui est substitué par un groupe alkylènediyle contenant éventuellement un atome d'oxygène ou de soufre ou par un groupe alkylène-dioxyle qui forme avec l'atome de carbone auquel il est lié un autre noyau pentagonal ou hexagonal, ou bien
A, B et l'atome de carbone auquel ils sont liés forment un reste cycloalkyle en C₃ à C₆ ou cycloalcényle en C₅ ou C₆ dont deux substituants forment conjointement avec les atomes de carbone auxquels ils sont liés un reste alcanediyle en C₃ ou C₄, alcènediyle en C₃ ou C₄ ou butadiènediyle, où dans chaque cas un groupe méthylène est éventuellement remplacé par de l'oxygène ou du soufre,
D représente l'hydrogène, un reste alkyle en C₁ à C₈, alcényle en C₃ ou C₄, alcynyle en C₃ ou C₄, (alkoxy en C₁ à C₆)-(alkyle en C₂ à C₄), poly(alkoxy en C₁ à C₄)-(alkyle en C₂ à C₄), (alkylthio en C₁ à C₄)-(alkyle en C₂ à C₄) ou cycloalkyle en C₃ à C₆ dont chacun est éventuellement substitué par du fluor ou du chlore et dans chacun desquels, le cas échéant, un ou deux groupes méthylène non contigus sont remplacés par de l'oxygène et/ou du soufre, ou bien un reste phényle, furannyle, pyridyle, thiényle ou benzyle dont chacun est éventuellement substitué par du fluor, du chlore, du brome, un radical méthyle, éthyle, n-propyle, isopropyle, méthoxy, éthoxy, trifluorométhyle, trifluorométhoxy, cyano ou nitro,
ou bien
A et D forment ensemble un groupe alcanediyle en C₃ à C₅ ou alcènediyle en C₃ à C₅, dans chacun desquels, le cas échéant, un groupe méthylène est remplacé par de l'oxygène ou du soufre et qui sont éventuellement substitués par un radical fluoro, chloro, hydroxy, mercapto ou par un radical alkyle en C₁ à C₆, alkoxy en C₁ à C₄, alkylthio en C₁ à C₄, cycloalkyle en C₃ à C₆, phényle ou benzyloxy dont chacun est éventuellement substitué par du fluor ou du chlore, ou bien
le cas échéant, l'un des groupes suivants : 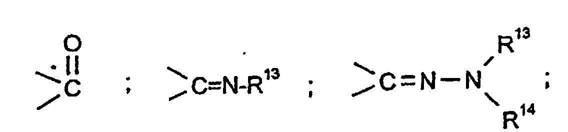 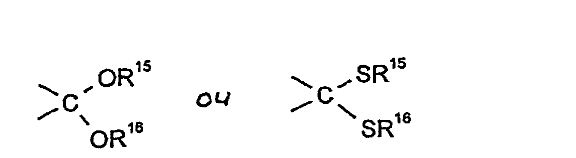 est présent,
ou bien A et D, dans le cas des composés de formule (I-1), forment conjointement avec les atomes auxquels ils sont liés l'un des groupes suivants : 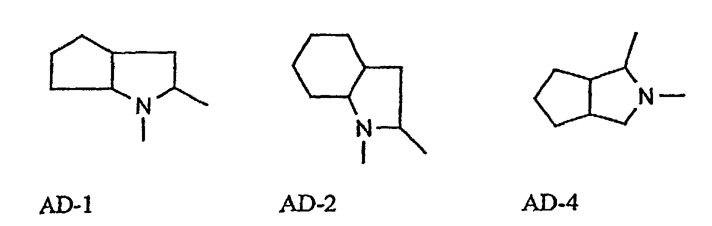 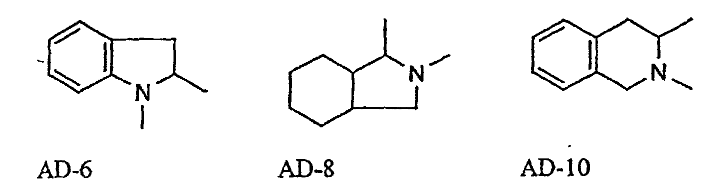 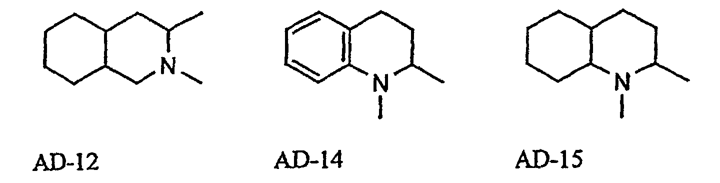 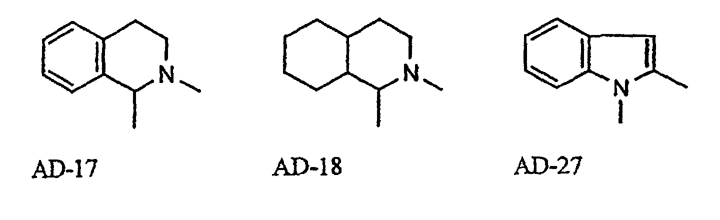
G, au cas où Het représente l'un des restes (1), (2), (3), (5) ou (6), est l'hydrogène (a) ou bien, au cas où Het représente l'un des groupes (1), (2), (3), (4), (5) ou (6), Het est l'un des groupes 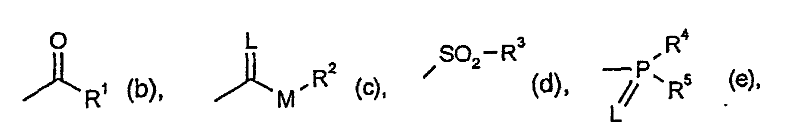 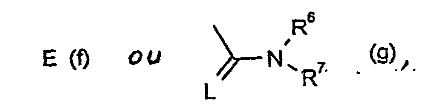 dans lesquels
E est un équivalent d'ion métallique ou un ion ammonium,
L représente l'oxygène ou le soufre et
M représente l'oxygène ou le soufre,
R¹ représente un reste alkyle en C₁ à C₁₄, alcényle en C₂ à C₁₄, (alkoxy en C₁ à C₄) - (alkyle en C₁ à C₆), (alkylthio en C₁ à C₄)-(alkyle en C₁ à C₆), poly(alkoxy en C₁ à C₄)-(alkyle en C₁ à C₄) dont chacun est éventuellement substitué par du fluor ou du chlore, ou un reste cycloalkyle en C₃ à C₆ éventuellement substitué par un radical fluoro, chloro, méthyle, éthyle, n-propyle, isopropyle, n-butyle, isobutyle, tertio-butyle, méthoxy, éthoxy, n-propoxy ou isopropoxy et dans lequel, le cas échéant, un ou deux groupes méthylène sont remplacés par de l'oxygène et/ou du soufre,
un reste phényle éventuellement substitué par un radical fluoro, chloro, bromo, cyano, nitro, méthyle, éthyle, n-propyle, isopropyle, méthoxy, éthoxy, trifluorométhyle, trifluorométhoxy, méthylthio, éthylthio, méthylsulfonyle ou éthylsulfonyle,
un reste benzyle éventuellement substitué par du fluor, du chlore, du brome, un radical méthyle, éthyle, n-propyle, isopropyle, méthoxy, éthoxy, trifluorométhyle ou trifluorométhoxy,
un reste furannyle, thiényle ou pyridyle dont chacun est éventuellement substitué par du fluor, du chlore, du brome, un radical méthyle ou éthyle,
un reste phénoxy-(alkyle en C₁ à C₄) éventuellement substitué par du fluor, du chlore, un radical méthyle ou éthyle, ou bien
un reste pyridyloxy-(alkyle en C₁ à C₄), pyrimidyloxy-(alkyle en C₁ à C₄) ou thiazolyloxy-(alkyle en C₁ à C₄) dont chacun est éventuellement substitué par du fluor, du chlore, un radical amino, méthyle ou éthyle,
R² représente un reste alkyle en C₁ à C₁₄, alcényle en C₂ à C₁₄, (alkoxy en C₁ à C₄)-(alkyle en C₂ à C₆) ou poly-(alkoxy en C₁ à C₄) - (alkyle en C₂ à C₆) dont chacun est éventuellement substitué par du fluor ou du chlore,
un reste cycloalkyle en C₃ à C₆ éventuellement substitué par du fluor, du chlore, un radical méthyle, éthyle, n-propyle, isopropyle ou méthoxy,
ou bien un radical phényle ou benzyle dont chacun est éventuellement substitué par du fluor, du chlore, un radical cyano, nitro, méthyle, éthyle, n-propyle, isopropyle, méthoxy, éthoxy, trifluorométhyle ou trifluorométhoxy,
R³ représente un reste méthyle, éthyle, propyle, isopropyle dont chacun est éventuellement substitué par du fluor ou du chlore ou un reste phényle ou benzyle dont chacun est éventuellement substitué par du fluor, du chlore, du brome, un radical méthyle, éthyle, propyle, isopropyle, tertio-butyle, méthoxy, éthoxy, isopropoxy, tertio-butoxy, trifluorométhyle, trifluorométhoxy, cyano ou nitro,
R⁴ et R⁵ représentent, indépendamment l'un de l'autre, un reste alkyle en C₁ à C₄, alkoxy en C₁ à C₄, alkylamino en C₁ à C₄, di-(alkyle en C₁ à C₄) amino ou alkylthio en Ci à C₄ dont chacun est éventuellement substitué par du fluor ou du chlore, ou un reste phényle, phénoxy ou phénylthio dont chacun est éventuellement substitué par du fluor, du chlore, du brome, un radical nitro, cyano, méthyle, méthoxy, trifluorométhyle ou trifluorométhoxy,
R⁶ et R⁷ représentent, indépendamment l'un de l'autre, l'hydrogène, un reste alkyle en C₁ à c₄, cycloalkyle en C₃ à C₆, alkoxy en C₁ à C₄, alcényle en C₃ ou C₄ ou (alkoxy en C₁ à C₄)-(alkyle en C₂ à C₄) dont chacun est éventuellement substitué par du fluor ou du chlore, un reste phényle ou benzyle dont chacun est éventuellement substitué par du fluor, du chlore, du brome, un radical méthyle, méthoxy ou trifluorométhyle, ou forment ensemble un reste alkylène en C₅ ou C₆ éventuellement substitué par un radical méthyle ou éthyle et dans lequel, le cas échéant, un groupe méthylène est remplacé par de l'oxygène ou du soufre,
R¹³ représente l'hydrogène ou un reste alkyle en C₁ à C₄ ou alkoxy en C₁ à C₄, dont chacun est éventuellement substitué par du fluor ou du chlore, un reste cycloalkyle en C₃ à C₆ ou un reste phényle, phényl-(alkyle en C₁ ou C₂) ou benzyloxy dont chacun est éventuellement substitué par du fluor, du chlore, du brome, un radical méthyle, éthyle, isopropyle, tertio-butyle, méthoxy, éthoxy, isopropoxy, tertio-butoxy, trifluorométhyle, trifluorométhoxy, nitro ou cyano,
R¹⁴ représente l'hydrogène ou un reste alkyle en C₁ à C₄, ou bien
R¹³ et R¹⁴ forment ensemble un reste alcanediyle en C₄ à C₆,
R¹⁵ et R¹⁶ sont identiques ou différents et représentent un reste méthyle ou éthyle, ou bien
R¹⁵ et R¹⁶ forment ensemble un reste alcanediyle en C₂ ou C₃ qui est substitué, le cas échéant, par un reste méthyle, éthyle, n-propyle, isopropyle, n-butyle, isobutyle, sec.-butyle ou tertio-butyle ou par un reste phényle éventuellement substitué par du fluor, du chlore, un radical méthoxy, trifluorométhyle, trifluorométhoxy, nitro ou cyano.

2. Composés de formule (I) suivant la revendication 1, formule dans laquelle les substituants ont les définitions indiquées dans le tableau suivant :

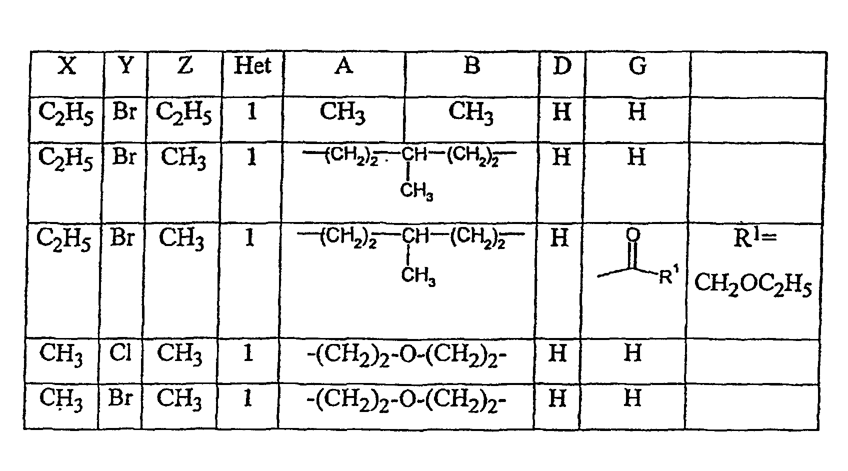

3. Procédé de production de composés de formule (I) suivant la revendication 1, **caractérisé en ce que** :
(A) on obtient des composés de formule (I-1-a) 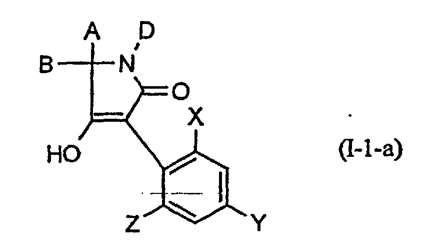 dans laquelle
A, B, D, X, Y et Z ont les définitions indiquées dans la revendication 1,
en effectuant la condensation intramoléculaire de composés de formule (II) 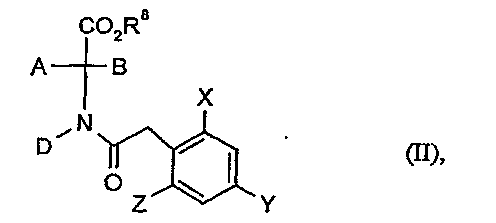 dans laquelle
A, B, D, X, Y et Z ont les définitions indiquées ci-dessus,
et
R⁸ est un reste alkyle,
en présence d'un diluant et en présence d'une base,
(B) on obtient des composés de formule (I-2-a) 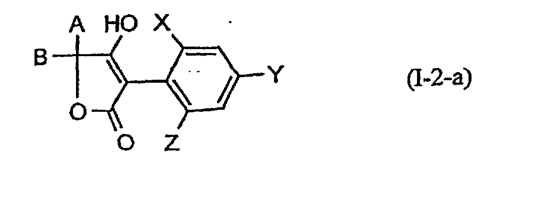 dans laquelle A, B, X, Y et Z ont les définitions indiquées ci-dessus,
en effectuant la condensation intramoléculaire de composés de formule (III) 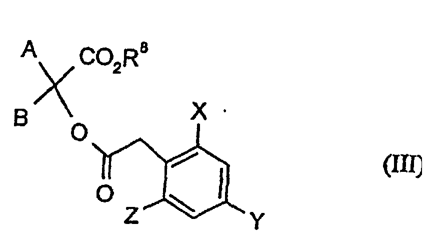 dans laquelle
A, B, X, Y, Z et R⁸ ont les définitions indiquées ci-dessus,
en présence d'un diluant et en présence d'une base,
(C) on obtient des composés de formule (1-3-a) 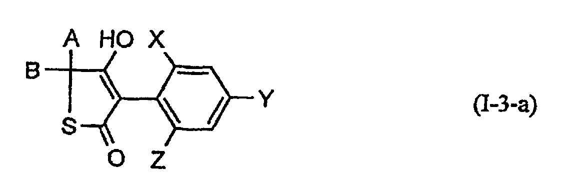 dans laquelle
A, B, X, Y et Z ont les définitions indiquées ci-dessus,
en effectuant la cyclisation intramoléculaire de composés de formule (IV) 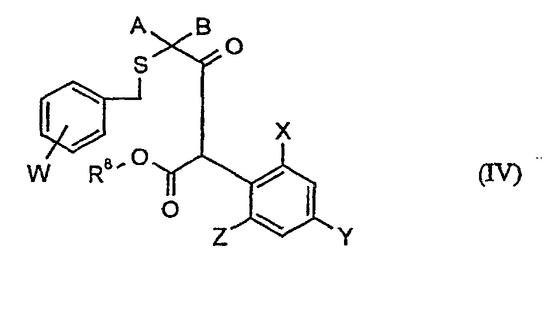 dans laquelle
A, B, X, Y, Z et R⁸ ont les définitions indiquées ci-dessus et
W représente l'hydrogène, un halogène, un reste alkyle ou alkoxy,
le cas échéant en présence d'un diluant et en présence d'un acide,
(E) on obtient les composés de formule (I-5-a) 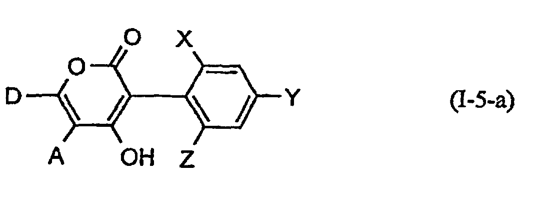 dans laquelle
A, D, X, Y et Z ont les définitions indiquées ci-dessus,
en faisant réagir des composés de formule (VIII) 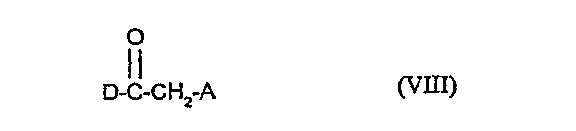 dans laquelle
A et D ont les définitions indiquées ci-dessus,
avec des composés de formule (V) 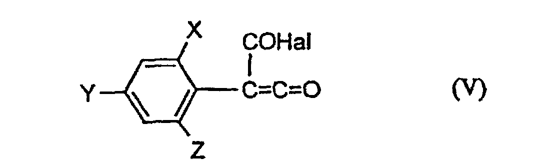 dans laquelle
X, Y et Z ont les définitions indiquées ci-dessus et
Hal représente un halogène,
le cas échéant en présence d'un diluant et en présence éventuelle d'un accepteur d'acide,
(F) on obtient les composés de formule (I-6-a) 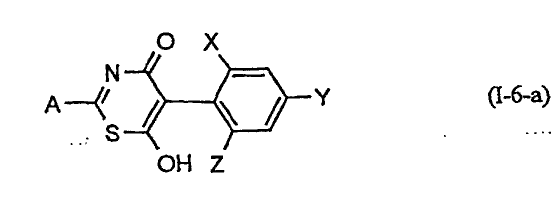 dans laquelle
A, X, Y et Z ont les définitions indiquées ci-dessus,
en faisant réagir des composés de formule (IX) 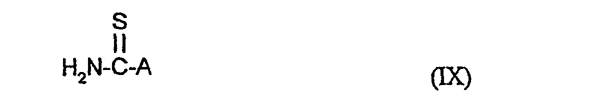 dans laquelle
A a la définition indiquée ci-dessus,
avec des composés de formule (V) 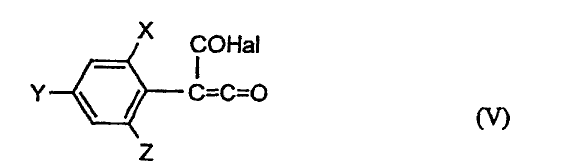 dans laquelle
Hal, X, Y et Z ont les définitions indiquées ci-dessus,
le cas échéant en présence d'un diluant et en présence éventuelle d'un accepteur d'acide puis, le cas échéant, en faisant réagir les composés ainsi obtenus, des formules (I-1-a) à (I-3-a), (I-5-a) et (I-6-a) indiquées ci-dessus ou des composés de formule (I-4-a) 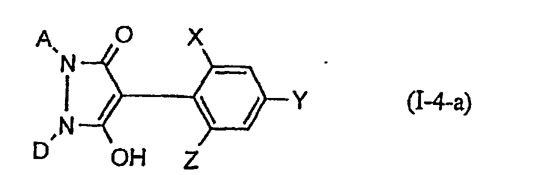 dans laquelle
A, D, X, Y et Z ont les définitions indiquées ci-dessus, dans chaque cas
(G)
α) avec des halogénures d'acides de formule (X) 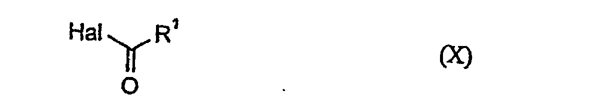 dans laquelle
R¹ a la définition indiquée ci-dessus et
Hal représente un halogène
ou bien
β) avec des anhydrides d'acides carboxyliques de formule (XI)
R¹-CO-O-CO-R¹ (XI)
dans laquelle
R¹ a la définition indiquée ci-dessus,
le cas échéant en présence d'un diluant et en présence éventuelle d'un accepteur d'acide,
(H) avec des esters d'acide chloroformique ou des thio-esters d'acide chloroformique de formule (XII)
R²-M-CO-Cl (XII)
dans laquelle
R² et M ont les définitions indiquées ci-dessus,
le cas échéant en présence d'un diluant et en présence éventuelle d'un accepteur d'acide,
ou bien
(I)
α) avec des esters d'acide chloromonothioformique ou des esters d'acide chlorodithioformique de formule (XIII) 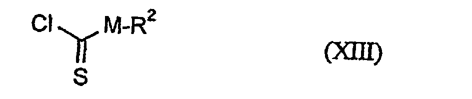 dans laquelle
M et R² ont les définitions indiquées ci-dessus,
le cas échéant en présence d'un diluant et en présence éventuelle d'un accepteur d'acide,
ou bien
β) avec le sulfure de carbone, puis avec des composés de formule (XIV)
R²-Hal (XIV)
dans laquelle
R² a la définition indiquée ci-dessus et
Hal représente le chlore, le brome ou l'iode,
le cas échéant en présence d'un diluant et en présence éventuelle d'une base,
ou bien
(J) avec des chlorures d'acide sulfonique de formule (XV)
R³-SO₂-Cl (XV)
dans laquelle
R³ a la définition indiquée ci-dessus,
le cas échéant en présence d'un diluant et en présence éventuelle d'un accepteur d'acide,
ou bien
(K) avec des composés phosphorés de formule (XVI) 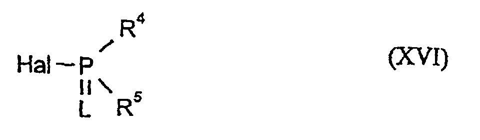 dans laquelle
L, R⁴ et R⁵ ont les définitions indiquées ci-dessus et
Hal représente un halogène,
le cas échéant en présence d'un diluant et en présence éventuelle d'un accepteur d'acide,
ou bien
(L) avec des composés métalliques ou des amines de formules (XVII) ou (XVIII)
Me(OR¹⁰)ₜ (XVII) 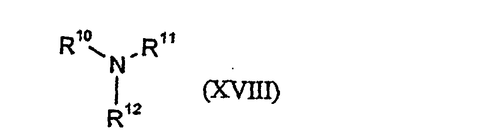
dans lesquelles
Me représente un métal monovalent ou divalent,
t représente le nombre 1 ou 2 et
R¹⁰, R¹¹ et R¹² représentent, indépendamment les uns des autres, l'hydrogène ou un reste alkyle,
le cas échéant en présence d'un diluant,
ou bien
(M)
α) avec des isocyanates ou des isothiocyanates de formule (XIX)
R⁶-N=C=L (XIX)
dans laquelle
R⁶ et L ont les définitions indiquées ci-dessus,
le cas échéant en présence d'un diluant et en présence éventuelle d'un catalyseur,
ou bien
β) avec des chlorures d'acide carbamique ou des chlorures d'acide thiocarbamique de formule (XX) 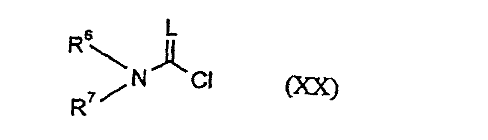 dans laquelle
L, R⁶ et R⁷ ont les définitions indiquées ci-dessus,
le cas échéant en présence d'un diluant et en présence éventuelle d'un accepteur d'acide.

4. Composés de formule (II) 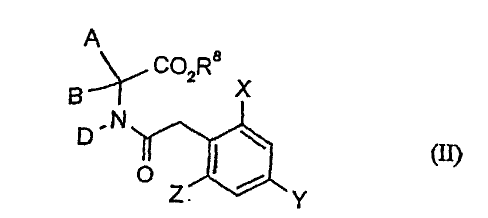 dans laquelle
A, B, D, X, Y et Z ont les définitions indiquées dans la revendication 1 et
R⁸ est un reste alkyle.

5. Composés de formule (XXIII) 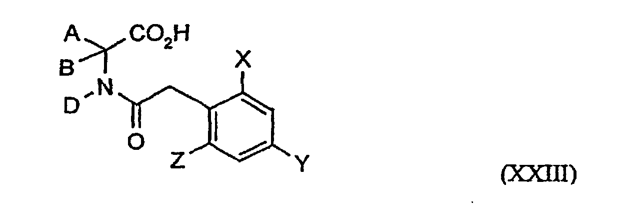 dans laquelle
A, B, D, X, Y et Z ont les définitions indiquées dans la revendication 1.

6. Composés de formule (XXII) 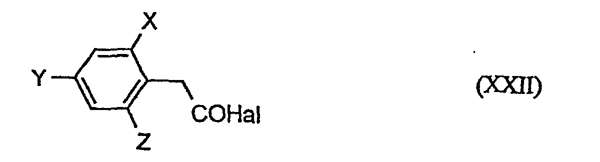 dans laquelle
X, Y et Z ont les définitions indiquées dans la revendication 1 et
Hal représente le chlore ou le brome.

7. Composés de formule (XXV) 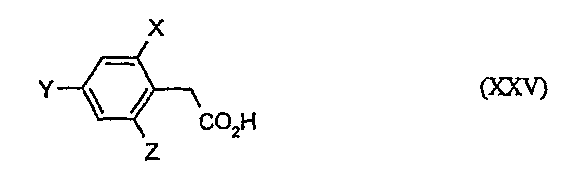 dans laquelle
X, Y et Z ont la définition indiquée dans la revendication 1.

8. Composés de formule (XXVI) 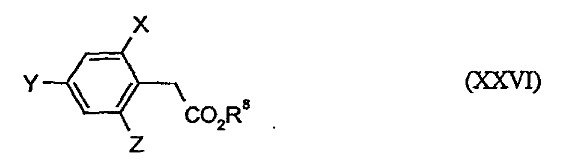 dans laquelle
X, Y et Z ont la définition indiquée ci-dessus et
R⁸ est un reste alkyle.

9. Composés de formule (XXVII) 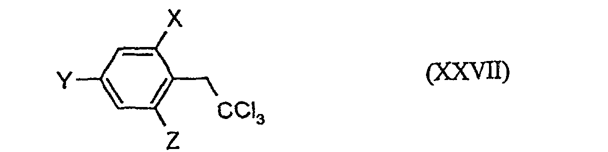 dans laquelle
X, Y et Z ont la définition indiquée dans la revendication 1.

10. Composés de formule (XXXI) 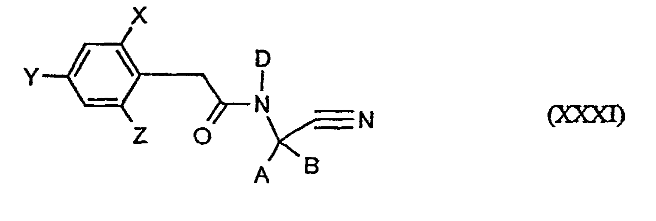 dans laquelle
A, B, D, X, Y et Z ont les définitions indiquées dans la revendication 1.

11. Composés de formule (III) 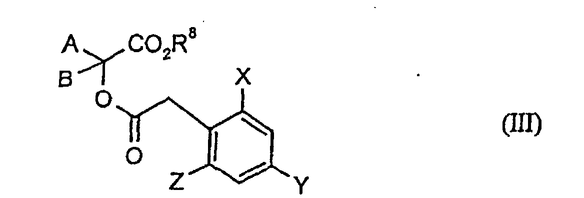 dans laquelle
A, B, X, Y et Z ont les définitions indiquées ci-dessus, et
R⁸ est un reste alkyle.

12. Composés de formule (IV) 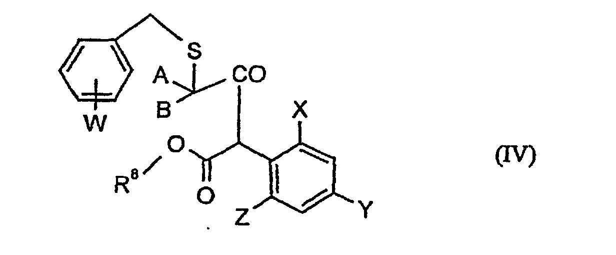 dans laquelle
A, B, X, Y et Z ont les définitions indiquées dans la revendication 1,
W représente l'hydrogène, un halogène, un reste alkyle ou alkoxy et
R⁸ est un reste alkyle.

13. Composés de formule (V) 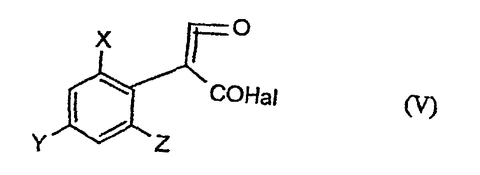 dans laquelle
X, Y et Z ont les définitions indiquées dans la revendication 1 et
Hal représente le chlore ou le brome.

14. Composés de formule (XXXV) 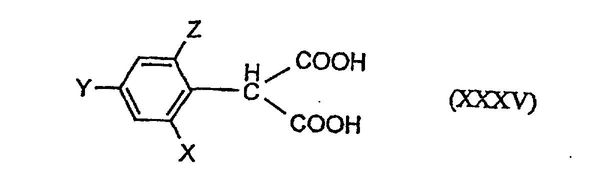 dans laquelle
X, Y et Z ont les définitions indiquées dans la revendication 1.

15. Composés de formule (VI) 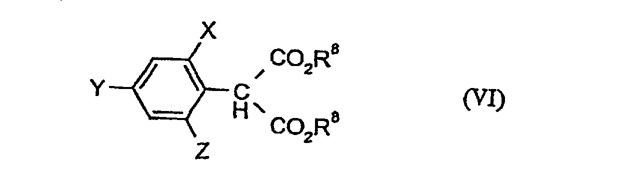 dans laquelle
X, Y et Z ont les définitions indiquées dans la revendication 1 et
R⁸ est un reste alkyle.

16. Compositions pesticides et compositions herbicides, **caractérisées par** une teneur en au moins un composé de formule (I) suivant la revendication 1.

17. Utilisation de composés de formule (I) suivant la revendication 1 pour combattre des parasites et des mauvaises herbes.

18. Procédé pour combattre des parasites et des mauvaises herbes, **caractérisé en ce qu'**on fait agir des composés de formule (I) suivant la revendication 1 sur les parasites et/ou sur leur milieu ou on les fait agir sur des mauvaises herbes et/ou sur leur milieu.

19. Procédé de préparation de compositions pesticides et de compositions herbicides, **caractérisé en ce qu'**on mélange des composés de formule (I) suivant la revendication 1 avec des diluants et/ou des agents tensioactifs.

20. Utilisation de composés de formule (I) suivant la revendication 1 pour la préparation de compositions pesticides et de compositions herbicides.
